(19) Europäisches Patentamt
European Patent Office
Office européen des brevets

(11) Numéro de publication : **0 452 204 B1**

(12) # FASCICULE DE BREVET EUROPEEN

(45) Date de publication du fascicule du brevet : **15.06.94 Bulletin 94/24**

(21) Numéro de dépôt : **91400942.8**

(22) Date de dépôt : **09.04.91**

(51) Int. Cl.$^5$ : **C07D 311/58,** C07D 335/06, C07D 405/12, C07D 498/04, // (C07D498/04, 263:00, 221:00)

(54) **Nouveaux dérivés du 3-aminochromane, leur procédé de préparation et les compositions pharmaceutiques qui les contiennent.**

(30) Priorité : **09.04.90 FR 9004481**

(43) Date de publication de la demande : **16.10.91 Bulletin 91/42**

(45) Mention de la délivrance du brevet : **15.06.94 Bulletin 94/24**

(84) Etats contractants désignés : **AT BE CH DE DK ES FR GB GR IT LI LU NL SE**

(56) Documents cités : **EP-A- 0 222 996**

(73) Titulaire : **ADIR ET COMPAGNIE
1 rue Carle Hébert
F-92415 Courbevoie Cédex (FR)**

(72) Inventeur : **Guillaumet, Gérald
2 rue Auguste Renoir
F-45100 Orléans (FR)**
Inventeur : **Guardiola, Béatrice
6 rue Edouard Nortier
F-92200 Neuilly sur Seine (FR)**

**EP 0 452 204 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés du 3-aminochromane, leur procédé de préparation et les compositions pharmaceutiques qui les contiennent.

Les 3-aminochromane et les 3-aminothiochromane sont connus pour être des ligands des récepteurs du système nerveux central utilisables en particulier, comme ligands des récepteurs de la sérotonine, dans le traitement des désordres du système nerveux central, de la dépression et de l'anxiété.

Des dérivés d'aminochromane et d'aminothiochromane ont, par exemple, été décrits dans les brevets européens EP279150 et EP222996. Les composés décrits dans ces brevets présentent une affinité certaine pour les récepteurs 5-$HT_{1A}$ mais également pour les récepteurs $D_2$, ce qui entraîne une très faible sélectivité. Un facteur 10 sépare les affinités 5-$HT_{1A}$ par rapport à $D_2$. En revanche, les composés de la présente invention qui sont des dérivés du 3-aminochromane et du 3-aminothiochromane, outre le fait que leurs structures soient nouvelles, possèdent des propriétés pharmacologiques remarquables. En effet, ce sont de très puissants ligands des récepteurs 5-$HT_{1A}$. Cette très forte affinité est d'autant plus intéressante qu'elle est doublée d'une très grande sélectivité vis à vis de ces récepteurs par rapport aux récepteurs $D_2$ et $\alpha_2$. L'affinité des composés de l'invention vis à vis des récepteurs 5-$HT_{1A}$ est respectivement $10^2$ fois et $10^4$ fois plus importante que celle observée pour les récepteurs $D_2$ et $\alpha_2$. Ces propriétés pharmacologiques remarquables rendent les composés de la présente invention utilisables dans le traitement des maladies du système nerveux central, spécialement du système sérotoninergique, comme la dépression, l'anxiété, le stress, la schizophrénie, la douleur et également dans le traitement de l'hypertension, la prévention de l'athérome et comme modificateur alimentaire et sexuel.

Plus spécifiquement, la présente invention concerne les dérivés de formule générale (I) :

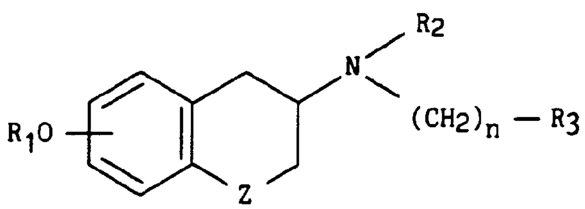

(I)

dans laquelle :

- Z représente un atome d'oxygène ou un atome de soufre,
- $R_1$ représente un atome d'hydrogène, ou un groupement alkyle ($C_1$-$C_6$) linéaire ou ramifié,
- $R_2$ représente un atome d'hydrogène ou un groupement alkyle ($C_1$-$C_6$) linéaire ou ramifié,
- n est un nombre entier compris entre 1 et 6,
- $R_3$ représente un groupement nitrile, un groupement amino éventuellement substitué par :
  . un groupement acyle linéaire ou ramifié comprenant de 2 à 7 atome de carbone,
  . un groupement alkylsulfonyl,
  . un groupement arylsulfonyl éventuellement substitué par un groupement alkyle, alkoxy, hydroxy ou un atome d'halogène,
  . un groupement alkyle ($C_1$-$C_6$) linéaire ou ramifié,
- ou $R_3$ représente l'un quelconque des groupements suivants :

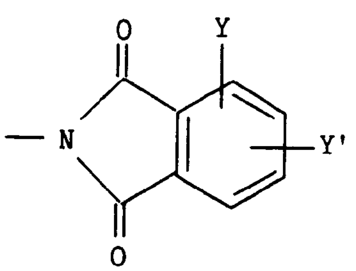
,
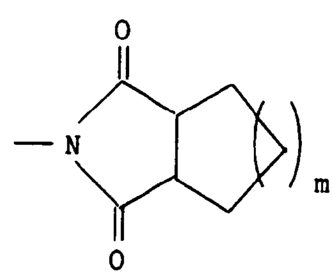
,

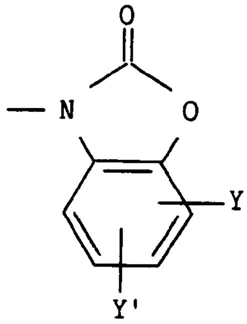 ,

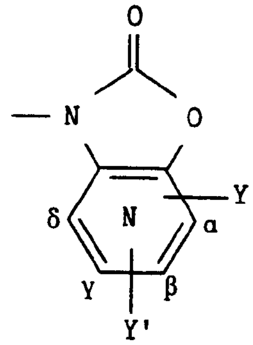 ,

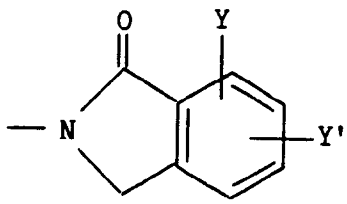 ,

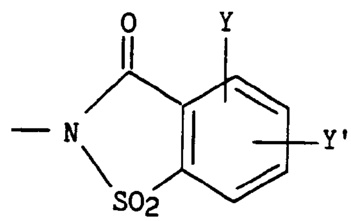 ,

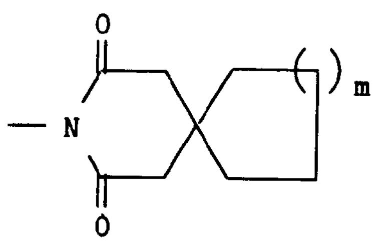 ,

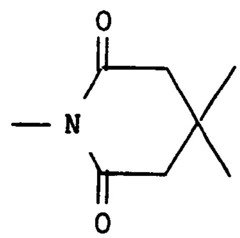 ,

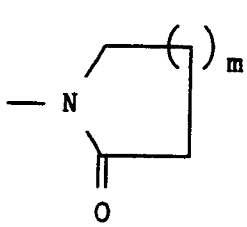

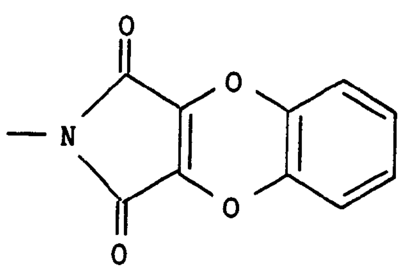 ,

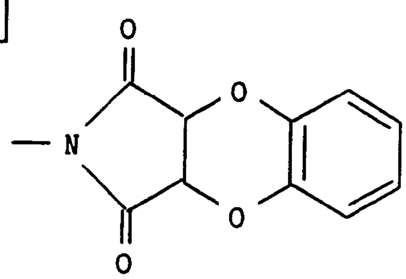 ,

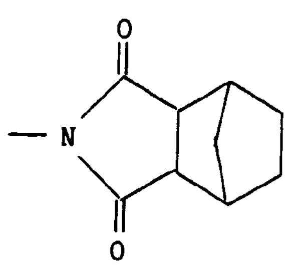 ,

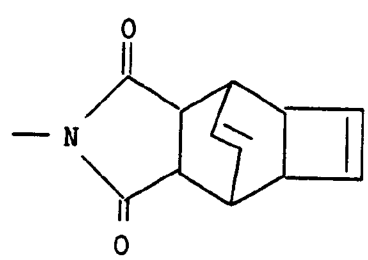

dans lesquels :

- . Y et Y′ identiques ou différents représentent un atome d'hydrogène, un atome d'halogène, un groupement alkyle, alkoxy ou hydroxy,
- . m est un nombre entier égal à 1 ou 2,
- . et l'azote du cycle pyridine se situe en position α, β, γ ou δ de la jonction du cycle,

leurs énantiomères, diastéréoisomères et épimères ainsi que leurs sels d'addition à un acide pharmaceutiquement acceptable.

Parmi les acides pharmaceutiquement acceptables, on peut citer à titre non limitatif les acides chlorhydrique, sulfurique, tartrique, maléique, fumarique, oxalique, méthane sulfonique, camphorique, etc...

L'invention s'étend aussi au procédé de préparation des dérivés de formule générale (I) caractérisé en ce que l'on utilise comme matière première,

ou bien

a un composé de formule (II) :

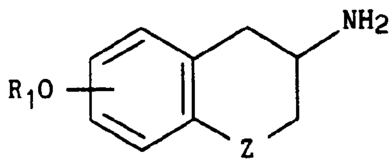

(II)

dans laquelle $R_1$ et Z ont la même signification que dans la formule (I), que l'on fait réagir sur un dérivé de formule (III) :

$R_3$-$(CH_2)_n$-X (III)

dans laquelle $R_3$ et n ont la même signification que dans la formule (I) et X est un atome d'halogène pour conduire à un dérivé de formule (I/a), cas particulier des dérivés de formule (I) :

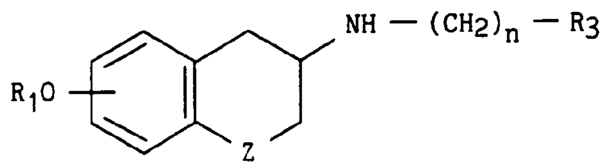

(I/a)

dans laquelle $R_1$, $R_3$, Z et n ont la même signification que dans la formule (I) que l'on soumet éventuellement à l'action d'un dérivé de formule (IV) :

$R'_2$-X (IV)

dans laquelle $R'_2$ représente un groupement alkyle (C1-C6) linéaire ou ramifié et X représente un atome d'halogène,

pour conduire à un dérivé de formule (I/b), cas particulier des dérivés de formule (I) :

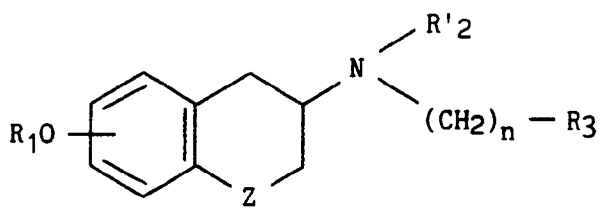

(I/b)

dans laquelle R, Z, $R'_2$, $R_3$ et n sont définis comme précédemment,

ou bien

b un composé de formule (V) :

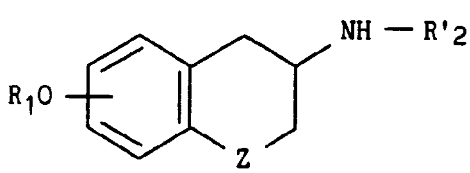

(V)

dans laquelle $R_1$, Z et $R'_2$ ont les mêmes significations que précédemment, que l'on fait réagir :

- soit sur un composé de formule (III) :

$R_3$-$(CH_2)_n$-X (III)

dans laquelle $R_3$, n et X sont définis comme précédemment, pour conduire à un dérivé de formule

(I/b), cas particulier des dérivés de formule (I) :

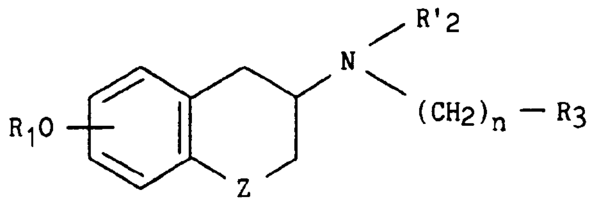

(I/b)

dans laquelle $R_1$, Z, $R'_2$, $R_3$ et n sont définis comme précédemment,

- soit sur un composé de formule (III/a) :

$$NC\text{-}(CH_2)_m\text{-}X \qquad (III/a)$$

dans laquelle X a la même signification que précédemment et m est un nombre entier compris entre 1 et 3, pour conduire à un dérivé de formule (I/c), cas particulier des dérivés de formule (I) :

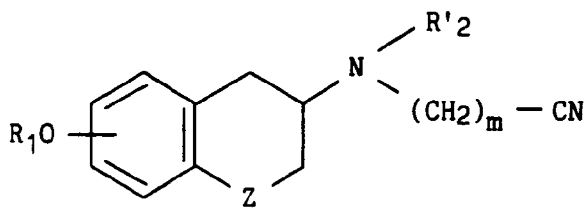

(I/c)

dans laquelle $R_1$, Z, $R'_2$ et m sont définis comme précédemment, que l'on réduit de façon catalytique en présence de nickel de Raney et d'ammoniac, ou chimiquement par l'hydrure de lithium/aluminium ou par le sodium dans l'alcool,
pour conduire au dérivé de formule (I/d), cas particulier des dérivés de formule (I) :

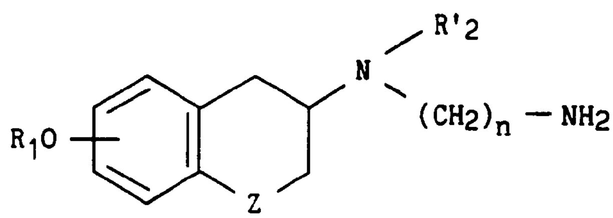

(I/d)

dans laquelle $R_1$, Z, $R'_2$ et n sont définis comme précédemment, que l'on fait réagir :

. ou bien sur un composé de formule (VI/A) :

$$R_{4A}\text{-}SO_2\text{-}X \qquad (VI/A)$$

dans laquelle $R_{4A}$ est un groupement alkyle ou un groupement aryle éventuellement substitué par un groupement alkyle et X est un atome d'halogène,
pour conduire à un dérivé de formule (I/e), cas particulier des dérivés de formule (I) :

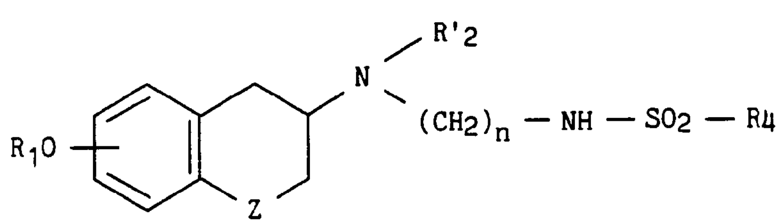

(I/e)

dans laquelle $R_1$, Z, $R'_2$, $R_4$, n sont définis comme précédemment,

. ou bien sur un composé de formule (VI/B) :

$$R_{4B}\text{-}CO\text{-}T \qquad (VI/B)$$

dans laquelle R4B représente un groupement alkyle linéaire ou ramifié de 1 à 6 atomes de carbone et T un groupe partant choisi parmi halogène, et alkoxy inférieur ou un composé de formule (VI/C) :

$R_{4B}$-CO-O-CO-$R_{4B}$ (VI/C)

dans laquelle $R_{4B}$ a la même définition que précédemment pour conduire à un composé de formule (I/F) :

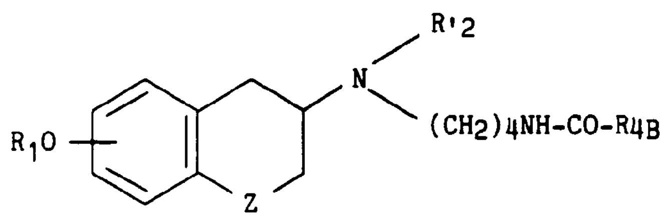

(I/F)

dans laquelle $R_1$, Z, $R'_2$, $R_{4B}$ et n ont la même définition que précédemment, cas particulier des dérivés de formule (I), pour lesquels $R_3$ représente un groupement amino substitué par un groupement acyle linéaire ou ramifié de deux à sept atomes de carbone,
ou bien
c un composé de formule (VII) :

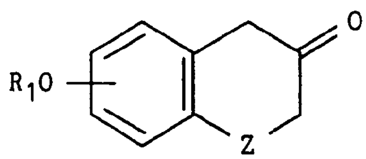

(VII)

dans laquelle $R_1$ et Z ont la même signification que dans la formule (I), que l'on soumet à une amination réductrice en présence d'un dérivé de formule (VIII) :

$R_2$-NH-$(CH_2)_n$-$R_3$ (VIII)

dans laquelle $R_2$, $R_3$ et n sont définis comme dans la formule (I),
pour conduire à un composé de formule (I), qui, lorsque $R_2$ représente un atome d'hydrogène peut être soumis à l'action d'un dérivé de formule (IV) :

$R'_2$-X (IV)

dans laquelle $R'_2$ et X sont définis comme précédemment,
pour conduire à un dérivé de formule (I/b), cas particulier des dérivés de formule (I) :

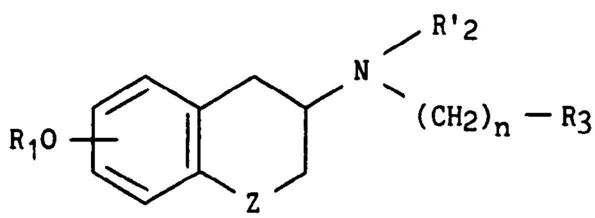

(I/b)

dans laquelle $R_1$, Z, $R'_2$, $R_3$ et n sont définis comme précédemment,
ou bien encore
d un composé de formule (IX) :

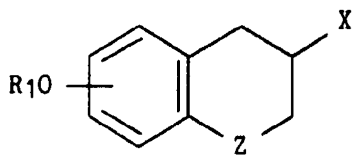

(IX)

dans laquelle $R_1$ et Z ont la même signification que dans la formule (I) et X est un atome d'halogène que l'on soumet à l'action d'un dérivé de formule (X) :

$H_2N-(CH_2)_n-R_3$ (X)

dans laquelle n et $R_3$ sont définis comme précédemment,pour conduire à un dérivé de formule (I/a), cas particulier des dérivés de formule (I) :

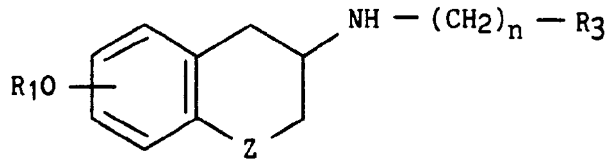

(I/a)

dans laquelle $R_1$, Z, n et $R_3$ sont définis comme précédemment, que l'on soumet éventuellement à l'action d'un dérivé de formule (IV)

$R'_2$-X (IV)

dans laquelle $R'_2$ et X sont définis comme précédemment,
pour conduire à un dérivé de formule (I/b), cas particulier des dérivés de formule (I) :

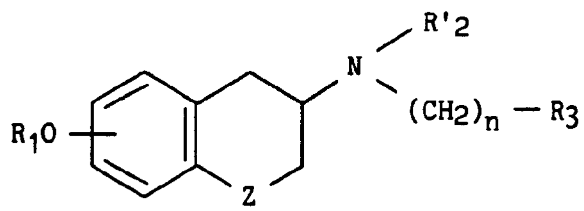

(I/b)

dans laquelle $R_1$, Z, $R'_2$, $R_3$ et n sont définis comme précédemment,
dérivés de formule (I/a), (I/b), (I/c), (I/d) et (I/e) dont on sépare éventuellement les isomères selon une technique classique de séparation, que l'on purifie, par une technique classique de purification et que l'on transforme, si on le désire, en leurs sels d'addition à un acide pharmaceutiquement acceptable.
Les composés de formule (I) possèdent des propriétés pharmacologiques très intéressantes.

Les essais de binding ont montré que les composés de l'invention se comportent comme de très puissants ligands des récepteurs 5-$HT_{1A}$, avec une activité agoniste ou antagoniste au niveau du système nerveux central. Cette très grande affinité est accompagnée d'une très grande sélectivité vis à vis de ces récepteurs par rapport aux récepteurs $D_2$ et $\alpha_2$.

Les composés de l'invention trouvent donc leur application dans le traitement du stress, de l'anxiété, de la dépression, de la schizophrénie et de la douleur, des maladies cardiovasculaires et de l'hypertension. Ils peuvent également modifier le comportement alimentaire et sexuel.

La présente invention a également pour objet les compositions pharmaceutiques renfermant comme principe actif au moins un composé de formule générale (I) ou un de ses sels d'addition à un acide pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie varie selon l'âge et le poids du patient, la nature et la sévérité de l'affection ainsi que la voie d'administration. Celle-ci peut être orale, nasale, rectale ou parentérale.

D'une manière générale, la posologie unitaire s'échelonne entre 0,1 et 100 mg pour un traitement en 1 à 3 prises par 24 heures.

Les exemples suivants illustrent l'invention

**EXEMPLE 1 :**

**3-[N-(4-PHTALIMIDO-BUTYL) AMINO]-5-METHOXY-CHROMANE**

Dans un ballon, 2,2 mmoles de 3-amino-5-méthoxy-chromane (décrit dans le brevet EP 279150) sont dissoutes dans 6 ml de diméthylformamide en présence de 2,4 mmoles de N-(4-bromobutyl) phtalimide, de 6,6 mmoles de carbonate de potassium et d'une quantité catalytique d'iodure de potassium. Le mélange est laissé sous agitation à 60°C pendant 6 heures. Après refroidissement, le solvant est évaporé et le brut réactionnel est extrait, après hydrolyse aqueuse, au dichlorométhane. Après lavage, séchage et évaporation de la phase organique, le produit attendu est obtenu après purification par chromatographie sur colonne de silice (solvant d'élution : éther/éther de pétrole : 5/95).

Rendement : 67 %

Résonance magnétique nucléaire du proton : ($CDCl_3$) :

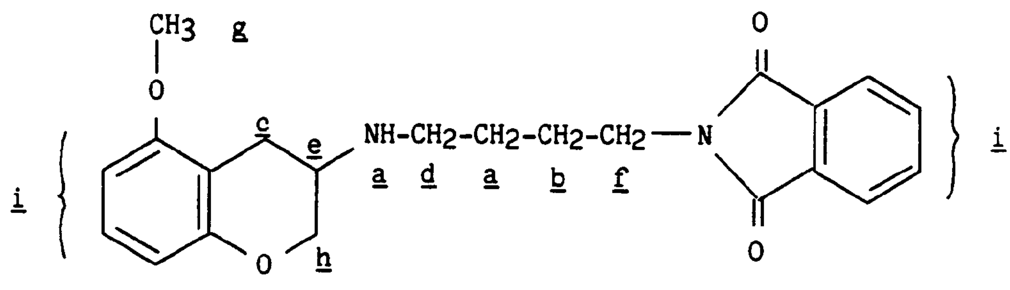

a δ = 1,56 ppm (3H,m)
b δ = 1,75 ppm (2H,m)
c δ entre 2,47 et 2,92 ppm (2H,dd)
d δ = 2,75 ppm (2H,t)
e δ = 3,09 ppm (1H,m)
f δ = 3,62 ppm (2H,t)
g δ = 3,80 ppm (3H,s)
h δ entre 3,84 et 4,13 ppm (2H,m)
i δ entre 6,42 et 7,83 ppm (7H,m)

**EXEMPLE 2** :

**3-[N-PROPYL-N-(4-PHTALIMIDO-BUTYL) AMINO]-5-METHOXY-CHROMANE**

1,5 mmoles du composé préparé dans l'exemple 1 est mis en solution dans 10 ml de diméthylformamide en présence de 4,4 mmoles de 1-iodopropane et de 4,4 mmoles de carbonate de potassium. Après 24 heures d'agitation à 60°C, le solvant est évaporé et le brut réactionnel est repris par 10 ml d'eau et extrait au dichlorométhane. La phase organique est séchée et évaporée et le produit attendu est obtenu après purification par chromatographie sur colonne de silice (solvant d'élution : éther/éther de pétrole : 1/99).

Rendement : 81 %

Résonance magnétique nucléaire du proton : ($CDCl_3$) :

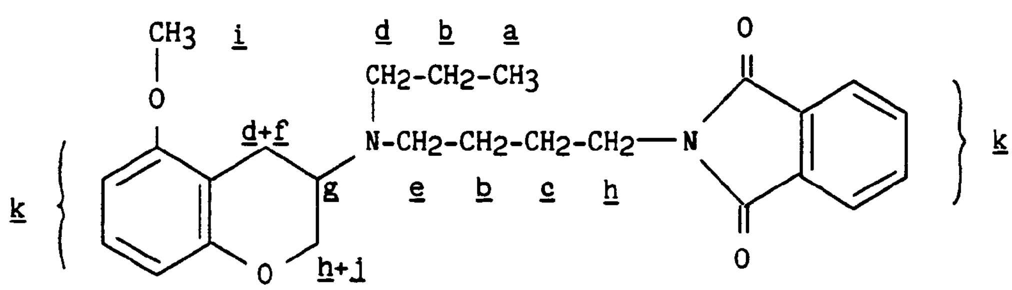

a δ = 0,88 ppm (3H,t)
b δ = 1,48 ppm (4H,m)
c δ = 1,72 ppm (2H,m)
d δ = 2,50 ppm (3H,m)
e δ = 2,60 ppm (2H,m)
f δ = 2,88 ppm (1H,dd)

g δ = 3,12 ppm (1H,m)
h δ = 3,70 ppm (3H,m)
i δ = 3,80 ppm (3H,s)
j δ = 4,24 ppm (1H,m)
k δ entre 6,42 et 7,84 ppm (7H,m)

**EXEMPLE 3 :**

**3-[N-(3-PHTALIMIDO-PROPYL) AMINO]-5-METHOXY-CHROMANE**

En procédant comme dans l'exemple 1 mais en remplaçant le N-(4-bromobutyl) phtalimide par le N-(3-bromopropyl) phtalimide, on obtient le produit attendu.

Rendement : 70 %

Résonance magnétique nucléaire du proton : ($CDCl_3$) :

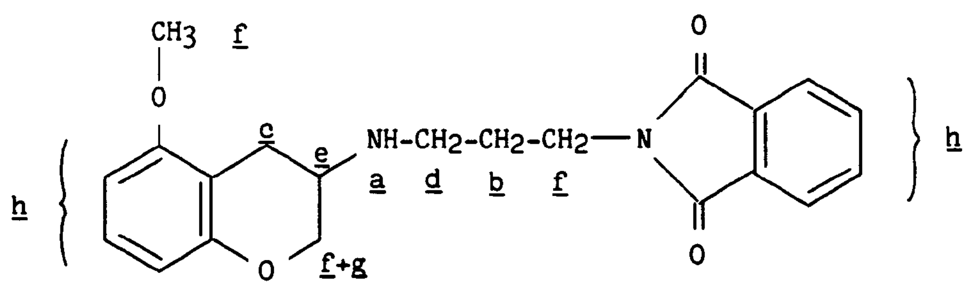

a δ = 1,56 ppm (1H,s)
b δ = 1,87 ppm (2H,m)
c δ entre 2,44 et 2,90 ppm (2H,dd)
d δ = 2,79 ppm (2H,m)
e δ = 3,06 ppm (1H,m)
f δ = 3,78 ppm (6H,m)
g δ = 4,12 ppm (1H,m)
h δ entre 6,40 et 7,83 ppm (7H,m)

**EXEMPLE 4 :**

**3-[N-PROPYL-N-(3-PHTALIMIDO-PROPYL) AMINO]-5-METHOXY-CHROMANE**

En procédant comme dans l'exemple 2, mais en remplaçant le composé de l'exemple 1 par le composé de l'exemple 3, on obtient le produit attendu.

Rendement : 76 %

Résonance magnétique nucléaire du proton : ($CDCl_3$) :

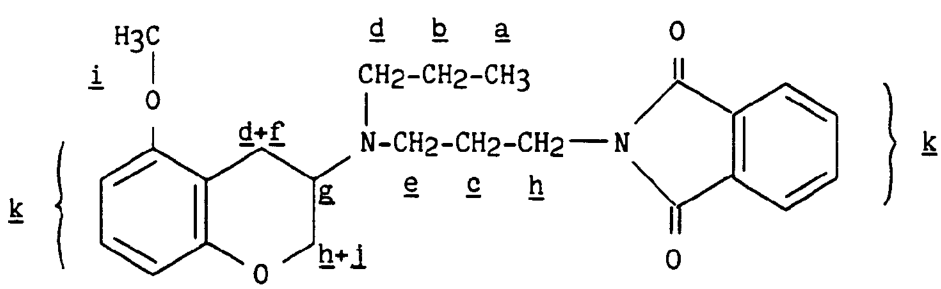

a δ = 0,90 ppm (3H,t)
b δ = 1,46 ppm (2H,m)
c δ = 1,81 ppm (2H,m)

d δ = 2,50 ppm (3H,m)
e δ = 2,64 ppm (2H,m)
f δ = 2,82 ppm (1H,dd)
g δ = 3,10 ppm (1H,m)
h δ = 3,72 ppm (3H,m)
i δ = 3,79 ppm (3H,s)
j δ = 4,23 ppm (1H,m)
k δ entre 6,40 et 7,83 ppm (7H,m)

**EXEMPLE 5 :**

**3-[N-(2-PHTALIMIDO-ETHYL) AMINO]-5-METHOXY-CHROMANE**

En procédant comme dans l'exemple 1 mais en remplaçant la N-(4-bromobutyl) phtalimide par la N-(2-bromoéthyl) phtalimide et en laissant 40 heures sous agitation, on obtient le produit attendu.

Rendement : 30 %

Résonance magnétique nucléaire du proton : ($CDCl_3$) :

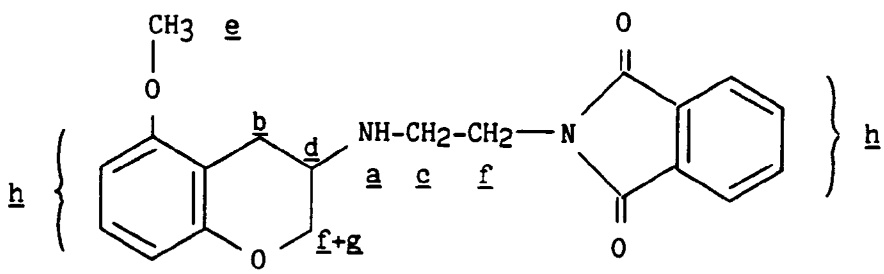

a δ = 1,48 ppm (1H,s)
b δ entre 2,44 et 2,88 ppm (2H,m)
c δ = 3,03 ppm (2H,m)
d δ = 3,15 ppm (1H,m)
e δ = 3,78 ppm (3H,s)
f δ = 3,81 ppm (3H,m)
g δ = 4,1 ppm (1H,m)
h δ entre 6,40 et 7,85 ppm (7H,m)

**EXEMPLE 6 :**

**3-[N-PROPYL-N-(2-PHTALIMIDO-ETHYL) AMINO]-5-METHOXY-CHROMANE**

En procédant comme dans l'exemple 2 mais en remplaçant le composé de l'exemple 1 par le composé de l'exemple 5, on obtient le produit attendu.

Rendement : 70 %

Résonance magnétique nucléaire du proton : ($CDCl_3$) :

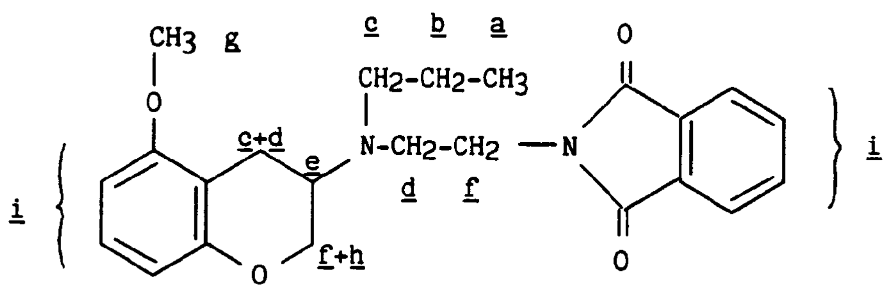

a δ = 0,79 ppm (3H,t)
b δ = 1,40 ppm (2H,m)
c δ = 2,50 ppm (3H,m)
d δ = 2,84 ppm (3H,m)
e δ = 3,19 ppm (1H,m)
f δ = 3,71 ppm (3H,m)
g δ = 3,79 ppm (3H,s)
h δ = 4,15 ppm (1H,m)
i δ entre 6,40 et 7,83 ppm (7H,m)

**EXEMPLE 7 :**

**3-[4- [N-(5-METHOXY-CHROMAN-3-YL) AMINO] BUTYL]-2-OXO-2,3-DIHYDRO-OXAZOLO[4,5-b]PYRIDINE**

En procédant comme dans l'exemple 1 mais en remplaçant la N-(4-bromobutyl) phtalimide par la N-(4-bromobutyl) oxo-2 oxozolo[4,5-b] pyridine et en laissant 48 heures sous agitation, on obtient le produit attendu.

Rendement : 53 %

Résonance magnétique nucléaire du proton : ($CDCl_3$) :

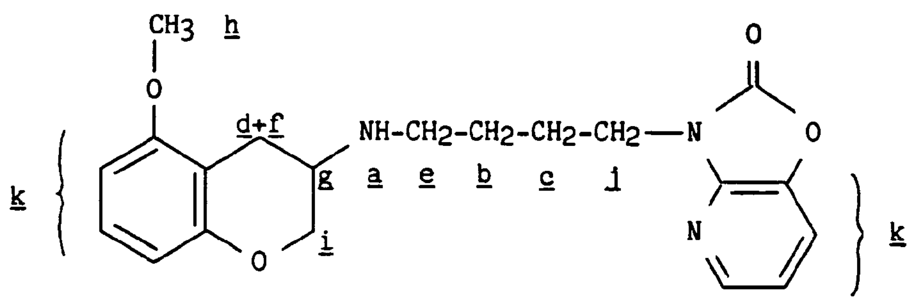

a δ = 1,34 ppm (1H,s)
b δ = 1,63 ppm (2H,m)
c δ = 1,90 ppm (2H,m)
d δ = 2,48 ppm (1H,dd)
e δ = 2,78 ppm (2H,m)
f δ = 2,92 ppm (1H,dd)
g δ = 3,07 ppm (1H,m)
h δ = 3,82 ppm (3H,s)
i δ entre 3,84 et 4,14 ppm (2H,m)
j δ = 3,97 ppm (2H,m)
k δ entre 6,44 et 8,10 ppm (6H,m)

**EXEMPLE 8 :**

**3-[4-[N-PROPYL-N-(5-METHOXY-CHROMAN-3-YL) AMINO]BUTYL]-2-OXO-2,3-DIHYDRO-OXAZOLO[4,5-b]PYRIDINE**

En procédant comme dans l'exemple 2 mais en remplaçant le composé de l'exemple 1 par le composé de l'exemple 5, on obtient le produit attendu.

Rendement : 65 %

Résonance magnétique nucléaire du proton : ($CDCl_3$) :

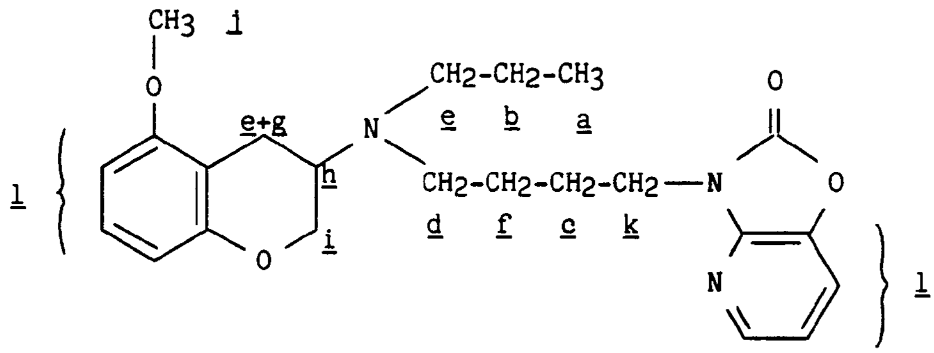

a δ = 0,84 ppm (3H,t)

b δ = 1,40 ppm (2H,m)

c δ = 1,50 ppm (2H,m)

d δ = 1,85 ppm (2H,m)

e δ = 2,49 ppm (3H,m)

f δ = 2,62 ppm (2H,m)

g δ = 2,83 ppm (1H,m)

h δ = 3,08 ppm (1H,m)

i δ = 3,66 ppm et 4,23 ppm (1H,m)

j δ = 3,81 ppm (3H,s)

k δ = 3,94 ppm (2H,t)

l δ entre 6,48 et 8,10 ppm (6H,m)

**EXEMPLE 9 :**

**3-[4-[N-(5-METHOXY-CHROMAN-3-YL) AMINO] BUTYL]-2,4-DIOXO-3-AZASPIRO[4,5]DECANE**

En procédant comme dans l'exemple 1 mais en remplaçant le N-(4-bromobutyl) phtalimide par le N-(4-bromobutyl)-2,4-dioxo-3-azaspiro[4,5] décane et en laissant 24 heures sous agitation, on obtient le produit attendu.

Rendement : 65 %

Résonance magnétique nucléaire du proton : ($CDCl_3$) :

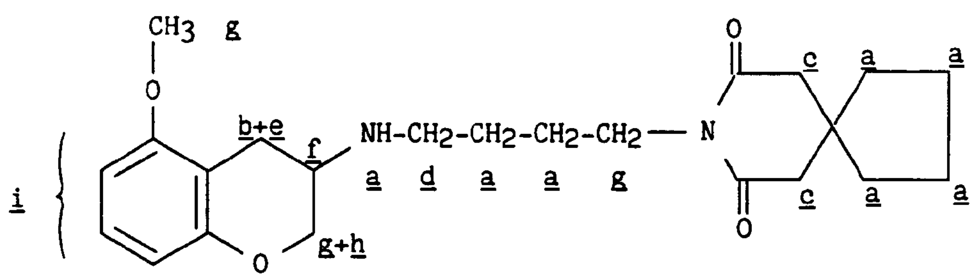

a δ entre 1,45 et 1,85 ppm (13H,m)

b δ = 2,46 ppm (1H,dd)

c δ = 2,59 ppm (4H,s)

d δ = 2,74 ppm (2H,m)

e δ = 2,92 ppm (1H,dd)

f δ = 3,05 ppm (1H,m)

g δ = 3,80 ppm (6H,m)

h δ = 4,13 ppm (1H,m)

i δ entre 6,41 et 7,04 ppm (3H,m)

**EXEMPLE 10 :**

**3-[4-[N-PROPYL-N-(5-METHOXY-CHROMAN-3-YL)AMINO]BUTYL]-2,4-DIOXO-3-AZASPIRO[4,5]DECANE**

En procédant comme dans l'exemple 2 mais en remplaçant le composé de l'exemple 1 par le composé de l'exemple 9, on obtient le produit attendu.

Rendement : 72 %

Résonance magnétique nucléaire du proton : ($CDCl_3$) :

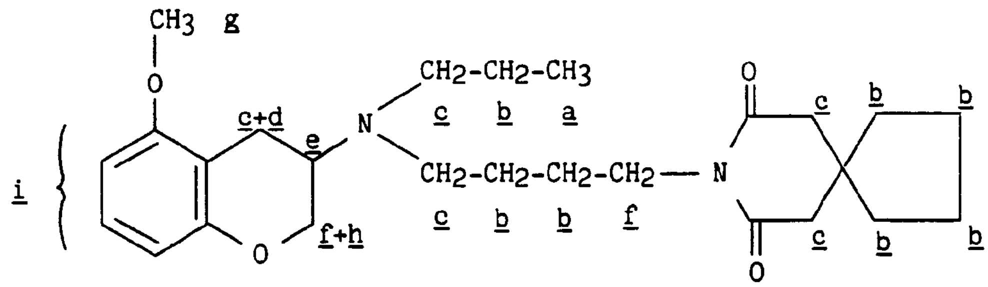

a δ = 0,87 ppm (3H,t)
b δ entre 1,45 et 1,75 ppm (14H,m)
c δ = 2,52 ppm (9H,m)
d δ = 2,84 ppm (1H,m)
e δ = 3,11 ppm (1H,m)
f δ = 3,73 ppm (3H,m)
g δ = 3,81 ppm (3H,s)
h δ = 4,22 ppm (1H,m)
i δ entre 6,39 et 7,02 ppm (3H,m)

**EXEMPLE 11 :**

**3-[4-[N-(5-METHOXY-CHROMAN-3-YL)AMINO]BUTYL]-2,4-DIOXO-3-AZABICYCLO[3.3.0] OCTANE**

En procédant comme dans l'exemple 1, mais en remplaçant le N-(4-bromobutyl) phtalimide par le N-(4-bromobutyl)-2,4-dioxo-3-azabicyclo[3.3.0] octane et en laissant 24 heures sous agitation, on obtient le produit attendu.

Rendement : 66 %

Résonance magnétique nucléaire du proton : ($CDCl_3$) :

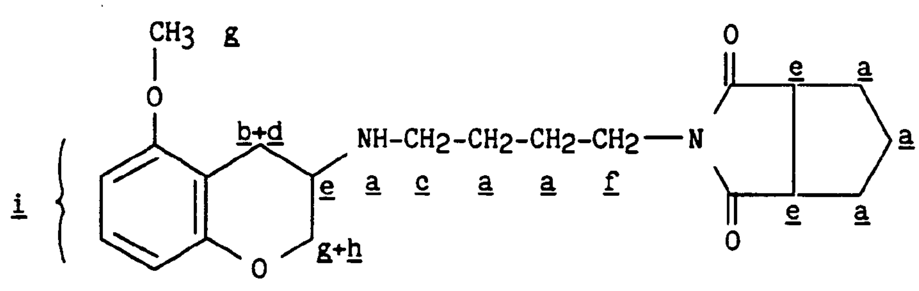

a δ entre 1,18 et 2,19 ppm (11H,m)
b δ = 2,54 ppm (1H,dd)
c δ = 2,57 ppm (2H,m)
d δ = 2,88 ppm (1H,m)
e δ = 3,29 ppm (3H,m)
f δ = 3,50 ppm (2H,t)

g δ entre 3,80 et 3,92 ppm (4H,m)
h δ = 4,35 ppm (1H,m)
i δ entre 6,43 et 7,06 ppm (3H,m)

**EXEMPLE 12 :**

**3-[4-[N-PROPYL-N-(5-METHOXY-CHROMAN-3-YL)AMINO]BUTYL]-2,4-DIOXO-3-AZABICYCLO[3.3.0] OCTANE**

En procédant comme dans l'exemple 2 mais en remplaçant le composé de l'exemple 2 par le composé de l'exemple 11, on obtient le produit attendu.

Rendement : 78 %

Résonance magnétique nucléaire du proton : ($CDCl_3$) :

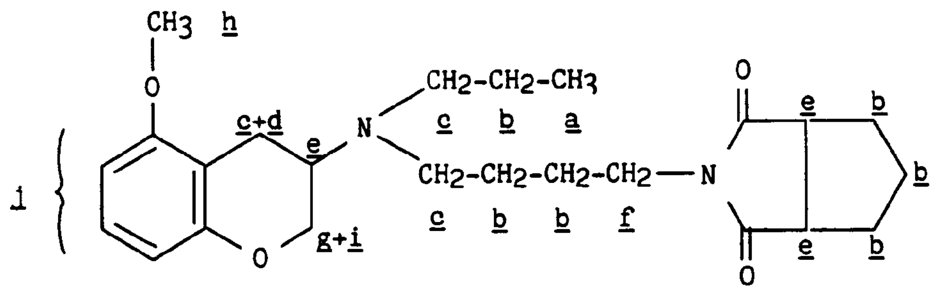

a δ = 0,88 ppm (3H,m)
b δ entre 1,18 et 2,18 ppm (12H,m)
c δ = 2,52 ppm (5H,m)
d δ = 2,84 ppm (1H,dd)
e δ = 3,12 ppm (3H,m)
f δ = 3,48 ppm (2H,t)
g δ = 3,74 ppm (1H,m)
h δ = 3,82 ppm (3H,s)
i δ = 4,21 ppm (1H,m)
j δ entre 6,41 et 7,06 ppm (3H,m)

**EXEMPLE 13 :**

**5-METHOXY-3-[N-PROPYL-N-(CYANOMETHYL) AMINO] CHROMANE**

Dans un ballon, 6,8 mmoles de 3-(N-propyl)amino-5-méthoxy-chromane (décrit dans le brevet EP 279150) sont dissoutes dans 10 ml de diméthylformamide en présence de 20 mmoles de chloroacétonitrile, de 20 mmoles de carbonate de potassium et d'une quantité catalytique d'iodure de potassium. Le mélange est mis sous agitation à 60°C pendant 24 heures. Après refroidissement, le solvant est évaporé et le brut réactionnel est extrait, après hydrolyse aqueuse, au dichlorométhane. Après lavage, séchage et évaporation de la phase organique, le produit attendu est obtenu après purification par chromatographie sur colonne de silice (solvant d'élution : dichlorométhane/méthanol : 99/1).

Rendement : 79 %

Résonance magnétique nucléaire du proton : ($CDCl_3$) :

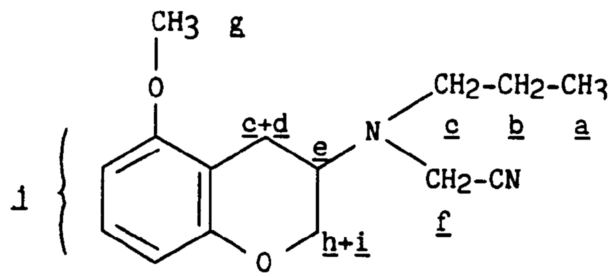

a δ = 0,92 ppm (3H,t)
b δ = 1,52 ppm (2H,m)
c δ = 2,68 ppm (3H,m)
d δ = 2,98 ppm (1H,dd)
e δ = 3,10 ppm (1H,m)
f δ = 3,70 ppm (2H,s)
g δ = 3,84 ppm (3H,s)
h δ = 3,91 ppm (1H,m)
i δ = 4,29 ppm (1H,m)
j δ entre 6,45 et 7,08 ppm (3H,m)

**EXEMPLE 14 :**

**5-METHOXY-3-[N-PROPYL-N-(P-AMINOETHYL) AMINO] CHROMANE**

Dans un ballon, 5,4 mmoles du composé préparé dans l'exemple 13 sont mises en solution dans 30 ml de tétrahydrofurane. 11 mmoles de $LiAlH_4$ sont additionnées lentement sous atmosphère inerte. Le mélange réactionnel est laissé sous agitation 30 minutes à température ambiante. 15 ml d'eau glacée sont ensuite additionnés au mélange refroidi dans de la glace. La phase organique est alors récupérée, séchée et évaporée et le produit attendu est obtenu après purification par chromatographie sur colonne de silice (solvant d'élution : dichlorométhane/méthanol : 99/1).

Rendement : 72 %

Résonance magnétique nucléaire du proton : ($CDCl_3$) :

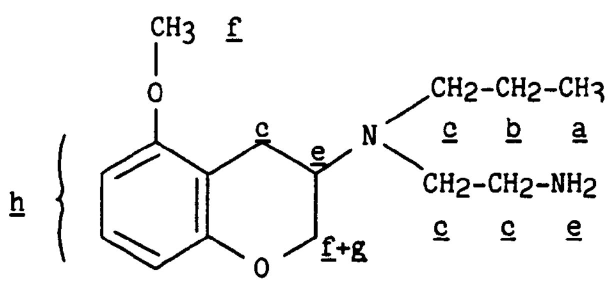

a δ = 0,89 ppm (3H,t)
b δ : 1,46 ppm (2H,m)
c δ entre 2,44 et 2,71 ppm (8H,m)
d δ = 3,14 ppm (1H,m)
e δ = 3,43 ppm (2H,massif)
f δ = 3,80 ppm (4H,m)
g δ = 4,25 ppm (1H,m)
h δ entre 6,40 et 7,04 ppm (3H,m)

**EXEMPLE 15 :**

**5-METHOXY-3-[N-PROPYL-N-[P-(4-TOLUENESULFONYLAMINO)ETHYL]AMINO]CHROMANE**

4 mmoles du composé préparé dans l'exemple 14 sont mises en solution dans 30 ml de dichlorométhane refroidi dans de la glace. 11,4 m moles de triéthylamine sont additionnées goutte à goutte puis 4 mmoles de chlorure de tosyle en solution dans du dichlorométhane. Le mélange est laissé sous agitation 30 minutes à température ambiante. Le solvant est alors évaporé et le produit attendu est obtenu après purification par chromatographie sur colonne de silice (solvant d'élution : dichlorométhane).

Rendement : 88 %

Résonance magnétique nucléaire du proton : ($CDCl_3$) :

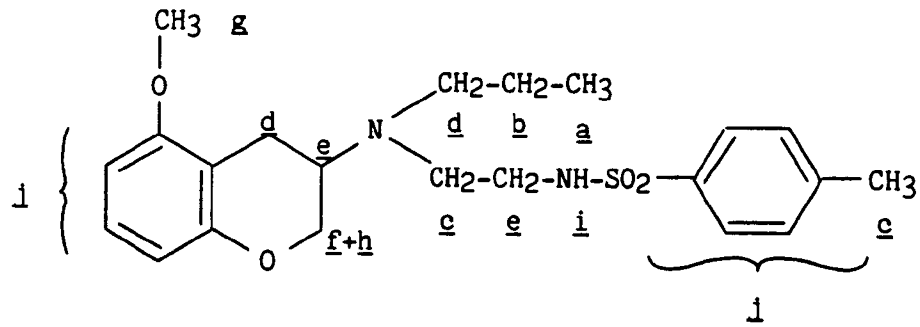

a δ = 0,76 ppm (3H,t)
b δ = 1,32 ppm (2H,m)
c δ = 2,36 ppm (5H,m)
d δ entre 2,48 et 2,52 ppm (4H,m)
e δ = 2,92 ppm (3H,m)
f δ = 3,69 ppm (1H,m)
g δ = 3,82 ppm (3H,s)
h δ = 4,10 ppm (1H,m)
i δ = 5,11 ppm (1H,massif)
j δ entre 6,43 et 7,73 ppm (7H,m)

**EXEMPLE 16 :**

**5-METHOXY-3-[N-[4-(4,4-DIMETHYL-2,6-DIOXO-PIPERIDIN-1-YL)BUTYL]AMINO]CHROMANE**

En procédant comme dans l'exemple 1 mais en remplaçant le N-(4-bromobutyl)phtalimide par la N-(4-bromo)butyl-4,4-diméthyl-2,6-dioxo-pipéridine et en laissant 24 heures sous agitation, on obtient le produit attendu.

Rendement : 58 %

Résonance magnétique nucléaire du proton : ($CDCl_3$) :

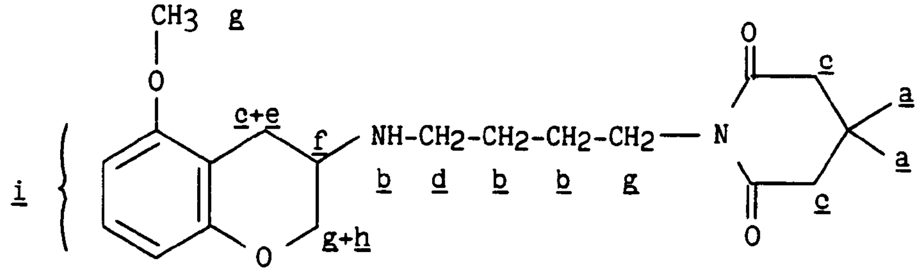

a δ = 1,06 ppm (6H,s)
b δ = 1,55 ppm (5H,m)
c δ = 2,40 ppm (5H,m)

d δ = 2,70 ppm (2H,m)
e δ = 2,90 ppm (1H,dd)
f δ = 3,09 ppm (1H,m)
g δ = 3,76 ppm (6H,m)
h δ = 4,17 ppm (1H,m)
i δ entre 6,40 et 7,06 ppm (3H,m)

**EXEMPLE 17 :**

**5-METHOXY-3-[N-PROPYL-N-[4-(4,4-DIMETHYL-2,6-DIOXO-PIPERIDIN-1-YL)BUTYL]AMINO]CHROMANE**

En procédant comme dans l'exemple 2 mais en remplaçant le composé de l'exemple 1 par le composé de l'exemple 16, on obtient le produit attendu.

Rendement : 77 %

Résonance magnétique nucléaire du proton : ($CDCl_3$) :

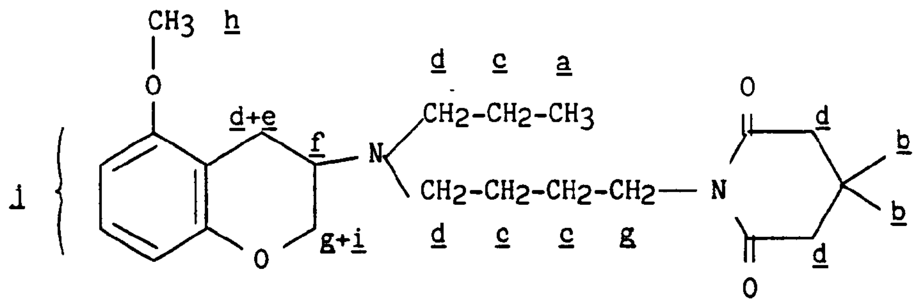

a δ = 0,88 ppm (3H,t)
b δ = 1,06 ppm (6H,s)
c δ = 1,48 ppm (6H,m)
d δ = 2,52 ppm (9H,m)
e δ = 2,84 ppm (1H,m)
f δ = 3,10 ppm (1H,m)
g δ = 3,75 ppm (3H,s)
h δ = 3,83 ppm (3H,s)
i δ = 4,26 ppm (1H,m)
j δ entre 6,42 et 7,05 ppm (3H,m)

**EXEMPLE 18 :**

**5-METHOXY-3-[N-PROPYL-N-[3-(4-TOLUENESULFONYLAMINO)PROPYL]AMINO]CHROMANE**

En procédant comme dans l'exemple 1 mais en remplacant le 3-amino-5-méthoxy-chromane par le 3-(N-propyl)amino-5-méthoxy-chromane et le N-(4-bromobutyl)phtalimide par le 1-bromo-3-(4-toluenesulfonyl) amino-propane, et en laissant pendant 72 heures sous agitation, on obtient le produit attendu.

Rendement : 65 %

Résonance magnétique nucléaire du proton : ($CDCl_3$) :

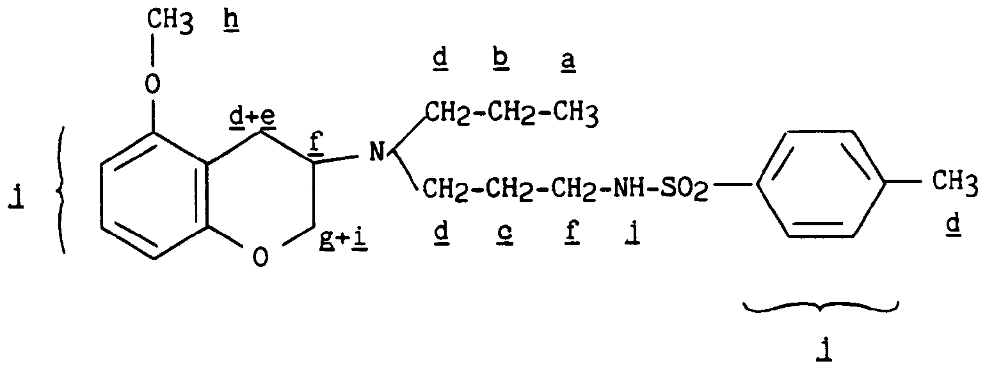

a δ = 0,88 ppm (3H,t)
b δ = 1,45 ppm (2H,m)
c δ = 1,63 ppm (2H,m)
d δ entre 2,40 et 2,70 ppm (8H,m)
e δ = 2,83 ppm (1H,dd)
f δ = 3,08 ppm (3H,m)
g δ = 3,75 ppm (1H,m)
h δ = 3,83 ppm (3H,s)
i δ = 4,17 ppm (1H,m)
j δ entre 6,25 et 7,75 ppm (8H,m)

**EXEMPLE 19 :**

**5-METHOXY-3-[N-PROPYL-N-(3-CYANOPROPYL)AMINO]CHROMANE**

En procédant comme dans l'exemple 13 mais en remplacant le chloroacétonitrile par le 4-bromobutyronitrile, on obtient le produit attendu.

Rendement : 73 %

Résonance magnétique nucléaire du proton : ($CDCl_3$) :

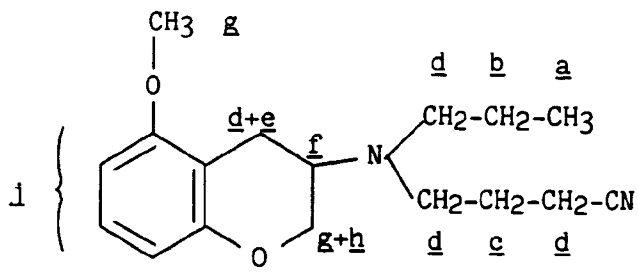

a δ = 0,91 ppm (3H,t)
b δ = 1,48 ppm (2H,m)
c δ = 1,80 ppm (2H,m)
d δ entre 2,42 et 2,78 ppm (7H,m)
e δ = 2,89 ppm (1H,dd)
f δ = 3,16 ppm (1H,m)
g δ = 3,82 ppm (4H,m)
h δ = 4,24 ppm (1H,m)
i δ entre 6,44 et 7,07 ppm (3H,m)

**EXEMPLE 20 :**

**5-METHOXY-3-[N-PROPYL-N-(4-AMINOBUTYL)AMINO]CHROMANE**

En procédant comme dans l'exemple 14 mais en remplacant le composé de l'exemple 13 par le composé de l'exemple 19, on obtient le produit attendu

Rendement : 69 %

Résonance magnétique nucléaire du proton : ($CDCl_3$) :

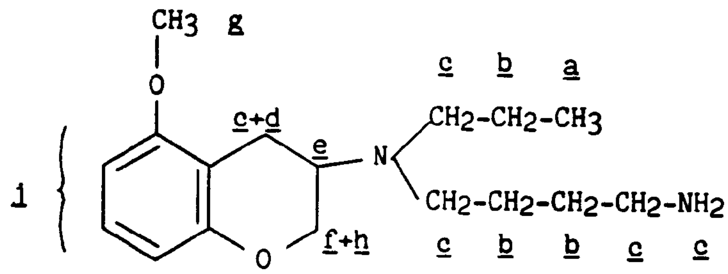

a δ = 0,81 ppm (3H,t)
b δ = 1,41 ppm (6H,m)
c δ entre 2,34 et 2,68 ppm (9H,m)
d δ = 2,80 ppm (1H,dd)
e δ = 3,07 ppm (1H,m)
f δ = 3,68 ppm (1H,m)
g δ = 3,76 ppm (3H,s)
h δ = 4,18 ppm (1H,m)
i δ entre 6,33 et 6,98 ppm (3H,m)

**EXEMPLE 21 :**

**5-METHOXY-3-[N-PROPYL-N-[4-(4-TOLUENESULFONYLAMINO)BUTYL]AMINO]CHROMANE**

En procédant comme dans l'exemple 15 mais en remplaçant le composé de l'exemple 14 par le composé de l'exemple 20, on obtient le produit attendu.

Rendement : 89 %

Résonance magnétique nucléaire du proton : ($CDCl_3$) :

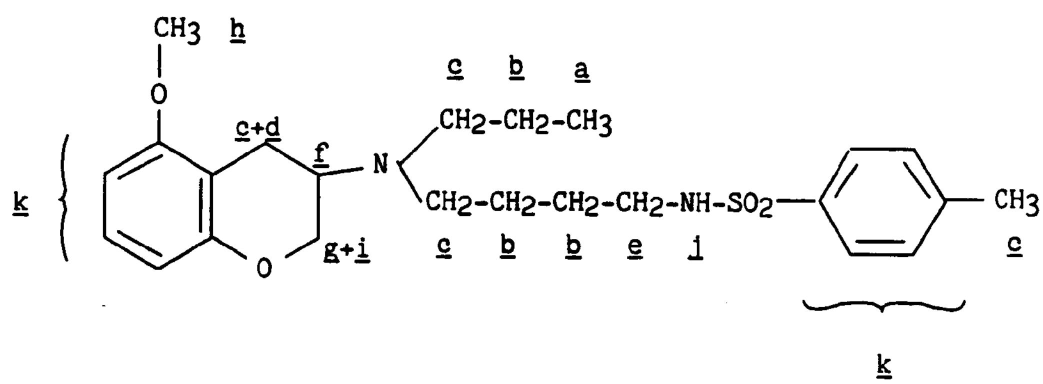

a δ = 0,84 ppm (3H,t)
b δ entre 1,35 et 1,57 ppm (6H,m)
c δ entre 2,39 et 2,56 ppm (8H,m)
d δ = 2,83 ppm (1H,dd)
e δ = 2,94 ppm (2H,q)
f δ = 3,06 ppm (1H,m)

g δ = 3,73 ppm (1H,m)
h δ = 3,82 ppm (3H,s)
i δ = 4,21 ppm (1H,m)
j δ = 5,51 ppm (1H,massif)
k δ entre 6,41 et 7,75 ppm (7H,m)

**EXEMPLE 22 :**

**3-[4-[N-PROPYL-N-(5-METHOXY-THIOCHROMAN-3-YL)AMINO]BUTYL]-2,4-DIOXO-3-AZASPIRO [4,5]DECANE**

En procédant comme dans l'exemple 10 mais en remplaçant le 3-amino-5-méthoxy-chromane par le 3-amino-5-méthoxy-thiochromane, on obtient le produit attendu.

**EXEMPLE 23 :**

**5-METHOXY 3-{N-[4-(1,1-DIOXIDO 3-OXO 2,3-DIHYDRO BENZISOTHIAZOL-2-YL)BUTYL]AMINO}CHROMANE**

En procédant comme dans l'exemple 1, mais en remplaçant le N(4-bromo butyl) phtalimide par le 1,1-dioxido 3-oxo 2,3-dihydro 2-(4-bromo butyl) benzisothiazole et en laissant 24 heures sous agitation, on obtient le produit du titre.

**EXEMPLE 24 :**

**5-METHOXY 3-{Nn PROPYL N-[4-(1,1-DIOXIDO 3-OXO 2,3-DIHYDRO BENZISOTHIAZOL-2-YL)BUTYL]AMINO}CHROMANE (OXALATE)**

En procédant comme dans l'exemple 2, mais en remplaçant le composé de l'exemple 1 par le composé obtenu dans l'exemple 23, on obtient le produit attendu que l'on salifie par l'acide oxalique.

Point de fusion : 85°C

Composition centésimale :

| | |
|---|---|
| Calculée | : C 57,17 H 5,71 N 4,98 S 5,93 |
| Trouvée | : C 56,92 H 5,88 N 5,11 S 5,84 |

**EXEMPLE 25 :**

**3-{2-[N-(5-METHOXY CHROMAN-3-YL)AMINO]ETHYL}3-AZASPIRO[4,5]DECANE 2,4-DIONE**

En procédant comme dans l'exemple 1, mais en remplaçant le N-(4-bromobutyl)phtalimide par le N-(2-bromobutyl)2,4-dioxo 3-azaspiro-[4,5]décane et en laissant 24 heures sous agitation, on obtient le produit du titre.

**EXEMPLE 26 :**

**3-{P-[Nn PROPYL N-(5-METHOXY CHROMAN-3 YL)AMINO]ETHYL}3-AZASPIRO [4,5] DECANE 2,4-DIONE (OXALATE)**

En procédant comme dans l'exemple 2, mais en remplaçant le composé de l'exemple 1 par le composé obtenu dans l'exemple 24, on obtient le produit attendu que l'on salifie par l'acide oxalique.

Point de fusion : 56°C

**EXEMPLE 27 :**

**3-[4-[Nn PROPYL N-(5-HYDROXY CHROMAN-3 YL)AMINO]BUTYL]3-AZASPIRO [4,5] DECANE 2,4-DIONE (OXALATE)**

Traiter le produit obtenu dans l'exemple 10 par le tribromure de base dans le chlorure de méthylène à une

température de -10°C. On obtient le produit du titre que l'on salifie.

Rendement : 60%
Point de fusion : 61°C
Composition centésimale :

| | |
|---|---|
| Calculée | : C 62,53 H 7,39 N 5,40 |
| Trouvée | : C 62,27 H 7,64 N 5,51 |

**EXEMPLE 28 :**

**OXALATE DE ⊕3-{4-[N-(5-METHOXY CHROMAN-3 YL)AMINO]BUTYL}2,4-DIOXO 3-AZASPIRO [4,5] DECANE**

En procédant comme dans l'exemple 9, mais en remplaçant le 3-amino 5-méthoxy chromane par le ⊕3-amino 5-méthoxy chromane (obtenu en traitant le 3-amino 5-méthoxy chromane par l'acétyl S ⊕valine en milieu méthanolique et filtration de la solution obtenue puis cristallisations successives) on obtient le produit du titre.

**EXEMPLE 29 :**

**OXALATE DE ⊕ 3-{4-[Nn PROPYL N-(5-METHOXY CHROMAN-3-YL)AMINO]BUTYL}2,4-DIOXO 3-AZASPIRO [4,5] DECANE**

En procédant comme dans l'exemple 10, mais en remplaçant le composé de l'exemple 9 par le composé de l'exemple 28, on obtient le produit du titre. Salification par l'acide oxalique.

Point de fusion : 68°C
Pouvoir rotatoire D : +50°

**EXEMPLE 30 :**

**⊖ 3-{4-[N-(5-METHOXY CHROMAN-3 YL)AMINO]BUTYL}2,4-DIOXO 3-AZASPIRO [4,5] DECANE**

En procédant comme l'exemple 28, mais en remplaçant le ⊕ 3-amino 5-méthoxy chromane par le ⊖ 3-amino 5-méthoxy chromane (obtenu en traitant le 3-amino 5-méthoxy chromanne par l'acétyl S⊖ valine en milieu méthanolique, filtration de la solution obtenue puis cristallisations successives), on obtient le produit du titre.

**EXEMPLE 31 :**

**OXALATE DE ⊖ 3-{ 4- [Nn PROPYL N-(5-METHOXY CHROMAN-3 YL)AMINO]BUTYL}2,4-DIOXO 3-AZASPIRO [4,5] DECANE (OXALATE)**

En procédant comme dans l'exemple 29, mais en remplaçant le composé de l'exemple 28 par le composé de l'exemple 30, on obtient le produit du titre.

Point de fusion : 68°C
Pouvoir rotatoire D : -50°

**EXEMPLE 32 :**

**⊕ 3-{4-[N-(5-METHOXY CHROMAN-3 YL)AMINO]BUTYL}2,4-DIOXO 3-AZABICYCLO [3.3.0] OCTANE**

En procédant comme dans l'exemple 11, mais en remplaçant le 3-amino 5-méthoxy chromane par le ⊕ 3-amino 5-méthoxy chromane, on obtient le produit du titre.

**EXEMPLE 33 :**

**⊕ 3-{4-[Nn PROPYL N-(5-METHOXY CHROMAN-3 YL) AMINO]BUTYL}2,4-DIOXO 3-AZABICYCLO [3.3.0] OCTANE (OXALATE)**

Procéder comme dans l'exemple 12, mais en remplaçant le composé de l'exemple 11 par le composé de l'exemple 32. Salifier par l'acide oxalique.

Pouvoir rotatoire $\alpha_D$ de l'amine libre : +57° (20 mg dans 3 ml de $CHCl_3$)

**EXEMPLE 34 :**

**⊖3-{4-[N-(5-METHOXY CHROMAN-3 YL)AMINO]BUTYL}2,4-DIOXO 3-AZABICYCLO [3.3.0] OCTANE**

En procédant comme dans l'exemple 11, mais en remplaçant le 3-amino 5-méthoxy chromane par le ⊖3-amino 5-méthoxy chromane, on obtient le produit du titre.

**EXEMPLE 35 :**

**OXALATE DE ⊖ 3-{4-[Nn PROPYL N-(5-METHOXY CHROMAN-3 YL)AMINO]BUTYL} 2,4-DIOXO 3-AZABICYCLO [3.3.0] OCTANE**

En procédant comme dans l'exemple 12, mais en remplaçant le composé de l'exemple 11 par le composé obtenu dans l'exemple 34, on obtient le produit du titre. Salifier par l'acide oxalique.

Pouvoir rotatoire $\alpha D$ de l'amine libre (20 mg, 3ml $CHCl_3$) : -57°

**EXEMPLE 36 :**

**5-METHOXY ⊕ 3-[Nn PROPYL N-(CYANOMETHYL)AMINO]CHROMANE**

En procédant comme dans l'exemple 13, mais en remplaçant le 3-amino 5-méthoxy chromane par le ⊕ 3-amino 5-méthoxy chromane, on obtient le produit du titre.

**EXEMPLE 37 :**

**5-METHOXY ⊕ 3-[Nn PROPYL N-(2-AMINO ETHYL)AMINO]CHROMANE**

En procédant comme dans l'exemple 14, mais en remplaçant le produit de l'exemple 13 par le produit obtenu dans l'exemple 36, on obtient le produit du titre.

**EXEMPLE 38 :**

**5-METHOXY ⊕ 3-[Nn PROPYL N-[2-(4-TOLUENE SULFONYLAMINO)ETHYL]AMINO] CHROMANE (OXALATE)**

En procédant comme dans l'exemple 15, mais en remplaçant le produit préparé dans l'exemple 14 par le produit obtenu dans l'exemple 37, on obtient le produit du titre. Salifier par l'acide oxalique.

Point de fusion : 132°C

Pouvoir rotatoire $\alpha D$ de l'amine libre : +44° (20 mg dans 3 ml de $CHCl_3$)

**EXEMPLE 39 :**

**5-METHOXY ⊖ 3-[Nn PROPYL N-(CYANOMETHYL)AMINO]CHROMANE**

En procédant comme dans l'exemple 13, mais en remplaçant le 3-amino 5-méthoxy chromane par le ⊖ 3-amino 5-méthoxy chromane, (obtenu dans l'exemple 30), on obtient le produit du titre.

**EXEMPLE 40 :**

**5-METHOXY ⊖ 3-[Nn PROPYL N-(2-AMINO ETHYL)AMINO]CHROMANE**

Procéder comme dans l'exemple 14, mais en remplaçant le produit de l'exemple 13 par le produit obtenu dans l'exemple 39, on obtient le produit du titre.

**EXEMPLE 41 :**

**5-METHOXY ⊖ 3-[Nn PROPYL N-[2-(4-TOLUENE SULFONYLAMINO)ETHYL]AMINO]CHROMANE (OXALATE)**

En procédant comme dans l'exemple 15, mais en remplaçant le produit préparé dans l'exemple 14 par le produit obtenu dans l'exemple 40, on obtient le produit du titre. Salifier par l'acide oxalique.

Point de fusion : 132°C

Pouvoir rotatoire $\alpha$D de l'amine libre : -44° (20 mg dans 3 ml de $CHCl_3$ )

**EXEMPLE 42 :**

**5-METHOXY ⊕ 3-[Nn PROPYL N-(3-CYANOPROPYL)AMINO ]CHROMANE**

En procédant comme dans l'exemple 19, mais en remplaçant le 3-amino 5-méthoxy chromane par le ⊕ 3-amino 5-méthoxy chromane, on obtient le produit du titre.

**EXEMPLE 43 :**

**5-METHOXY ⊕ 3-[Nn PROPYL N-(4-AMINO BUTYL)AMINO]CHROMANE**

En procédant comme dans l'exemple 20, mais en remplaçant le produit de l'exemple 19 par le produit obtenu dans l'exemple 42, on obtient le produit du titre.

**EXEMPLE 44 :**

**5-METHOXY ⊕ 3-[Nn PROPYL N-[4-(4-TOLUENE SULFONYLAMINO)BUTYL]AMINO]CHROMANE (OXALATE)**

En procédant comme dans l'exemple 21, mais en remplaçant le produit de l'exemple 20 par le produit de l'exemple 43, on obtient le produit du titre. Salifier par l'acide oxalique.

Point de fusion : 95°C

Pouvoir rotatoire $\alpha$D de l'amine libre : +52° (20 mg dans 3 ml de $CHCl_3$)

**EXEMPLE 45 :**

**5-METHOXY ⊖ 3-[Nn PROPYL N-(3-CYANOPROPYL)AMINO] CHROMANE**

En procédant comme dans l'exemple 19, mais en remplaçant le 3-amino 5-méthoxy chromane par le ⊖ 3-amino 5-méthoxy chromane, on obtient le produit du titre.

**EXEMPLE 46 :**

**5-METHOXY ⊖ 3-[Nn PROPYL N-(4-AMINO BUTYL)AMINO]CHROMANE**

Procéder comme dans l'exemple 20, mais en remplaçant le produit de l'exemple 19 par le produit de l'exemple 45, on obtient le produit du titre.

**EXEMPLE 47 :**

**5-METHOXY ⊖ 3-[Nn PROPYL N-[4-(4-TOLUENE SULFONYLAMINO)BUTYL]AMINO] CHROMANE (OXALATE)**

En procédant comme dans l'exemple 21, mais en remplaçant le produit de l'exemple 20 par le produit de l'exemple 46, on obtient le produit du titre. Salifier par l'acide oxalique.

Point de fusion : 95°C

Pouvoir rotatoire de l'amine libre : -52°C (20 mg dans 3 ml de $CHCl_3$)

**EXEMPLE 48 :**

**N{2-[N'n PROPYL N'(5-METHOXY CHROMANYL)AMINO]ETHYL}ACETAMIDE OXALATE**

Dans un ballon 17 mmoles du composé préparé dans l'exemple 13 sont mis en solution dans 95 ml de tétrahydrofuranne. 34 mmoles de $LiAlH_4$ sont additionnés lentement sous atmosphère inerte. Le mélange réactionnel est laissé sous agitation 30 minutes à température ambiante. 20 ml d'acétate d'éthyle sont ensuite additionnés au mélange refroidi dans de la glace. Maintenir l'agitation dix minutes environ puis ajouter goutte à goutte 20 ml d'eau. Séparer la phase organique, sécher sur sulfate de magnésium. Evaporer le solvant sous pression réduite et purifier l'échantillon par chromatographie sur colonne à l'aide d'un mélange chlorure de méthylène méthanol. Salifier par l'acide oxalique.

Rendement : 75%

Point de fusion : 54°C

Caractéristiques spectrales (base libre) :

Infrarouge : 3280 $cm^{-1}$ ($\nu$NH)

Résonance Magnétique Nucléaire :

$\delta$ = 1,96 ppm, singulet, 3H, CO-$\underline{CH_3}$

**EXEMPLE 49 :**

**3-[4-[N-(5-METHOXY BENZOTHIOPYRAN-3-YL)AMINO]BUTYL]2,4-DIOXO 3- AZASPIRO [4,5]DECANE**

En procédant comme dans l'exemple 9, mais en remplaçant le 3-amino 5-méthoxy chromane par la 3-amino 3,4-dihydro 5-méthoxy [2H] 1-benzothiopyran décrit dans la demande de brevet EP 0 222 996, on obtient le produit du titre.

Caractéristiques spectrales :

$N(CO\text{-}CH_2)_2$ : d : 2,59 ppm singulet

**EXEMPLE 50 :**

**5-METHOXY 3-[Nn PROPYL N-(CYANOMETHYL)AMINO]3,4-DIHYDRO [2H]1-BENZOTHIOPYRANNE**

En procédant comme dans l'exemple 13, mais en remplaçant le 3-(Nn propylamino)5-méthoxy chromane par le 3-(Nn propylamino)5-méthoxy 1-benzothiopyranne, on obtient le produit du titre.

**EXEMPLE 51 :**

**5-METHOXY 3-[Nn PROPYL N-(2-AMINO ETHYL)AMINO]3,4-DIHYDRO [2H]1-BENZOTHIOPYRANNE**

Procéder comme dans l'exemple 14 en remplaçant le produit de l'exemple 13 par le produit obtenu dans l'exemple 50, on obtient le produit du titre.

**EXEMPLE 52 :**

**5-METHOXY 3-[Nn PROPYL N-[2-(4-TOLUENE SULFONYLAMINO)ETHYL]AMINO]3,4-DIHYDRO [2H]1-BENZOTHIOPYRANNE**

Procéder comme dans l'exemple 15 en remplaçant le produit de l'exemple 14 par le composé obtenu dans

l'exemple 51.

Caractéristiques spectrales :

Résonance Magnétique Nucléaire $^1H$ ($CDCl_3$)

$\delta$ = 3,82 ppm, singulet, 3H, $C_6H_4$-$CH_3$

EXEMPLE 53 :

5-METHOXY 3-[Nn PROPYL N-(3-CYANOPROPYL)AMINO] 3,4-DIHYDRO [2H]1-BENZOTHIOPYRANNE

En procédant comme dans l'exemple 50, mais en remplaçant le chloroacétonitrile par le 4-bromobutyronitrile, on obtient le produit attendu.

EXEMPLE 54 :

5-METHOXY 3-[Nn PROPYL N-(4-AMINOBUTYL)AMINO]3,4-DIHYDRO [2H]1-BENZOTHIOPYRANNE

En procédant comme dans l'exemple 51, mais en remplaçant le composé de l'exemple 50 par le composé de l'exemple 53, on obtient le produit du titre.

EXEMPLE 55 :

5-METHOXY 3-[Nn PROPYL N-[4-(4-TOLUENE SULFONYLAMINO)BUTYL]AMINO]3,4-DIHYDRO [2H]1-BENZOTHIOPYRANNE

En procédant comme dans l'exemple 52, mais en remplaçant le composé de l'exemple 51 par le composé de l'exemple 54, on obtient le produit du titre.

Caractéristiques spectrales :

Résonance Magnétique Nucléaire $^1H$ ($CDCl_3$)

$\delta$ = 3,82 ppm, singulet, 3H, $C_6H_4$-$CH_3$

EXEMPLE 56 :

5-METHOXY 3-[Nn PROPYL N-[3-(4-TOLUENE SULFONYLAMINO)PROPYL]AMINO] 3,4-DIHYDRO [2H]1-BENZOTHIOPYRANNE

En procédant comme dans l'exemple 18, mais en remplaçant le 3-(N-propylamino)5-méthoxychromane par le 3-(N-propylamino)5-méthoxy 3,4-dihydro [2H]1-benzothiopyranne, on obtient le produit du titre.

EXEMPLE 57 :

N{2-[Nn PROPYL N-(5-METHOXY CHROMANYL)AMINO]ETHYL}ISOBUTYRAMIDE

Procéder comme dans l'exemple 48, mais en remplaçant l'acétate d'éthyle par l'isobutyrate d'éthyle.

En remplaçant dans les exemples précédents le 3-amino 5-méthoxy chromane par respectivement les 3-amino 6-méthoxy chromane, 3-amino 7-méthoxy chromane ou 3-amino 8-méthoxy chromane ou en remplaçant le 3-amino 5-méthoxy 3,4-dihydro [2H]1-benzothiopyranne par le 3-amino 6-méthoxy 3,4-dihydro [2H]1-benzothiopyranne par le 3-amino 7-méthoxy chromane ou par le 3-amino 8-méthoxy chromane, on obtient les isomères des exemples n° 1 à 56 respectivement méthoxylés en position 6, 7 ou 8.

ETUDE PHARMACOLOGIQUE DES DERIVES DE L'INVENTION

EXEMPLE 58 :

TESTS D'AFFINITE IN VITRO POUR LES RECEPTEURS 5-$HT_{1A}$, $D_2$ ET $\alpha_2$

Les tests d'affinité in vitro pour les récepteurs 5-$HT_{1A}$, $D_2$ et $\alpha_2$ ont été réalisés selon des techniques classiques de binding.

Les résultats de ces études montrent que les composés de l'invention possèdent des $K_{0,5}$ de l'ordre de

$10^{-10}$ M vis à vis des récepteurs $5\text{-}HT_{1A}$. Cette très grande affinité est complétée par une très grande sélectivité. En effet, le rapport des affinités $5\text{-}HT_{1A}/D_2$ est égal à $10^2$ ; celui des affinités $5\text{-}HT_{1A}/\alpha_2$ est égal à 104.

**EXEMPLE 59 :**

**ETUDE DE LA TOXICITE AIGUE**

La toxicité aiguë a été effectuée après administration orale à des lots de cinq souris (20 ± 2 grammes) de doses croissantes (0,1 - 0,25 -0,50 - 0,75 - 1 g/$kg^{-1}$) des produits de l'invention.
Les animaux ont été observés à intervalles réguliers au cours de la première journée et quotidiennement pendant les deux semaines suivants le traitement. Il apparaît que les composés de l'invention sont totalement atoxiques. Aucun décès n'est observé après administration d'une dose de 1 g.$kg^{-1}$. On ne constate pas de trouble après administration de cette dose.

**EXEMPLE 60 :**

**ACTIVITE AGONISTE $5HT_{1A}$**

**TEST DE LA RETRACTION DE LA LEVRE INFERIEURE CHEZ LE RAT**

Le protocole employé est celui décrit par Berendsen et Collaborateurs (Berendsen H.H.G, Jenck F ; Broekkamp C.L.E. Pharmacology Biochemistry and Behavior 1989, 33, 821-827).
Les composés de l'invention sont administrés aux rats qui sont placés immédiatement individuellement dans des cages de plastique transparent (20 × 10 × 10 cm).
La rétraction de la lèvre inférieure est notée toutes les quinze minutes durant les trois heures qui suivent l'injection de la façon suivante :
0 = incisives inférieures non visibles
1 = incisives inférieures partiellement visibles
2 = incisives inférieures totalement visibles
Les scores sont totalisés pour chaque rat et l'effet obtenu est exprimé en pourcentage du maximum possible à trois heures.
Six rats sont utilisés par groupe. L'expérience est réalisée simultanément sur un témoin aveugle. Les produits ont été administrés aux doses de 0,5, 2 et 8 mg.$kg^{-1}$ par voie sous cutanée.
Ce test montre que les composés de l'invention ont une intense activité agoniste $5HT_{1A}$.

**EXEMPLE 61 :**

**ETUDE DE L'ACTIVITE ANTIDEPRESSIVE**

**EFFET SUR LES ECHECS D'ECHAPPEMENT**

L'étude des produits est réalisée sur le modèle de "learned helplessness" ou renoncement appris qui consiste à induire chez l'animal, par une série d'évènements aversifs incontrôlables, un déficit lors des tâches d'évitement ultérieures (Martin et al., 1986, Pharmacol. Biochem. Behav., 24, 177-181).
Nous utilisons des rats mâles Wistar A.F. provenant des élevages homogènes CERJ, d'un poids compris entre 180 et 200 grammes. Les animaux sont maintenus à l'animalerie une semaine avant le test, dans des boîtes en plastique, par groupes de 10, à une température ambiante de 21°C ∓ 1°C, avec libre accès à l'eau et à la nourriture.
Les animaux sont isolés dans des boîtes de petites dimensions et sont soumis à 60 chocs électriques inévitables (0,8 mA toutes les minutes ∓ 15 secondes). Un groupe de rats témoins ne reçoit pas de chocs électriques. La capacité des animaux à réaliser un apprentissage d'évitement (shuttle-box) est appréciée 48 heures après et pendant 3 jours consécutifs. Lors des séances d'apprentissage, les animaux subissent 2 essais par minute pendant 15 minutes. Le nombre d'échecs d'échappement (escape failure) est noté pour chaque rat.
Les animaux sont traités (i.p.; 0.5 ml/100 g) 6 heures après les chocs inévitables et 4 jours de suite, le matin 30 minutes avant la séance de shuttle-box et le soir entre 18 et 19h.
Les produits étudiés sont mis en solution dans l'eau distillée.
Les produits étudiés sont administrés aux doses 0.25 mg.kg/jour.
Le test prouve que les produits de l'invention diminuent significativement le nombre d'échecs d'échappement ce qui traduit une activité de type antidépressive.

**EXEMPLE 62 :**

**ACTIVITE ANTIHYPERTENSIVE**

Les animaux sont acclimatés durant une période de six jours avant le début de l'étude.
Au début de l'expérience les rats sont anesthésiés avec 1000 $mg.kg^{-1}$ d'uréthanne administré par voie intrapéritonéale. Dans la veine jugulaire est introduit un cathéter.
Un cathéter relié à un enregistreur de précision est placé dans l'artère carotide. On laisse s'écouler un intervalle de 10 minutes pour permettre la stabilisation de la tension artérielle avant la prise de la première mesure.
Dans un premier temps, on administre le solvant par voie intraveineuse à tous les animaux, la pression artérielle est enregistrée pendant 30 minutes après l'administration et des lectures de pression artérielle sont effectuées 10, 20 et 30 minutes après l'administration.
Les composés de l'invention sont administrés en solution saline également par voie intraveineuse. L'enregistrement de la tension artérielle réalisé pendant 30 minutes et des mesures de pression sont effectuées 10, 20 et 30 minutes après l'administration. Les produits de l'invention entraînent une diminution significative de la pression artérielle.

**EXEMPLE 63 :**

**ETUDE DE L'ACTIVITE ANXIOLYTIQUE - TEST DU CONFLIT CHEZ LE PIGEON**

Six pigeons White Carneaux expérimentalement vierges sont entrainés à picorer une clé de plexiglas qui est transilluminée par des lumières rouges ou blanches. La clé réponse est montée sur la paroi frontale de la chambre expérimentale. Les pigeons sont amenés à 85% de leur poids normal avant le début de l'expérimentation laquelle est menée en utilisant la méthode des approximations successives (Frester 1953). Au départ, chaque picorage de la clé (illuminée avec une lumière rouge ou blanche) qui dépasse une force de 0,15N permet l'accès à un mélange de céréales par l'intermédiaire d'un distributeur automatique situé sous la clé. Après plusieurs jours les céréales ne sont plus délivrées qu'au trentième picorage sur la clé. Lorsque cette réponse au 30ème coup est obtenue, et qu'elle survient de façon régulière, permettant la délivrance de nourriture, la couleur de la lumière de la clé est alternée toutes les trois minutes (du blanc au rouge et vice-versa). La mesure du taux de réponse au 30ème coup reste en vigueur pendant chaque phase lumineuse. Pendant cette phase et pour la durée de l'expérience une session quotidienne se compose de 5 cycles de 3 minutes ce chaque séquence lumineuse, ces séquences étant séparées par une pause de 30 secondes pendant laquelle les clés lumineuses sont éteintes et les réponses n'ont pas de conséquence. Par conséquent, une séquence dure environ 35 à 40 minutes. Lorsque ces taux de réponses sont stables et identiques pour chaque couleur pendant une période de 5 jours (cela nécessite 3 à 4 semaines) chaque 30ème réponse dans l'une des phases colorées entraîne simultanément une libération de nourriture et un choc électrique bref (200 milli secondes) et modéré (1,3 mA) délivré par des électrodes placées au niveau des os pubiens. Le taux de réponses est réduit dans un premier temps puis retourne au niveau initial.

L'administration des produits de l'invention est réalisée après l'obtention d'un taux de réponse stable sur une période de 5 jours.

L'administration intramusculaire des produits de l'invention à une dose de 0,3 $mg.kg^{-1}$ entraîne une augmentation significative de réponses suivies ou non de chocs électriques prouvant l'activité anxiolytique de ces produits.

**EXEMPLE 64 :**

**COMPOSITION PHARMACEUTIQUE**

Comprimés dosés à 10 mg de ⊖3-[4-[N-propyl-N-(5-méthoxy-chroman-3-yl) amino] butyl]-2,4-dioxo-3-azaspiro[4,5] décane.

Formule pour 1000 comprimés :

| | |
|---|---|
| Θ3-[4-[N-propyl-N-5-méthoxy-chroman-3-yl) amino] butyl]-2,4-dioxo-3-azaspiro[4,5] décane............... | 10 g |
| Amidon de blé........................................ | 15 g |
| Amidon de maïs....................................... | 15 g |
| Lactose.............................................. | 65 g |
| Stéarate de magnésium................................ | 2 g |
| Silice............................................... | 1 g |
| Hydroxypropyl cellulose.............................. | 2 g |

## Revendications

**Revendications pour les Etats contractants suivants : AT, LI, NL, BE, DE, FR, IT, SE, CH, DK, GB, LU**

**1.** Composés de formule générale (I) :

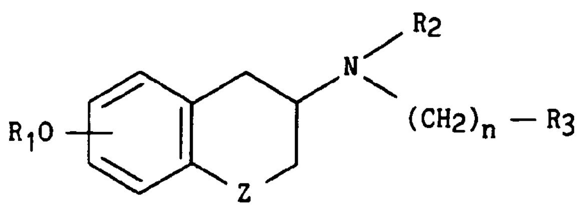

(I)

dans laquelle :

- Z représente un atome d'oxygène ou un atome de soufre,
- $R_1$ représente un atome d'hydrogène, ou un groupement alkyle ($C_1$-$C_6$) linéaire ou ramifié,
- $R_2$ représente un atome d'hydrogène ou un groupement alkyle ($C_1$-$C_6$) linéaire ou ramifié,
- n est un nombre entier compris entre 1 et 6,
- $R_3$ représente un groupement nitrile, un groupement amino éventuellement substitué par :
  - . un groupement acyle linéaire ou ramifié comprenant de 2 à 7 atomes de carbone
  - . un groupement alkylsulfonyl,
  - . un groupement arylsulfonyl éventuellement substitué par un groupement alkyle, alkoxy, hydroxy ou un atome d'halogène,
  - . un groupement alkyle ($C_1$-$C_6$) linéaire ou ramifié,
- ou $R_3$ représente l'un quelconque des groupements suivants :

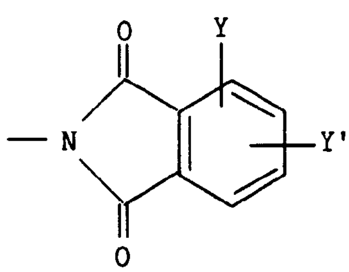

,

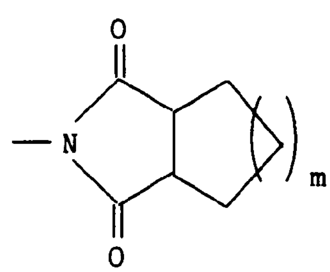

,

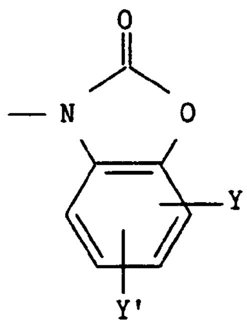 ,

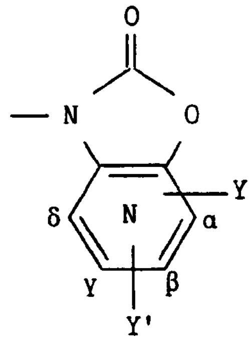 ,

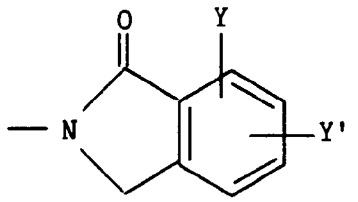 ,

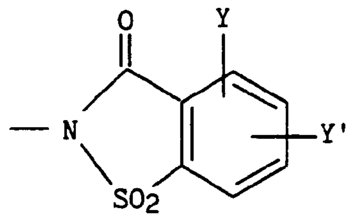 ,

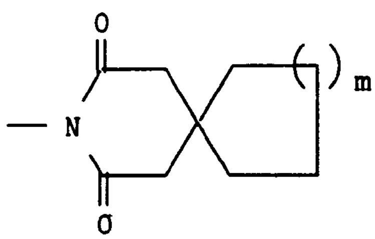 ,

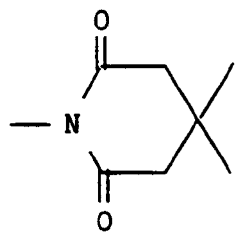 ,

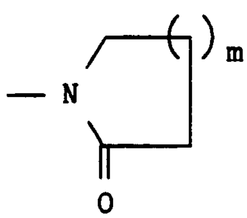

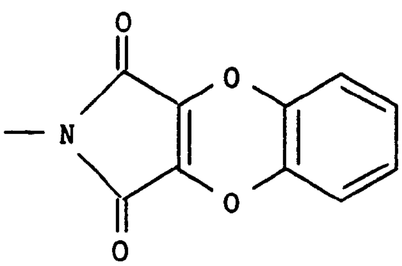 ,

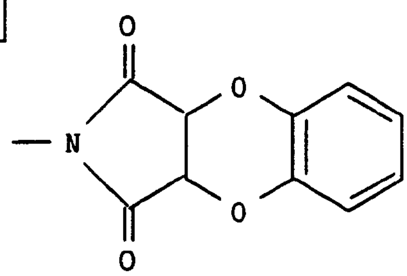 ,

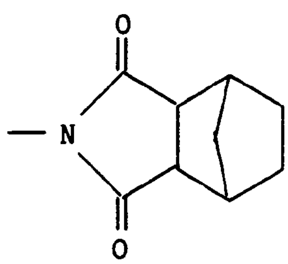 ,

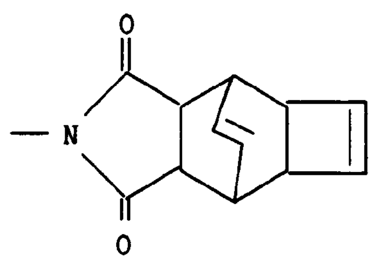

dans lesquels :

. Y et Y′ identiques ou différents représentent un atome d'hydrogène, un atome d'halogène, un groupement alkyle, alkoxy ou hydroxy,

. m est un nombre entier égal à 1 ou 2,

. et l'azote du cycle pyridine se situe en position α, β, γ ou δ de la jonction du cycle, leurs énantiomères, diastéréoisomères et épimères ainsi que leurs sels d'addition à un acide pharmaceutiquement acceptable.

2. Composés selon la revendication 1 tels que $R_1$ est un groupement méthyle.

3. Composés selon la revendication 1 tels que $R_2$ est un groupement n-propyle.

4. Composés selon la revendication 1 tels que $OR_1$ se trouve en position 5.

5. Composés selon la revendication 1 tels que $R_2$ est un atome d'hydrogène.

6. Composé selon la revendication 1 qui est le 3-[4-[N-propyl-N-(5-méthoxy-chroman-3-yl) amino] butyl]-2,4-dioxo-3-azaspiro[4,5] décane, ses isomères ainsi que ses sels d'addition à un acide pharmaceutiquement acceptable.

7. Composé selon la revendication 1 qui est le 3⊖-[4-[Nn-propyl-N-(5-méthoxy-chroman-3-yl) amino] butyl]-2,4-dioxo-3-azaspiro[4,5] décane, ainsi que ses sels d'addition à un acide pharmaceutiquement acceptable.

8. Composé selon la revendication 1 qui est le 3⊕-[4-[Nn-propyl-N-(5-méthoxy-chroman-3-yl) amino] butyl]-2,4-dioxo-3-azaspiro[4,5] décane, ainsi que ses sels d'addition à un acide pharmaceutiquement acceptable.

9. Composé selon la revendication 1 qui est le 5-méthoxy-3-[N-propyl-N-[2-(4-toluène-sulfonylamino) éthyl] amino] chromane, ses isomères ainsi que ses sels d'addition à un acide pharmaceutiquement acceptable.

10. Composé selon la revendication 1 qui est le 5-méthoxy-3⊕-[N-propyl-N-[2-(4-toluène-sulfonylamino) éthyl] amino] chromane, ainsi que ses sels d'addition à un acide pharmaceutiquement acceptable.

11. Composé selon la revendication 1 qui est le 5-méthoxy-3⊖-[N-propyl-N-[2-(4-toluène-sulfonylamino) éthyl] amino] chromane, ainsi que ses sels d'addition à un acide pharmaceutiquement acceptable.

12. Composé selon la revendication 1 qui est le 5-méthoxy-3[Nn-propyl-N-[4-(4-toluène-sulfonylamino) butyl] amino] chromane, ses isomères ainsi que ses sels d'addition à un acide pharmaceutiquement acceptable.

13. Composé selon la revendication 1 qui est le 5-méthoxy-3⊕[Nn-propyl-N-[4-(4-toluène-sulfonylamino) butyl] amino] chromane, ainsi que ses sels d'addition à un acide pharmaceutiquement acceptable.

14. Composé selon la revendication 1 qui est le 5-méthoxy-3⊖[Nn-propyl-N-[4-(4-toluène-sulfonylamino) butyl] amino]chromane, ainsi que ses sels d'addition à un acide pharmaceutiquement acceptable.

15. Composé selon la revendication 1 qui est le 3-[4-[N-propyl-N-(5-méthoxy chroman-3-yl)amino]butyl]2,4-dioxo 3-azabicyclo [3.3.0] octane, ses isomères ainsi que ses sels d'addition à un acide pharmaceutiquement acceptable.

16. Composé selon la revendication 1 qui est le 3⊕-[4-[N-propyl-N-(5-méthoxy chroman-3-yl)amino]butyl-]2,4-dioxo 3-azabicyclo [3.3.0] octane, ainsi que ses sels d'addition à un acide pharmaceutiquenent acceptable.

17. Composé selon la revendication 1 qui est le 3⊖-[4-[N-propyl-N-(5-méthoxy chroman-3-yl)amino]butyl]2, 4-dioxo 3-azabicyclo [3.3.0] octane, ainsi que ses sels d'addition à un acide pharmaceutiquenent acceptable.

18. Procédé de préparation des composés de formule (I) caractérisé en ce que l'on utilise comme matière première :
ou bien
a un composé de formule (II) :

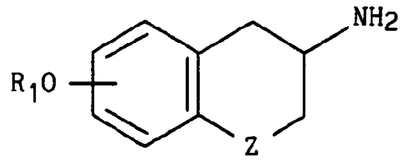

(II)

dans laquelle $R_1$ et Z ont la même signification que dans la formule (I), que l'on fait réagir sur un dérivé de formule (III) :

$R_3$-$(CH_2)_n$-X (III)

dans laquelle $R_3$ et n ont la même signification que dans la formule (I) et X est un atome d'halogène pour conduire à un dérivé de formule (I/a), cas particulier des dérivés de formule (I) :

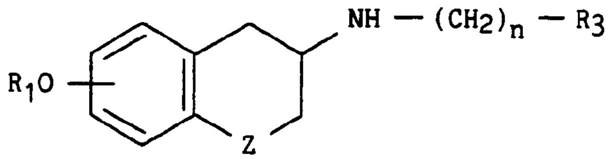

(I/a)

dans laquelle $R_1$, $R_3$, Z et n ont la même signification que dans la formule (I) que l'on soumet éventuellement à l'action d'un dérivé de formule (IV) :

$R'_2$-X (IV)

dans laquelle $R'_2$ représente un groupement alkyle (C1-C6) linéaire ou ramifié et X représente un atome d'halogène,
pour conduire à un dérivé de formule (I/b), cas particulier des dérivés de formule (I) :

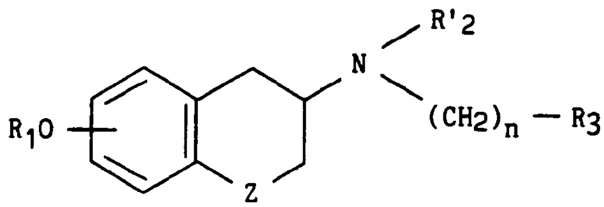

(I/b)

dans laquelle $R_1$, Z, $R'_2$, $R_3$ et n sont définis comme précédemment,
ou bien
b un composé de formule (V) :

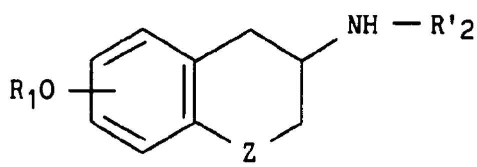

(V)

dans laquelle $R_1$, Z et $R'_2$ ont les mêmes significations que précédemment, que l'on fait réagir :

- soit sur un composé de formule (III) :

  $R_3$-$(CH_2)_n$-X (III)

  dans laquelle $R_3$, n et X sont définis comme précédemment, pour conduire à un dérivé de formule (I/b), cas particulier des dérivés de formule (I) :

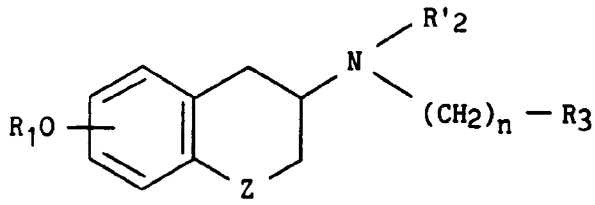

(I/b)

dans laquelle $R_1$, Z, $R'_2$, $R_3$ et n sont définis comme précédemment,

- soit sur un composé de formule (III/a) :

$NC-(CH_2)_m-X$ (III/a)

dans laquelle X a la même signification que précéderaient et m est un nombre entier compris entre 1 et 3,
pour conduire à un dérivé de formule (I/c), cas particulier des dérivés de formule (I) :

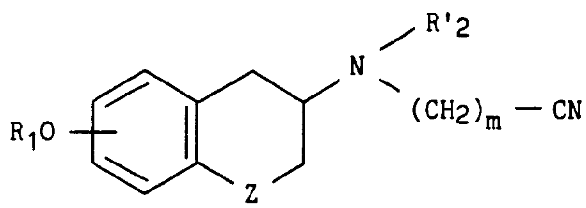

(I/c)

dans laquelle $R_1$, Z, $R'_2$ et m sont définis comme précédemment, que l'on réduit de façon catalytique en présence de nickel de Raney et d'ammoniac, ou chimiquement par l'hydrure de lithium/aluminium ou par le sodium dans l'alcool,
pour conduire au dérivé de formule (I/d), cas particulier des dérivés de formule (I) :

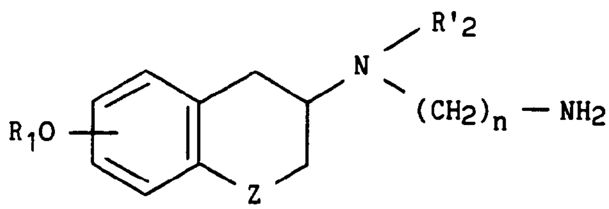

(I/d)

dans laquelle $R_1$, Z, $R'_2$ et n sont définis comme précédemment, que l'on fait réagir :

. ou bien sur un composé de formule (VI/A) :

$R_{4A}-SO_2-X$ (VI/A)

dans laquelle $R_{4A}$ est un groupement alkyle ou un groupement aryle éventuellement substitue par un groupement alkyle et X est un atome d'halogène,
pour conduire à un derivé de formule (I/e), cas particulier des dérivés de formule (I) :

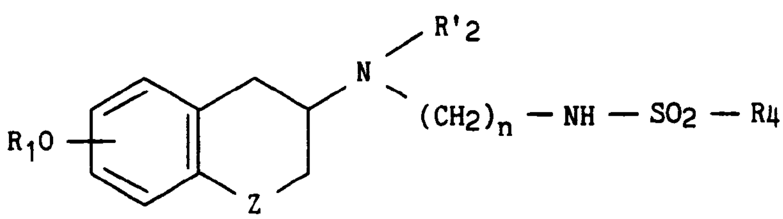

(I/e)

dans laquelle $R_1$, Z, $R'_2$, $R_4$, n sont définis comme précédemment,

. ou bien sur un composé de formule (VI/B) :

$R_{4B}-CO-T$ (VI/B)

dans laquelle R4B représente un groupement alkyle linéaire ou ramifié de 1 à 6 atomes de carbone et T un groupe partant choisi parmi halogène, ou alkoxy inférieur ou un composé de formule (VI/C) :

$R_{4B}$-CO-O-CO-$R_{4B}$ (VI/C)

dans laquelle $R_{4B}$ a la même définition que précédemment pour conduire à un composé de formule (I/F) :

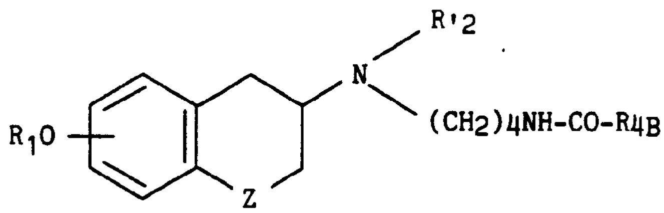

(I/F)

dans laquelle $R_1$, Z, $R'_2$, $R_{4B}$ et n ont la même définition que précédemment, cas particulier des dérivés de formule (I), pour lesquels $R_3$ représente un groupement amino substitué par un groupement acyle linéaire ou ramifié de deux à sept atomes de carbone,

ou bien

c un composé de formule (VII) :

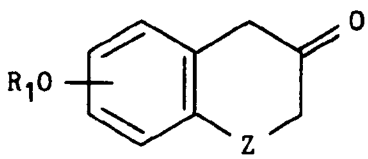

(VII)

dans laquelle $R_1$ et Z ont la même signification que dans la formule (I), que l'on soumet à une amination réductrice en présence d'un dérivé de formule (VIII) :

$R_2$-NH-$(CH_2)_n$-$R_3$ (VIII)

dans laquelle $R_2$, $R_3$ et n sont définis comme dans la formule (I), pour conduire à un composé de formule (I), qui, lorsque $R_2$ représente un atome d'hydrogène peut être soumis à l'action d'un dérivé de formule (IV) :

$R'_2$-X (IV)

dans laquelle $R'_2$ et X sont définis comme precédemment,

pour conduire à un dérive de formule (I/b), cas particulier des dérivés de formule (I) :

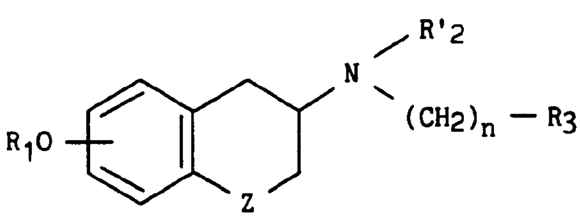

(I/b)

dans laquelle $R_1$, Z, $R'_2$, $R_3$ et n sont définis comme précédemment,

ou bien encore

d un composé de formule (IX) :

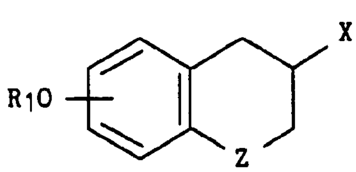

(IX)

dans laquelle $R_1$ et Z ont la même signification que dans la formule (I) et X est un atome d'halogène

que l'on soumet à l'action d'un dérivé de formule (X) :

$H_2N-(CH_2)_n-R_3$ (X)

dans laquelle n et $R_3$ sont définis comme precédemment,pour conduire à un dérivé de formule (I/a), cas particulier des dérives de formule (I) :

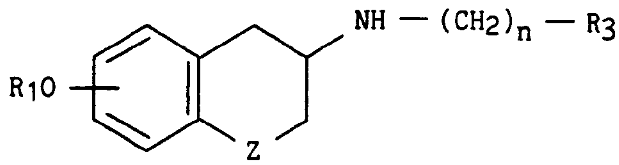

(I/a)

dans laquelle $R_1$, Z, n et $R_3$ sont définis comme précédemment, que l'on soumet éventuellement à l'action d'un dérivé de formule (IV)

$R'_2$-X (IV)

dans laquelle $R'_2$ et X sont définis comme précédemment,
pour conduire à un dérivé de formule (I/b), cas particulier des dérives de formule (I) :

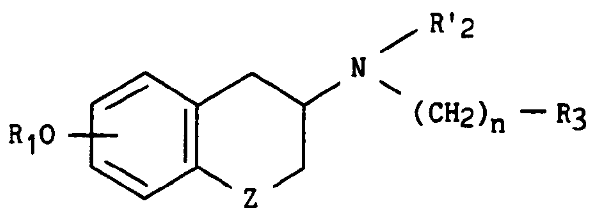

(I/b)

dans laquelle $R_1$, Z, $R'_2$, $R_3$ et n sont définis comme précédemment,
dérivés de formule (I/a), (I/b), (I/c), (I/d) et (I/e) dont on sépare éventuellement les isomères selon une technique classique de séparation, que l'on purifie, par une technique classique de purification et que l'on transforme, si on le désire, en leurs sels d'addition à un acide pharmaceutiquement acceptable.

**19.** Compositions pharmaceutiques contenant comme principe actif au moins un composé selon l'une quelconque des revendications 1 à 17 seul ou en combinaison avec un ou plusieurs excipients ou véhicules inertes, non toxiques, pharmaceutiquement acceptables.

**20.** Compositions pharmaceutiques selon la revendication 19 contenant au moins un principe actif selon l'une des revendications 1 à 17 utiles pour le traitement de la dépression, du stress, les psychoses, de l'anxiété, de la douleur, de la schizophrénie, l'hypertension, la prévention de l'athérome et comme modificateur du comportement alimentaire et sexuel.

**Revendications pour l'Etat contractant suivant : ES**

**1.** Procédé de préparation de composés de formule générale (I) :

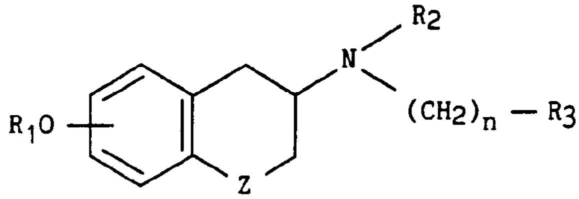

(I)

dans laquelle :

- Z représente un atome d'oxygène ou un atome de soufre,
- $R_1$ représente un atome d'hydrogène, ou un groupement alkyle ($C_1$-$C_6$) linéaire ou ramifié,
- $R_2$ représente un atome d'hydrogène ou un groupement alkyle ($C_1$-$C_6$) linéaire ou ramifié,
- n est un nombre entier compris entre 1 et 6,

- $R_3$ représente un groupement nitrile, un groupement amino éventuellement substitué par :
  . un groupement acyle linéaire ou ramifié comprenant de 2 à 7 atomes de carbone
  . un groupement alkylsulfonyl,
  . un groupement arylsulfonyl éventuellement substitué par un groupement alkyle, alkoxy, hydroxy ou un atome d'halogène,
  . un groupement alkyle ($C_1$-$C_6$) linéaire ou ramifié,
- ou $R_3$ représente l'un quelconque des groupements suivants :

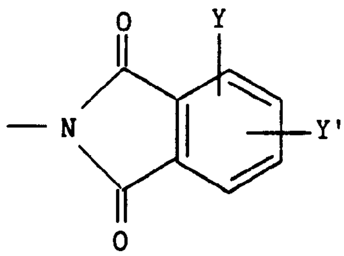 ,

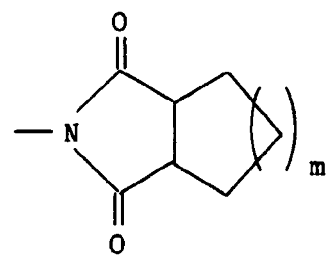 ,

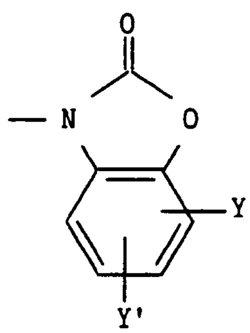 ,

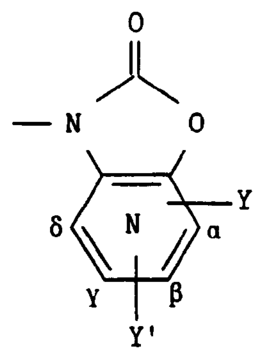 ,

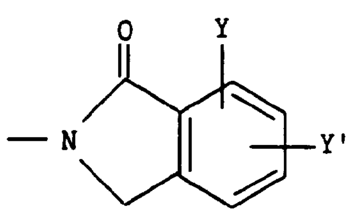 ,

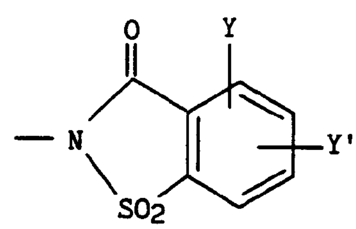 ,

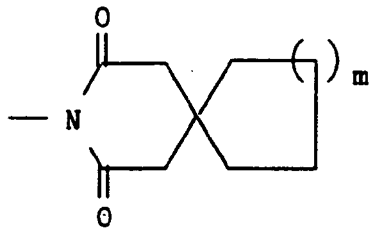

,

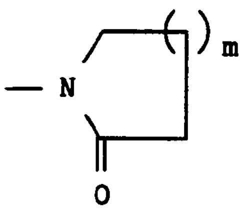

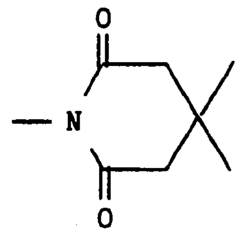

,

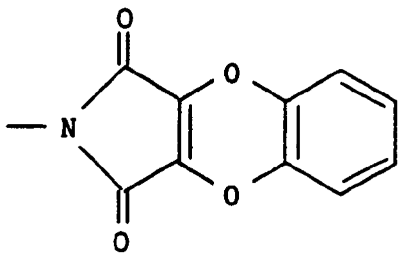

,

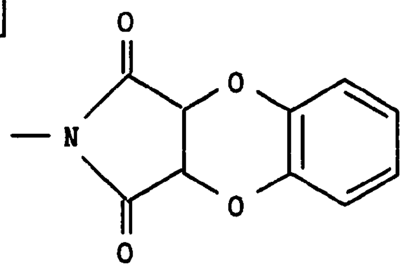

,

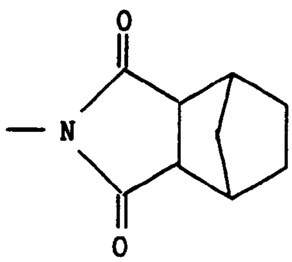

,

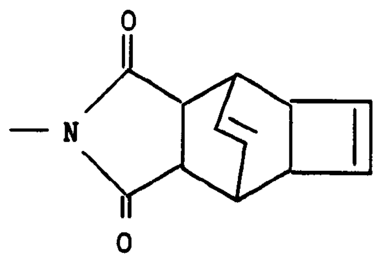

dans lesquels :

. Y et Y′ identiques ou différents représentent un atome d'hydrogène, un atome d'halogène, un groupement alkyle, alkoxy ou hydroxy,

. m est un nombre entier égal à 1 ou 2,

. et l'azote du cycle pyridine se situe en position α, β, γ ou δ de la jonction du cycle,

leurs énantiomères, diastéréoisomères et épimères ainsi que leurs sels d'addition à un acide pharmaceutiquement acceptable,

caractérisé en ce que l'on utilise comme matière première :

ou bien

a un composé de formule (II) :

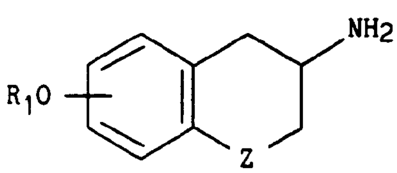

(II)

dans laquelle $R_1$ et Z ont la même signification que dans la formule (I), que l'on fait réagir sur un dérivé de formule (III) :

$$R_3\text{-}(CH_2)_n\text{-}X \qquad (III)$$

dans laquelle $R_3$ et n ont la même signification que dans la formule (I) et X est un atome d'halogène pour conduire à un dérivé de formule (I/a), cas particulier des dérivés de formule (I) :

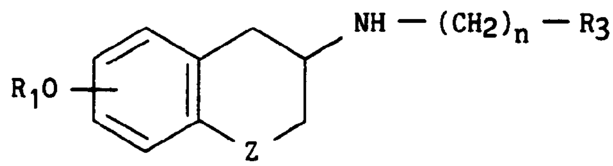

(I/a)

dans laquelle $R_1$, $R_3$, Z et n ont la même signification que dans la formule (I) que l'on soumet éventuellement à l'action d'un dérivé de formule (IV) :

$R'_2$-X (IV)

dans laquelle $R'_2$ représente un groupement alkyle (C1-C6) linéaire ou ramifié et X représente un atome d'halogène,
pour conduire à un dérivé de formule (I/b), cas particulier des dérivés de formule (I) :

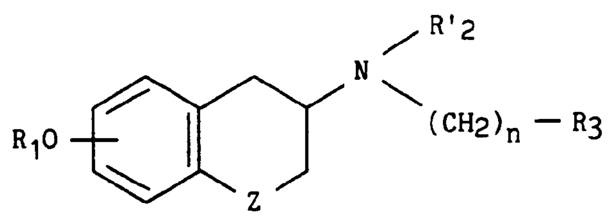

(I/b)

dans laquelle R, Z, $R'_2$, $R_3$ et n sont définis comme précédemment,
ou bien
b un composé de formule (V) :

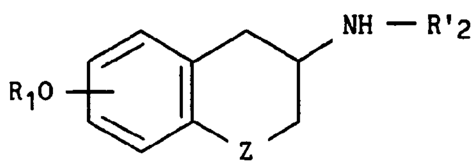

(V)

dans laquelle $R_1$, Z et $R'_2$ ont les mêmes significations que précédemment, que l'on fait réagir :

- soit sur un composé de formule (III) :

  $R_3$-$(CH_2)_n$-X (III)

  dans laquelle $R_3$, n et X sont définis comme précédemment,
  pour conduire à un dérivé de formule (I/b), cas particulier des dérivés de formule (I) :

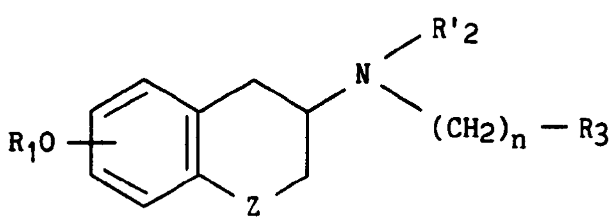

(I/b)

  dans laquelle $R_1$, Z, $R'_2$, $R_3$ et n sont définis comme précédemment,

- soit sur un composé de formule (III/a) :

  NC-$(CH_2)_m$-X (III/A)

dans laquelle X a la même signification que précédemment et m est un nombre entier compris entre 1 et 3,
pour conduire à un dérivé de formule (I/c), cas particulier des dérivés de formule (I) :

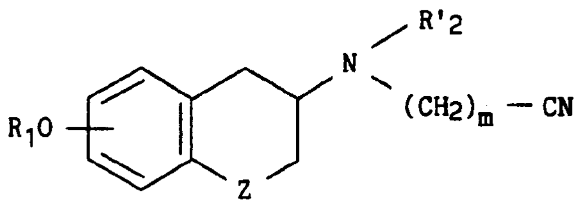

(I/c)

dans laquelle $R_1$, Z, $R'_2$ et m sont définis comme précédemment, que l'on réduit de façon catalytique en présence de nickel de Raney et d'ammoniac, ou chimiquement par l'hydrure de lithium/aluminium ou par le sodium dans l'alcool, pour conduire au dérivé de formule (I/d), cas particulier des dérivés de formule (I) :

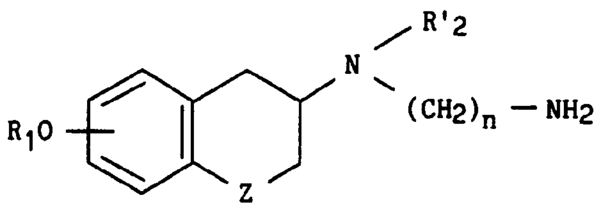

(I/d)

dans laquelle $R_1$, Z, $R'_2$ et n sont définis comme précédemment, que l'on fait réagir :

. ou bien sur un composé de formule (VI/A) :

$R_{4A}$-$SO_2$-X (VI/A)

dans laquelle $R_{4A}$ est un groupement alkyle ou un groupement aryle éventuellement substitué par un groupement alkyle et X est un atome d'halogène, pour conduire à un dérivé de formule (I/e), cas particulier des dérivés de formule (I) :

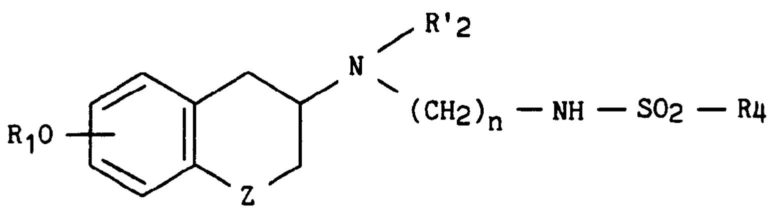

(I/e)

dans laquelle $R_1$, Z, $R'_2$, $R_4$, n sont définis comme précédemment,

. ou bien sur un composé de formule (VI/B) :

$R_{4B}$-CO-T (VI/B)

dans laquelle R4B représente un groupement alkyle linéaire ou ramifié de 1 à 6 atomes de carbone et T un groupe partant choisi parmi halogène, ou alkoxy inférieur ou un composé de formule (VI/C) :

$R_{4B}$-CO-O-CO-$R_{4B}$ (VI/C)

dans laquelle $R_{4B}$ a la même définition que précédemment pour conduire à un composé de formule (I/F) :

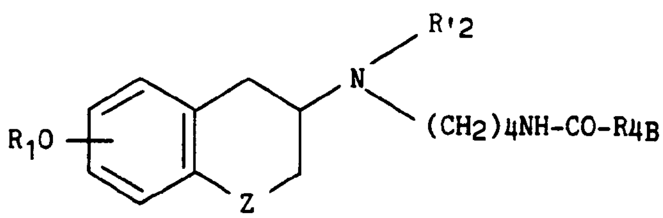

(I/F)

dans laquelle $R_1$, Z, $R'_2$, $R_{4B}$ et n ont la même définition que précédemment, cas particulier des dérivés de formule (I), pour lesquels $R_3$ représente un groupement amino substitué par un groupement acyle

linéaire ou ramifié de deux à sept atomes de carbone,
ou bien
c un composé de formule (VII) :

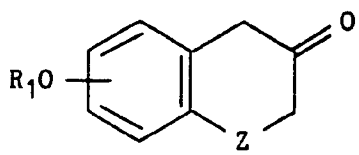

(VII)

dans laquelle $R_1$ et Z ont la même signification que dans la formule (I), que l'on soumet à une amination réductrice en présence d'un derivé de formule (VIII) :

$R_2$-NH-$(CH_2)_n$-$R_3$ (VIII)

dans laquelle $R_2$, $R_3$ et n sont définis comme dans la formule (I), pour conduire à un composé de formule (I), qui, lorsque $R_2$ représente un atome d'hydrogène peut être soumis à l'action d'un dérive de formule (IV) :

$R'_2$-X (IV)

dans laquelle $R'_2$ et X sont définis comme précédemment,
pour conduire à un dérivé de formule (I/b), cas particulier des dérivés de formule (I) :

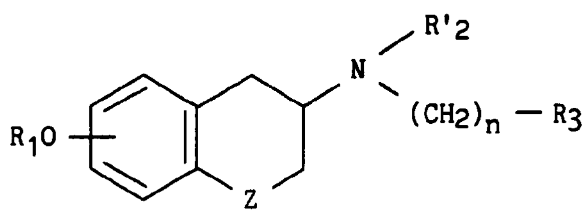

(I/b)

dans laquelle $R_1$, Z, $R'_2$, $R_3$ et n sont définis comme précédemment,
ou bien encore
d un composé de formule (IX) :

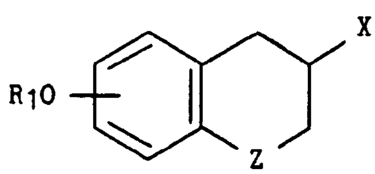

(IX)

dans laquelle $R_1$ et Z ont la même signification que dans la formule (I) et X est un atome d'allogène que l'on soumet à l'action d'un dérivé de formule (X) :

$H_2N$-$(CH_2)_n$-$R_3$ (X)

dans laquelle n et $R_3$ sont définis comme précédemment,pour conduire à un dérivé de formule (I/a), cas particulier des dérivés de formule (I) :

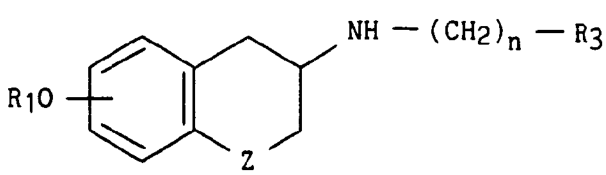

(I/a)

dans laquelle $R_1$, Z, n et $R_3$ sont définis comme précédemment, que l'on soumet éventuellement à l'action d'un dérivé de formule (IV)

$R'_2$-X (IV)

dans laquelle $R'_2$ et X sont définis comme précédemment,
pour conduire à un dérivé de formule (I/b), cas particulier des dérivés de formule (I) :

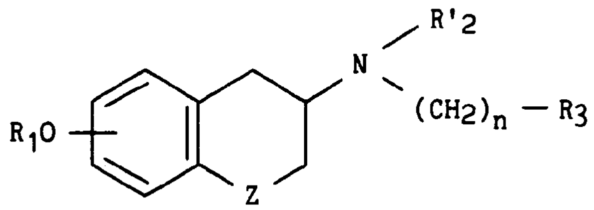

(I/b)

dans laquelle $R_1$, Z, $R'_2$, $R_3$ et n sont définis comme précédemment,
dérivés de formule (I/a), (I/b), (I/c), (I/d) et (I,e) dont on sépare éventuellement les isomères selon une technique classique de séparation, que l'on purifie, par une technique classique de purification et que l'on transforme, si on le désire, en leurs sels d'addition à un acide pharmaceutiquement acceptable.

2. Procédé de préparation selon la revendication 1 de composés de formule (I) tels que $R_1$ est un groupement méthyle.

3. Procédé de préparation selon la revendication 1 tels que $R_2$ est un groupement n-propyle.

4. Procédé de préparation selon la revendication 1 de composés de formule (I) tels que le groupement $OR_1$ se trouve en position 5.

5. Procédé de préparation selon la revendication 1 de composés de formule (I) tels que $R_2$ est un atome d'hydrogène.

6. Procédé de préparation selon la revendication 1 du composé de formule (I) qui est le 3-[4-[N-propyl-N-(5-méthoxy-chroman-3-yl) amino] butyl]-2,4-dioxo-3-azaspiro[4,5] décane, ses isomères ainsi que ses sels d'addition à un acide pharmaceutiquement acceptable.

7. Procédé de préparation selon la revendication 1 du composé de formule (I) qui est le 3⊖-[4-[Nn-propyl-N-(5-méthoxy-chroman-3-yl) amino] butyl]-2,4-dioxo-3-azaspiro[4,5] décane, ainsi que ses sels d'addition à un acide pharmaceutiquement acceptable.

8. Procédé de préparation selon la revendication 1 du composé de formule (I) qui est le 3⊕-[4-[Nn-propyl-N-(5-méthoxy-chroman-3-yl) amino] butyl]-2,4-dioxo-3-azaspiro[4,5] décane, ainsi que ses sels d'addition à un acide pharmaceutiquement acceptable.

9. Procédé de préparation selon la revendication 1 du composé de formule (I) qui est le 5-méthoxy-3-[N-propyl-N-[2-(4-toluène-sulfonylamino) éthyl] amino] chromane, ses isomères ainsi que ses sels d'addition à un acide pharmaceutiquement acceptable.

10. Procédé de préparation selon la revendication 1 du composé de formule (I) qui est le 5-méthoxy-3⊕-[N-propyl-N-[2-(4-toluène-sulfonylamino) éthyl] amino] chromane, ainsi que ses sels d'addition à un acide pharmaceutiquement acceptable.

11. Procédé de préparation selon la revendication 1 du composé de formule (I) qui est le 5-méthoxy-3⊖-[N-propyl-N-[2-(4-toluène-sulfonylamino) éthyl] amino] chromane, ainsi que ses sels d'addition à un acide pharmaceutiquement acceptable.

12. Procédé de préparation selon la revendication 1 du composé de formule (I) qui est le 5-méthoxy-3[Nn-propyl-N-[4-(4-toluène-sulfonylamino) butyl] amino] chromane, ses isomères ainsi que ses sels d'addition à un acide pharmaceutiquement acceptable.

13. Procédé de préparation selon la revendication 1 du composé de formule (I) qui est le 5-méthoxy-3⊕[Nn-propyl-N-[4-(4-toluène-sulfonylamino) butyl] amino] chromane, ainsi que ses sels d'addition à un acide pharmaceutiquement acceptable.

**14.** Procédé de préparation selon la revendication 1 du composé de formule (I) qui est le 5-méthoxy-3⊖[Nn-propyl-N-[4-(4-toluène-sulfonylamino) butyl] amino]chromane, ainsi que ses sels d'addition à un acide pharmaceutiquement acceptable.

**15.** Procédé de préparation selon la revendication 1 du composé de formule (I) qui est le 3-[4-[N-propyl-N-(5-méthoxy chroman-3-yl)amino]butyl]2,4-dioxo 3-azabicyclo [3.3.0] octane, ses isomères ainsi que ses sels d'addition à un acide pharmaceutiquement acceptable.

**16.** Procédé de préparation selon la revendication 1 du composé de formule (I) qui est le 3⊕-[4-[N-propyl-N-(5-méthoxy chroman-3-yl)amino]butyl]2,4-dioxo 3-azabicyclo [3.3.0] octane, ainsi que ses sels d'addition à un acide pharmaceutiquement acceptable.

**17.** Procédé de préparation selon la revendication 1 du composé de formule (I) qui est le 3⊖-[4-[N-propyl-N-(5-méthoxy chroman-3-yl)amino]butyl]2,4-dioxo 3-azabicyclo [3.3.0] octane, ainsi que ses sels d'addition à un acide pharmaceutiquement acceptable.

**18.** Procédé de préparation de compositions pharmaceutiques contenant comme principe actif au moins un composé obtenu selon l'une quelconque des revendications 1 à 17 seul ou en combinaison avec un ou plusieurs excipients ou véhicules inertes, non toxiques, pharmaceutiquement acceptables.

**19.** Procédé de préparation de compositions pharmaceutiques obtenus selon la revendication 18 contenant au moins un principe actif obtenu selon l'une des revendications 1 à 17 utiles pour le traitement de la dépression, du stress, des psychoses, de l'anxiété, de la douleur, de la schizophrénie, l'hypertension, la prévention de l'athérome et comme modificateur du comportement alimentaire et sexuel.

**Revendications pour l'Etat contractant suivant : GR**

**1.** Procédé de préparation de composés de formule générale (I) :

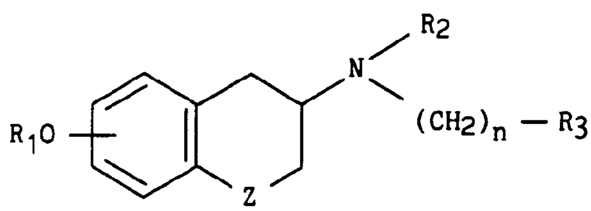

(I)

dans laquelle :

- Z représente un atome d'oxygène ou un atome de soufre,
- $R_1$ représente un atome d'hydrogène, ou un groupement alkyle ($C_1$-$C_6$) linéaire ou ramifié,
- $R_2$ représente un atome d'hydrogène ou un groupement alkyle ($C_1$-$C_6$) linéaire ou ramifié,
- n est un nombre entier compris entre 1 et 6,
- $R_3$ représente un groupement nitrile, un groupement amino éventuellement substitué par :
  . un groupement acyle linéaire ou ramifié comprenant de 2 à 7 atomes de carbone
  . un groupement alkylsulfonyl,
  . un groupement arylsulfonyl éventuellement substitué par un groupement alkyle, alkoxy, hydroxy ou un atome d'halogène,
  . un groupement alkyle ($C_1$-$C_6$) linéaire ou ramifié,
- ou $R_3$ représente l'un quelconque des groupements suivants :

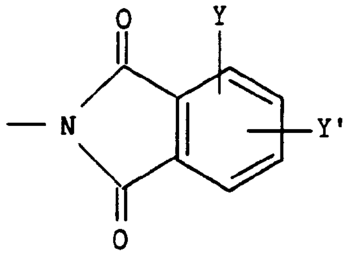 ,

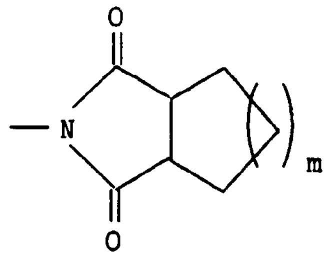 ,

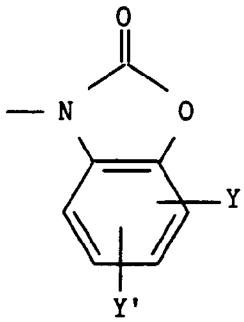 ,

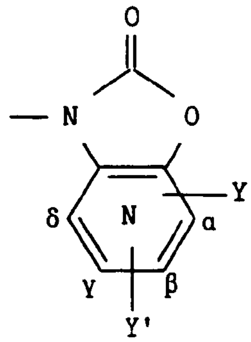 ,

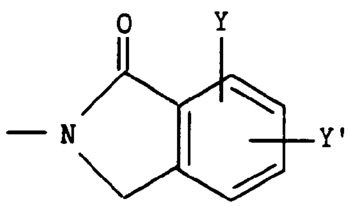 ,

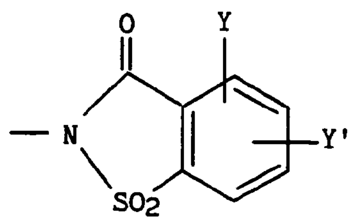 ,

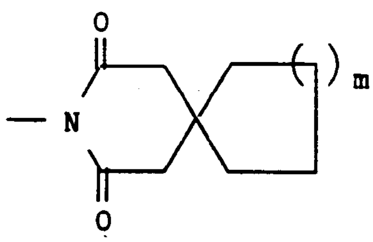 ,

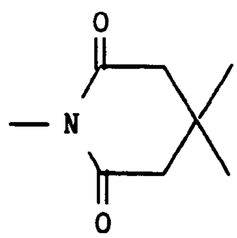 ,

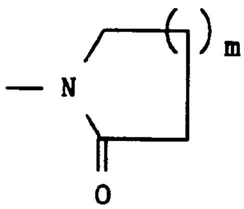

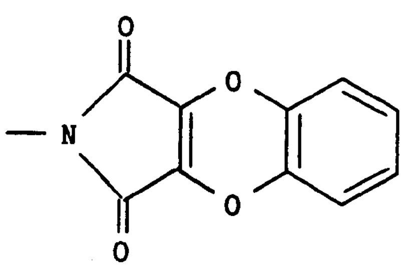 ,

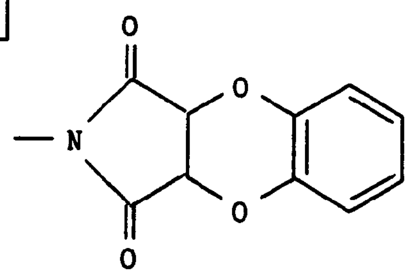 ,

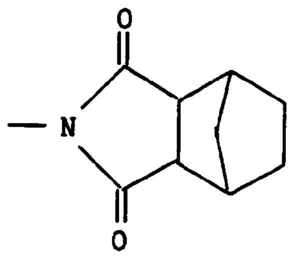 , 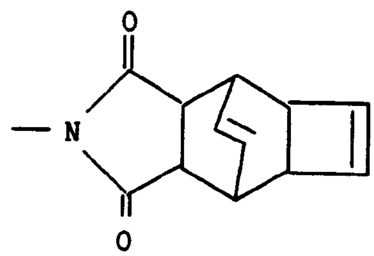

dans lesquels :

. Y et Y' identiques ou différents représentent un atome d'hydrogène, un atome d'halogène, un groupement alkyle, alkoxy ou hydroxy,

. m est un nombre entier égal à 1 ou 2,

. et l'azote du cycle pyridine se situe en position α, β, γ ou δ de la jonction du cycle,
leurs énantiomères, diastéréoisomères et épimères ainsi que leurs sels d'addition à un acide pharmaceutiquement acceptable,

caractérisé en ce que l'on utilise comme matière première :
ou bien
a un composé de formule (II) :

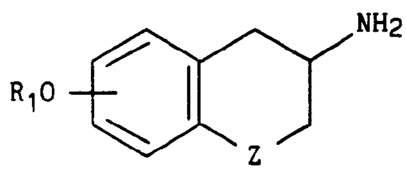 (II)

dans laquelle $R_1$ et Z ont la même signification que dans la formule (I), que l'on fait réagir sur un dérivé de formule (III) :

$$R_3\text{-}(CH_2)_n\text{-}X \qquad \text{(III)}$$

dans laquelle $R_3$ et n ont la même signification que dans la formule (I) et X est un atome d'halogène pour conduire à un dérivé de formule (I/a), cas particulier des dérivés de formule (I) :

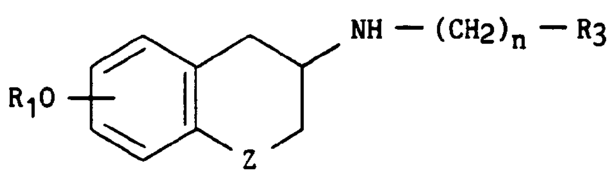 (I/a)

dans laquelle $R_1$, $R_3$, Z et n ont la même signification que dans la formule (I) que l'on soumet éventuellement à l'action d'un dérivé de formule (IV) :

$$R'_2\text{-}X \qquad \text{(IV)}$$

dans laquelle $R'_2$ représente un groupement alkyle (C1-C6) linéaire ou ramifié et X représente un atome d'halogène,
pour conduire à un dérivé de formule (I/b), cas particulier des dérivés de formule (I) :

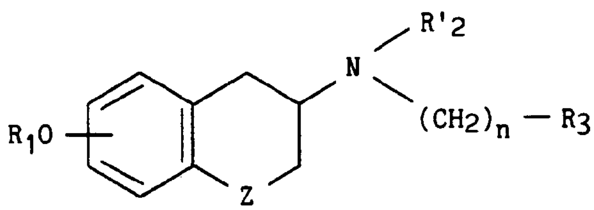 (I/b)

dans laquelle R, Z, $R'_2$, $R_3$ et n sont définis comme précédemment,

ou bien

b un composé de formule (V) :

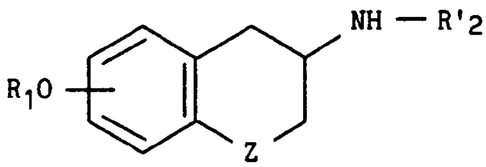

(V)

dans laquelle $R_1$, Z et $R'_2$ ont les mêmes significations que précédemment, que l'on fait réagir :

- soit sur un composé de formule (III) :

$$R_3\text{-}(CH_2)_n\text{-}X \qquad (III)$$

dans laquelle $R_3$, n et X sont définis comme précédemment,
pour conduire à un derivé de formule (I/b), cas particulier des dérivés de formule (I) :

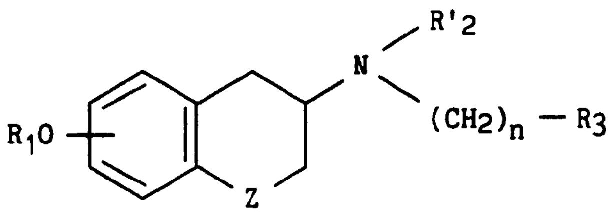

(I/b)

dans laquelle $R_1$, Z, $R'_2$, $R_3$ et n sont définis comme précédemment,

- soit sur un composé de formule (III/a) :

$$NC\text{-}(CH_2)_m\text{-}X \qquad (III/a)$$

dans laquelle X a la même signification que précédemment et m est un nombre entier compris entre 1 et 3,
pour conduire à un dérivé de formule (I/c), cas particulier des dérivés de formule (I) :

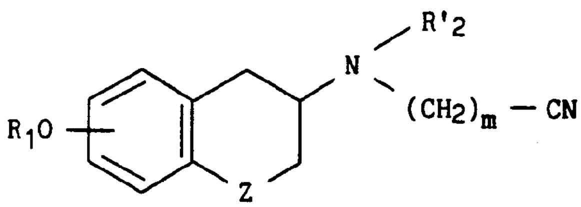

(I/c)

dans laquelle $R_1$, Z, $R'_2$ et m sont définis comme précédemment,
que l'on réduit de façon catalytique en présence de nickel de Raney et d'ammoniac, ou chimiquement par l'hydrure de lithium/aluminium ou par le sodium dans l'alcool,
pour conduire au dérivé de formule (I/d), cas particulier des dérivés de formule (I) :

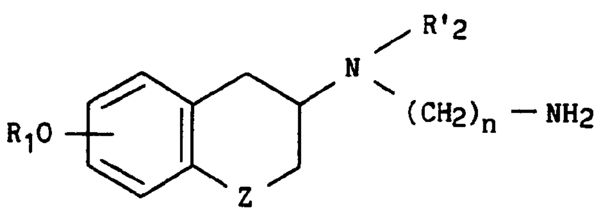

(I/d)

dans laquelle $R_1$, Z, $R'_2$ et n sont définis comme précédemment, que l'on fait réagir :

. ou bien sur un composé de formule (VI/A) :

$$R_{4A}\text{-}SO_2X \qquad (VI/A)$$

dans laquelle $R_{4A}$ est un groupement alkyle ou un groupement aryle éventuellement substitué par un groupement alkyle et X est un atome d'halogène,
pour conduire à un dérivé de formule (I/e), cas particulier des dérivés de formule (I) :

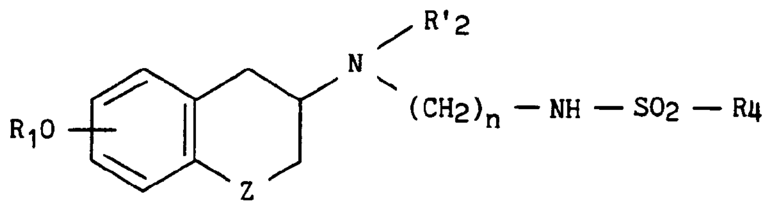

(I/e)

dans laquelle $R_1$, Z, $R'_2$, $R_4$, n sont définis comme précédemment,
. ou bien sur un composé de formule (VI/B) :

$R_{4B}$-CO-T (VI/B)

dans laquelle R4B représente un groupement alkyle linéaire ou ramifié de 1 à 6 atomes de carbone et T un groupe partant choisi parmi halogène, ou alkoxy inférieur ou un composé de formule (VI/C) :

$R_{4B}$-CO-O-CO-$R_{4B}$ (VI/C)

dans laquelle $R_{4B}$ a la même définition que précédemment pour conduire à un composé de formule (I/F) :

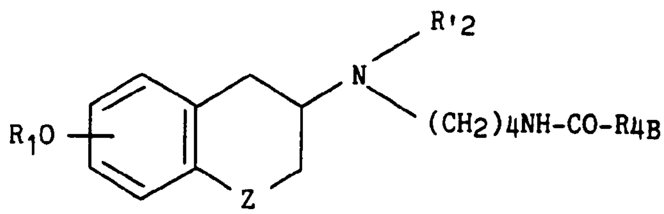

(I/F)

dans laquelle $R_1$, Z, $R'_2$, $R_{4B}$ et n ont la même définition que précédemment, cas particulier des dérivés de formule (I), pour lesquels $R_3$ représente un groupement amino substitué par un groupement acyle linéaire ou ramifié de deux à sept atomes de carbone,
ou bien
c un composé de formule (VII) :

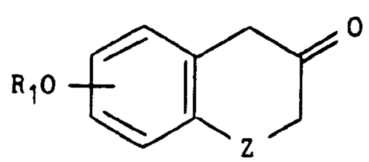

(VII)

dans laquelle $R_1$ et Z ont la même signification que dans la formule (I), que l'on soumet à une amination réductrice en présence d'un dérivé de formule (VIII) :

$R_2$-NH-$(CH_2)_n$-$R_3$ (VIII)

dans laquelle $R_2$, $R_3$ et n sont définis comme dans la formule (I), pour conduire à un composé de formule (I), qui, lorsque $R_2$ représente un atome d'hydrogène peut être soumis à l'action d'un dérivé de formule (IV) :

$R'_2$-X (IV)

dans laquelle $R'_2$ et X sont définis comme précédemment, pour conduire à un dérivé de formule (I/b), cas particulier des dérivés de formule (I) :

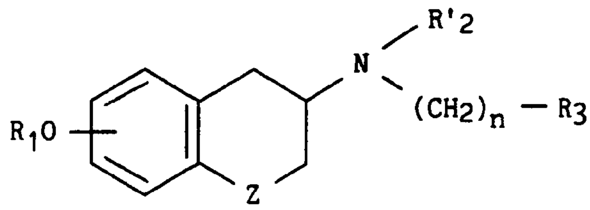

(I/b)

dans laquelle $R_1$, Z, $R'_2$, $R_3$ et n sont définis comme précédemment,
ou bien encore
d un composé de formule (IX) :

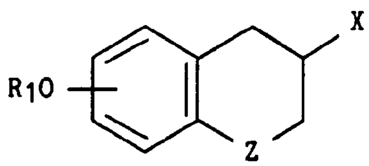

(IX)

dans laquelle $R_1$ et Z ont la même signification que dans la formule (1) et X est un atome d'halogène que l'on soumet à l'action d'un dérivé de formule (X) :

$H_2N-(CH_2)_n-R_3$ (X)

dans laquelle n et $R_3$ sont définis comme précédemment,pour conduire à un dérivé de formule (I/a), cas particulier des dérivés de formule (I) :

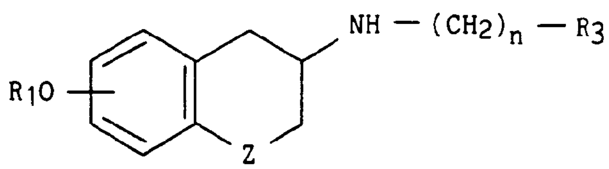

(I/a)

dans laquelle $R_1$, Z, n et $R_3$ sont définis comme précédemment, que l'on soumet éventuellement à l'action d'un dérivé de formule (IV)

$R'_2$-X (IV)

dans laquelle $R'_2$ et X sont définis comme précédemment,
pour conduire à un dérivé de formule (I/b), cas particulier des dérivés de formule (I) :

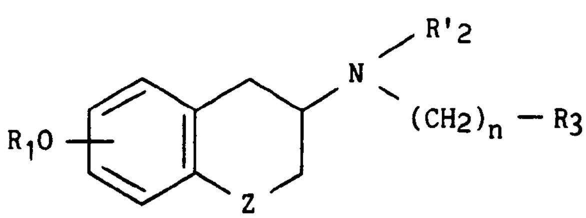

(I/b)

dans laquelle $R_1$, Z, $R'_2$, $R_3$ et n sont définis comme précédemment,
dérivés de formule (I/a), (I/b), (I/c), (I/d) et (I,e) dont on sépare éventuellement les isomères selon une technique classique de séparation, que l'on purifie, par une technique classique de purification et que l'on transforme, si on le désire, en leurs sels d'addition à un acide pharmaceutiquement acceptable.

2. Procédé de préparation selon la revendication 1 de composés de formule (I) tels que $R_1$ est un groupement méthyle.

3. Procédé de préparation selon la revendication 1 tels que $R_2$ est un groupement n-propyle.

4. Procédé de préparation selon la revendication 1 de composés de formule (I) tels que le groupement $OR_1$ se trouve en position 5.

5. Procédé de préparation selon la revendication 1 de composés de formule (I) tels que $R_2$ est un atome d'hydrogène.

6. Procédé de préparation selon la revendication 1 du composé de formule (I) qui est le 3-[4-[N-propyl-N-(5-méthoxy-chroman-3-yl) amino] butyl]-2,4-dioxo-3-azaspiro[4,5] décane, ses isomères ainsi que ses sels d'addition à un acide pharmaceutiquement acceptable.

7. Procédé de préparation selon la revendication 1 du composé de formule (I) qui est le 3⊖-[4-[Nn-propyl-N-(5-méthoxy-chroman-3-yl) amino] butyl]-2,4-dioxo-3-azaspiro[4,5] décane, ainsi que ses sels d'addition à un acide pharmaceutiquement acceptable.

8. Procédé de préparation selon la revendication 1 du composé de formule (I) qui est le 3⊕-[4-[Nn-propyl-N-(5-méthoxy-chroman-3-yl) amino] butyl]-2,4-dioxo-3-azaspiro[4,5] décane, ainsi que ses sels d'addition à un acide pharmaceutiquement acceptable.

9. Procédé de préparation selon la revendication 1 du composé de formule (I) qui est le 5-méthoxy-3-[N-propyl-N-[2-(4-toluène-sulfonylamino) éthyl] amino] chromane, ses isomères ainsi que ses sels d'addition à un acide pharmaceutiquement acceptable.

10. Procédé de préparation selon la revendication 1 du composé de formule (I) qui est le 5-méthoxy-3⊕-[N-propyl-N-[2-(4-toluène-sulfonylamino) éthyl] amino] chromane, ainsi que ses sels d'addition à un acide pharmaceutiquement acceptable.

11. Procédé de préparation selon la revendication 1 du composé de formule (I) qui est le 5-méthoxy-3⊖-[N-propyl-N-[2-(4-toluène-sulfonylamino) éthyl] amino] chromane, ainsi que ses sels d'addition à un acide pharmaceutiquement acceptable.

12. Procédé de préparation selon la revendication 1 du composé de formule ( 1 ) qui est le 5-méthoxy-3[Nn-propyl-N-[4-(4-toluène-sulfonylamino ) butyl ] amino ] chromane, ses isomères ainsi que ses sels d'addition à un acide pharmaceutiquement acceptable.

13. Procédé de préparation selon la revendication 1 du composé de formule (I) qui est le 5-méthoxy-3⊕[Nn-propyl-N-[4-(4-toluène-sulfonylamino) butyl] amino] chromane, ainsi que ses sels d'addition à un acide pharmaceutiquement acceptable.

14. Procédé de préparation selon la revendication 1 du composé de formule (I) qui est le 5-méthoxy-3⊖[Nn-propyl-N-[4-(4-toluène-sulfonylamino) butyl] amino]chromane, ainsi que ses sels d'addition à un acide pharmaceutiquement acceptable.

15. Procédé de préparation selon la revendication 1 du composé de formule (I) qui est le 3-[4-[N-propyl-N-(5-méthoxy chroman-3-yl)amino]butyl]2,4-dioxo 3-azabicyclo [3.3.0] octane, ses isomères ainsi que ses sels d'addition à un acide pharmaceutiquement acceptable.

16. Procédé de préparation selon la revendication 1 du composé de formule (I) qui est le 3⊕-[4-[N-propyl-N-(5-méthoxy chroman-3-yl)amino]butyl]2,4-dioxo 3-azabicyclo [3.3.0] octane, ainsi que ses sels d'addition à un acide pharmaceutiquement acceptable.

17. Procédé de préparation selon la revendication 1 du composé de formule (I) qui est le 3⊖-[4-[N-propyl-N-(5-méthoxy chroman-3-yl)amino]butyl]2,4-dioxo 3-azabicyclo [3.3.0] octane, ainsi que ses sels d'addition à un acide pharmaceutiquement acceptable.

18. Procédé de préparation de compositions pharmaceutiques contenant comme principe actif au moins un composé obtenu selon l'une quelconque des revendications 1 à 17 seul ou en combinaison avec un ou plusieurs excipients ou véhicules inertes, non toxiques, pharmaceutiquement acceptables.

19. Procédé de préparation de compositions pharmaceutiques obtenus selon la revendication 18 contenant au moins un principe actif obtenu selon l'une des revendications 1 à 17 utiles pour le traitement de la

dépression, du stress, des psychoses, de l'anxiété, de la douleur, de la schizophrénie, l'hypertension, la prévention de l'athérome et comme modificateur du comportement alimentaire et sexuel.

## Patentansprüche

### Patentansprüche für folgende Vertragsstaaten : AT, CH, LI, NL, BE, DE, FR, IT, SE, DK, GB, LU

1. Verbindungen der allgemeinen Formel (I):

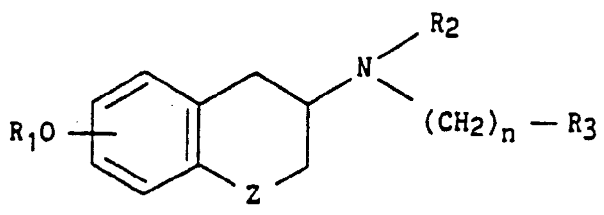

(I)

in der
- Z ein Sauerstoffatom oder ein Schwefelatom,
- $R_1$ ein Wasserstoffatom oder eine geradkettige oder verzweigte ($C_1$-$C_6$)-Alkylgruppe,
- $R_2$ ein Wasserstoffatom oder eine geradkettige oder verzweigte ($C_1$-$C_6$)-Alkylgruppe,
- n eine ganze Zahl zwischen 1 und 6,
- $R_3$ eine Nitrilgruppe, eine Aminogruppe, die gegebenenfalls durch:
  . eine geradkettige oder verzweigte Acylgruppe mit zwei bis sieben Kohlenstoffatomen,
  . eine Alkylsulfonylgruppe,
  . eine Arylsulfonylgruppe, die gegebenenfalls durch eine Alkylgruppe, Alkoxygruppe, Hydroxylgruppe oder ein Halogenatom substituiert ist, oder
  . eine geradkettige oder verzweigte ($C_1$-$C_6$)-Alkylgruppe substituiert ist,
- oder $R_3$ eine der folgenden Gruppen bedeuten:

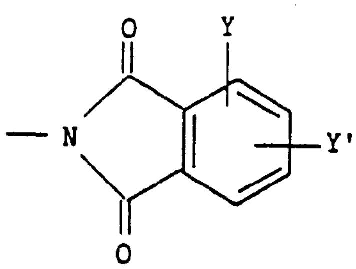
,
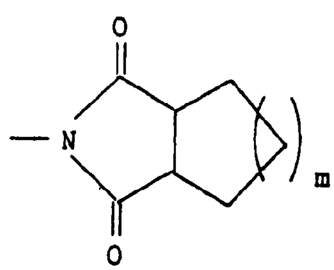
,

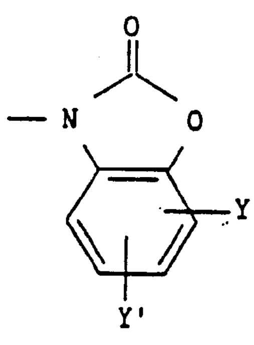
,
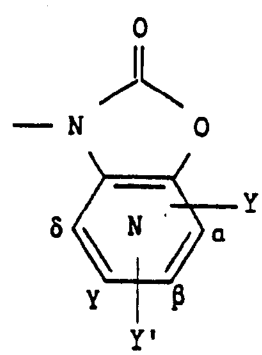
,

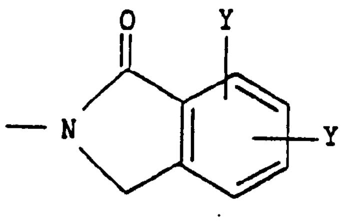

,

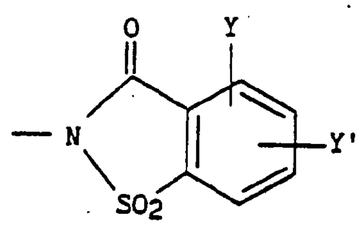

,

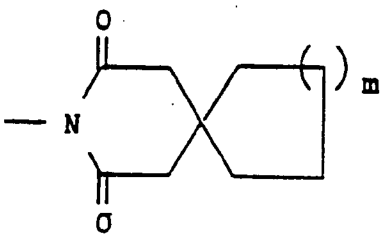

,

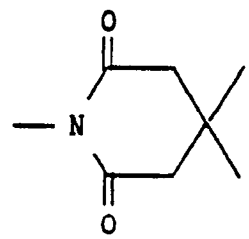

,

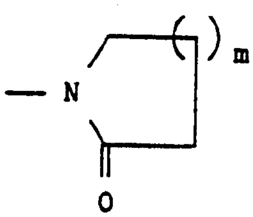

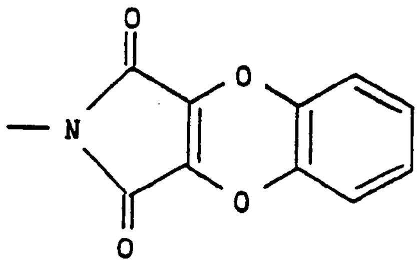

,

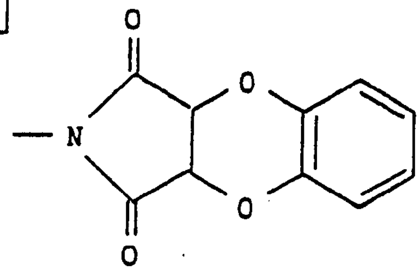

,

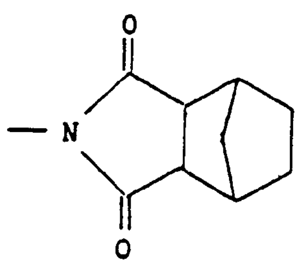

,

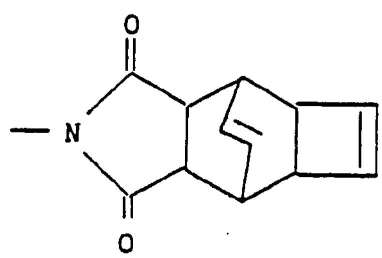

in der:

- . Y, Y', die gleichartig oder verschieden sein können, jeweils ein Wasserstoffatom, ein Halogenatom, eine Alkylgruppe, eine Alkoxygruppe oder eine Hydroxylgruppe,
- . m eine ganze Zahl mit einem Wert von 1 oder 2 darstellen und
- . das Stickstoffatom des Pyridinringes an der α-, β-, γ- oder δ-Stellung der Bindung des Ringes angeordnet ist,

deren Enantiomere, Diastereolsomere und Epimere sowie deren Additionssalze mit einer pharmazeutisch annehmbaren Säure.

**2.** Verbindungen nach Anspruch 1, worin $R_1$ eine Methylgruppe darstellt.

**3.** Verbindungen nach Anspruch 1, worin $R_2$ eine n-Propylgruppe darstellt.

**4.** Verbindungen nach Anspruch 1, worin $OR_1$ in der 5-Stellung steht.

**5.** Verbindungen nach Anspruch 1, worin $R_2$ ein Wasserstoffatom darstellt.

**6.** Verbindung nach Anspruch 1, nämlich 3-[4-[N-Propyl-N-(5-methoxy-chroman-3-yl)-amino]-butyl]-2,4-dioxo-3-azaspiro[4,5]decan, dessen Isomere sowie dessen Additionssalze mit einer pharmazeutisch annehmbaren Säure.

7. Verbindung nach Anspruch 1, nämlich 3⊖-[4-[Nn-Propyl-N-(5-methoxy-chroman-3-yl)-amino]-butyl]-2,4-dioxo-3-azaspiro[4,5]decan und dessen Additionssalze mit einer pharmazeutisch annehmbaren Säure.

8. Verbindung nach Anspruch 1, nämlich 3⊕-[4-[Nn-Propyl-N-(5-methoxy-chroman-3-yl)-amino]-butyl]-2, 4-dioxo-3-azaspiro[4,5]decan und dessen Additionssalze mit einer pharmazeutisch annehmbaren Säure.

9. Verbindung nach Anspruch 1, nämlich 5-Methoxy-3-[N-propyl-N-[2-(4-toluol-sulfonylamino)-ethyl]-amino]-chroman, dessen Isomere sowie dessen Additionssalze mit einer pharmazeutisch annehmbaren Säure.

10. Verbindung nach Anspruch 1, nämlich 5-Methoxy-3⊕-[N-propyl-N-[2-(4-toluol-sulfonylamino)-ethyl]-amino]-chroman und dessen Additionssalze mit einer pharmazeutisch annehmbaren Säure.

11. Verbindung nach Anspruch 1, nämlich 5-Methoxy-3⊖-[N-propyl-N-[2-(4-toluol-sulfonylamino)-ethyl]-amino]-chroman und dessen Additionssalze mit einer pharmazeutisch annehmbaren Säure.

12. Verbindung nach Anspruch 1, nämlich 5-Methoxy-3-[Nn-propyl-N-[4-(4-toluol-sulfonylamino)-butyl]-amino]chroman, dessen Isomere sowie dessen Additionssalze mit einer pharmazeutisch annehmbaren Säure.

13. Verbindung nach Anspruch 1, nämlich 5-Methoxy-3⊕-[Nn-propyl-N-[4-(4-toluol-sulfonylamino)-butyl]-amino]-chroman und dessen Additionssalze mit einer pharmazeutisch annehmbaren Säure.

14. Verbindung nach Anspruch 1, nämlich 5-Methoxy-3⊖-[Nn-propyl-N-[4-(4-toluol-sulfonylamino)-butyl]-amino]-chroman und dessen Additionssalze mit einer pharmazeutisch annehmbaren Säure.

15. Verbindung nach Anspruch 1, nämlich 3-[4-[N-Propyl-N-(5-methoxy-chroman-3-yl)-amino]-butyl]-2,4-dioxo-3-azabicyclo[3.3.0]octan, dessen Isomere und dessen Additionssalze mit einer pharmazeutisch annehmbaren Säure.

16. Verbindung nach Anspruch 1, nämlich 3⊕-[4-[N-Propyl-N-(5-methoxy-chroman-3-yl)-amino]-butyl]-2,4-dioxo-3-azabicyclo[3.3.0]octan und dessen Additionssalze mit einer pharmazeutisch annehmbaren Säure.

17. Verbindung nach Anspruch 1, nämlich 3⊖-[4-[N-Propyl-N-(5-methoxy-chroman-3-yl)-amino]-butyl]-2,4-dioxo-3-azabicyclo[3,3,0]octan und dessen Addltionssalze mit einer pharmazeutisch annehmbaren Säure.

18. Verfahren zur Herstellung der Verbindungen der Formel (I), **dadurch gekennzeichnet,** daß man als Ausgangsmaterial
entweder
a eine Verbindung der Formel (II):

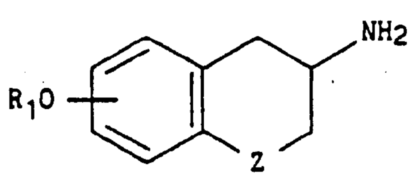

(II)

in der $R_1$ und Z die bezüglich der Formel (I) angegebenen Bedeutungen besitzen, verwendet und mit einem Derivat der Formel (III):

$$R_3\text{-}(CH_2)_n\text{-}X \qquad (III)$$

in der $R_3$ und n die bezüglich der Formel (I) angegebenen Bedeutungen besitzen und X ein Halogenatom darstellt, umsetzt zur Bildung eines Derivats der Formel (I/a), einem Sonderfall der Derivate der Formel (I):

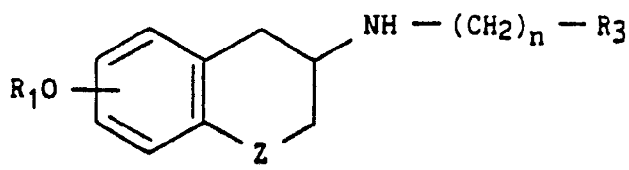

(I/a)

in der $R_1$, $R_3$, Z und n die bezüglich der Formel (I) angegebenen Bedeutungen besitzen, welches man gegebenenfalls der Einwirkung eines Derivats der Formel (IV):

$R'_2$-X (IV)

in der $R'_2$ eine geradkettige oder verzweigte ($C_1$-$C_6$)-Alkylgruppe und X ein Halogenatom darstellen, unterwirft, zur Bildung eines Derivats der Formel (I/b), einem Sonderfall der Derivate der Formel (I):

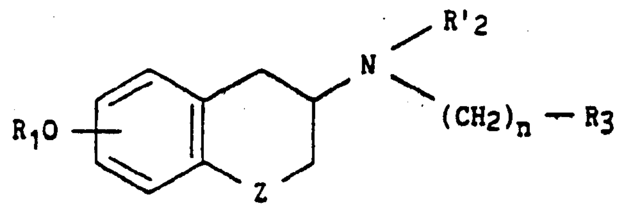

(I/b)

in der $R_1$, Z, $R'_2$, $R_3$ und n die oben angegebenen Bedeutungen besitzen,
oder
b eine Verbindung der Formel (V):

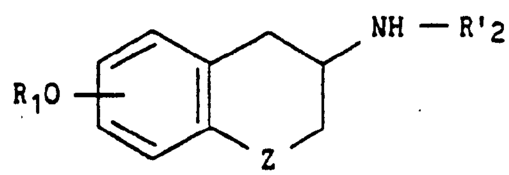

(V)

in der $R_1$, Z und $R'_2$ die oben angegebenen Bedeutungen besitzen, verwendet, welche man

- entweder mit einer Verbindung der Formel (III):

  $R_3$-$(CH_2)_n$-X (III)

  in der $R_3$, n und X die oben angegebenen Bedeutungen besitzen, umsetzt, so daß man ein Derivat der Formel (I/b) erhält, einem Sonderfall der Derivate der Formel (I):

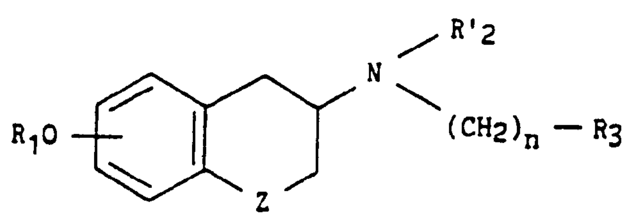

(I/b)

  in der $R_1$, Z, $R'_2$, $R_3$ und n die oben angegebenen Bedeutungen besitzen,
- oder mit einer Verbindung der Formel (III/a):

  NC-$(CH_2)_m$-X (III/a)

in der X die oben angegebenen Bedeutungen besitzt und m eine ganze Zahl mit einem Wert zwischen 1 und 3 darstellt, umsetzt, so daß man ein Derivat der Formel (I/c) erhält, einem Sonderfall der Derivate der Formel (I):

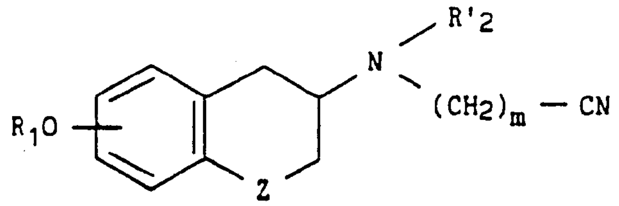

(I/c)

in der $R_1$, Z, $R'_2$ und n die oben angegebenen Bedeutungen besitzen, welche man katalytisch in Gegenwart von Raney-Nickel und Ammoniak oder chemisch durch Lithiumaluminiumhydrid oder Natrium in Alkohol reduziert,
so daß man ein Derivat der Formel (I/d) erhält, einem Sonderfall der Derivate der Formel (I):

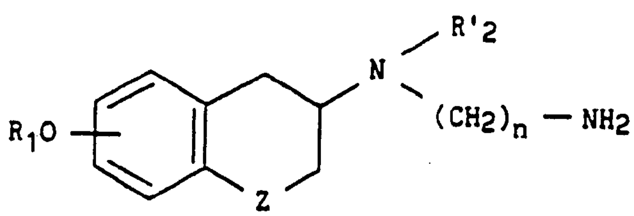

(I/d)

in der $R_1$, Z, $R'_2$ und n die oben angegebenen Bedeutungen besitzen, welches man

. entweder mit einer Verbindung der Formel (VI/A):

$R_{4A}$-$SO_2$-X (VI/A)

in der $R_{4A}$ eine Alkylgruppe oder eine gegebenenfalls durch eine Alkylgruppe substituierte Arylgruppe und X ein Halogenatom darstellen, umsetzt,
zur Bildung eines Derivats der Formel (I/e), einem Sonderfall der Derivate der Formel (I):

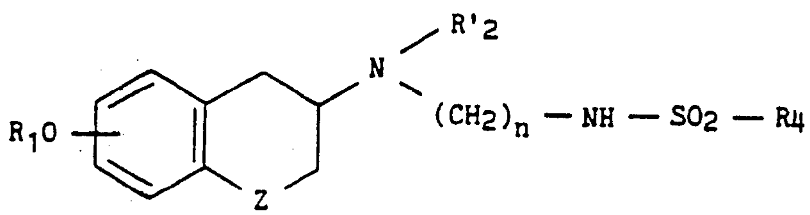

(I/e)

in der $R_1$, Z, $R'_2$, $R_4$ und n die oben angegebenen Bedeutungen besitzen,

. oder mit einer Verbindung der Formel (VI/B):

$R_{4B}$-CO-T (VI/B)

in der $R_{4B}$ eine geradkettige oder verzweigte Alkylgruppe mit 1 bis 4 Kohlenstoffatomen und T eine austretende Gruppe, ausgewählt aus Halogenen oder Niedrigalkoxygruppen bedeuten, oder mit einer Verbindung der Formel (VI/C):

$R_{4B}$-CO-O-CO-$R_{4B}$ (VI/C)

in der R4B die oben angegebenen Bedeutungen besitzen, umsetzt, so daß man eine Verbindung der Formel (I/F):

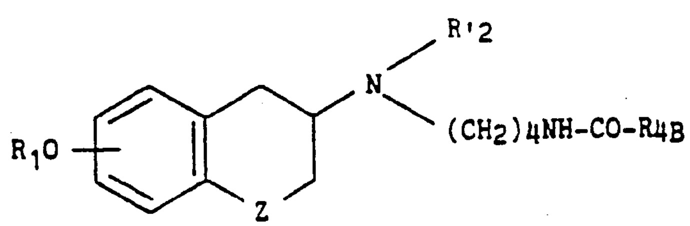

(I/F)

in der $R_1$, Z, $R'_2$, $R_{4B}$ und n die oben angegebenen Bedeutungen besitzen, erhält, einem Sonderfall

der Derivate der Formel (I), worin $R_3$ eine durch eine geradkettige oder verzweigte Acylgruppe mit 2 bis 7 Kohlenstoffatomen substituierte Aminogruppe darstellt,
oder
c eine Verbindung der Formel (VII):

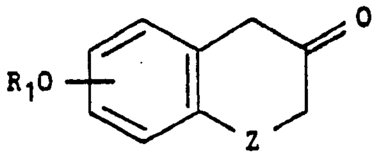

(VII)

in der $R_1$ und Z die bezüglich der Formel (I) angegebenen Bedeutungen besitzen, verwendet, welche man einer reduzierenden Aminierung in Gegenwart eines Derivats der Formel (VIII):

$$R_2\text{-NH-}(CH_2)_n\text{-}R_3 \qquad \text{(VIII)}$$

in der $R_2$, $R_3$ und n die bezüglich der Formel (I) angegebenen Bedeutungen besitzen, unterwirft zur Bildung einer Verbindung der Formel (I), die, wenn $R_2$ ein Wasserstoffatom darstellt, der Einwirkung eines Derivats der Formel (IV):

$$R'_2\text{-X} \qquad \text{(IV)}$$

in der $R'_2$ und X die oben angegebenen Bedeutungen besitzen, unterworfen werden kann, zur Bildung eines Derivats der Formel (I/b), einem Sonderfall der Derivate der Formel (I):

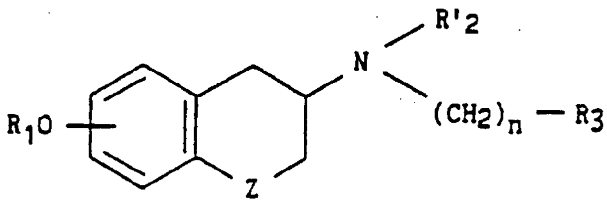

(I/b)

in der $R_1$, Z, $R'_2$, $R_3$ und n die oben angegebenen Bedeutungen besitzen,
oder schließlich
d eine Verbindung der Formel (IX):

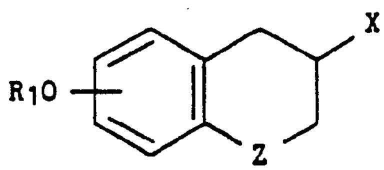

(IX)

in der $R_1$ und Z die bezüglich der Formel (I) angegebenen Bedeutungen besitzen und X ein Halogenatom darstellt, verwendet, welche man der Einwirkung eines Derivats der Formel (X):

$$H_2N\text{-}(CH_2)_n\text{-}R_3 \qquad \text{(X)}$$

in der n und $R_3$ die oben angegebenen Bedeutungen besitzen, unterwirft zur Bildung eines Derivats der Formel (I/a), einem Sonderfall der Derivate der Formel (I):

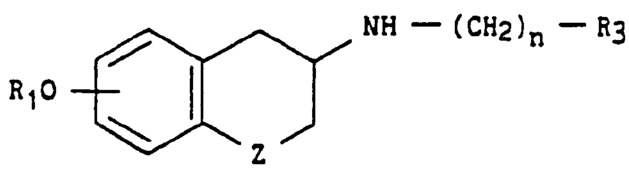

(I/a)

in der $R_1$, Z, n und $R_3$ die oben angegebenen Bedeutungen besitzen, welches man gegebenenfalls der Einwirkung eines Derivates der Formel (IV):

$$R'_2\text{-X} \qquad \text{(IV)}$$

in der $R'_2$ und X die oben angegebenen Bedeutungen besitzen, unterwirft, zur Bildung eines Derivats

der Formel (I/b), einem Sonderfall der Derivate der Formel (I):

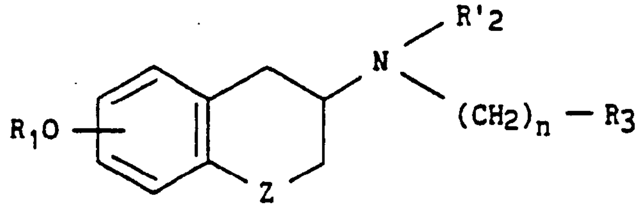

(I/b)

in der $R_1$, Z, $R'_2$, $R_3$ und n die oben angegebenen Bedeutungen besitzen, welche Derivate der Formeln (I/a), (I/b), (I/c), (I/d) und (I/e) man gegebenenfalls mit Hilfe eines klassischen Trennverfahrens in die Isomeren auftrennt, die man mit Hilfe einer klassischen Reinigungsmethode reinigt und gewünschtenfalls mit einer pharmazeutisch annehmbaren Säure in ihre Additionssalze überführt.

**19.** Pharmazeutische Zubereitungen enthaltend als Wirkstoff mindestens eine Verbindung nach einem der Ansprüche 1 bis 17 allein oder in Kombination mit einem oder mehreren inerten, nicht toxischen, pharmazeutisch annehmbaren Trägermaterialien oder Bindemitteln.

**20.** Pharmazeutische Zubereitungen nach Anspruch 19 enthaltend mindestens einen Wirkstoff nach einem der Ansprüche 1 bis 17 zur Behandlung der Depression, von Streß, von Psychosen, der Angst, von Schmerzen, der Schizophrenie, der Hypertension, zur Vorbeugung von Atheromen und als Modifizierungsmittel für das Ernährungsverhalten und das Sexualverhalten.

**Patentansprüche für folgenden Vertragsstaat : ES**

**1.** Verfahren zur Herstellung von Verbindungen der allgemeinen Formel (I):

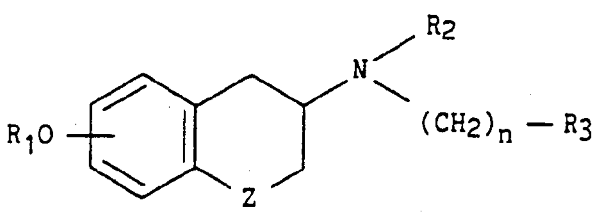

(I)

in der
- Z ein Sauerstoffatom oder ein Schwefelatom,
- $R_1$ ein Wasserstoffatom oder eine geradkettige oder verzweigte ($C_1$-$C_6$)-Alkylgruppe,
- $R_2$ ein Wasserstoffatom oder eine geradkettige oder verzweigte ($C_1$-$C_6$)-Alkylgruppe,
- n eine ganze Zahl zwischen 1 und 6,
- $R_3$ eine Nitrilgruppe, eine Aminogruppe, die gegebenenfalls durch:
  . eine geradkettige oder verzweigte Acylgruppe mit zwei bis sieben Kohlenstoffatomen,
  . eine Alkylsulfonylgruppe,
  . eine Arylsulfonylgruppe, die gegebenenfalls durch eine Alkylgruppe, Alkoxygruppe, Hydroxylgruppe oder ein Halogenatom substituiert ist, oder
  . eine geradkettige oder verzweigte ($C_1$-$C_6$)-Alkylgruppe substituiert ist,
- oder $R_3$ eine der folgenden Gruppen bedeuten:

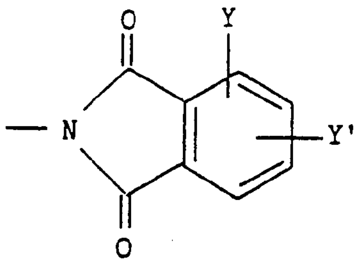 , 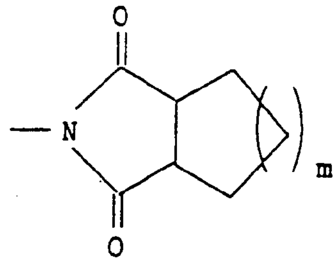 ,

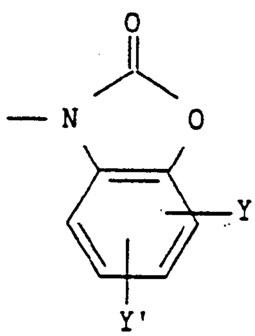 , 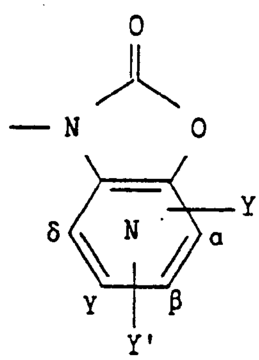 ,

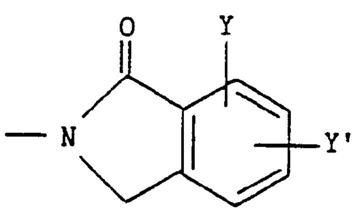 , 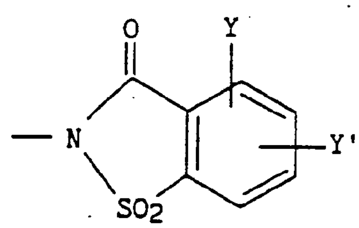 ,

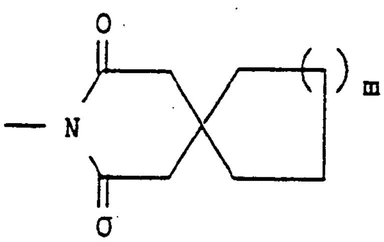 , 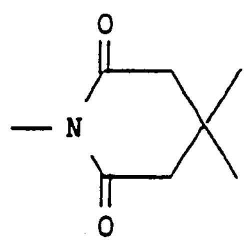 ,

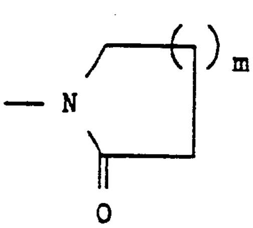

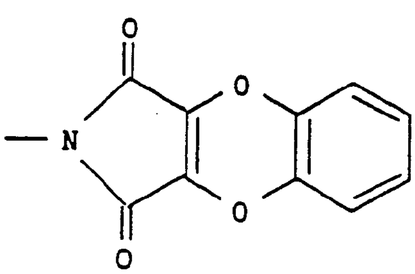 , 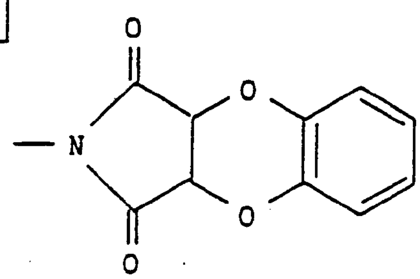 ,

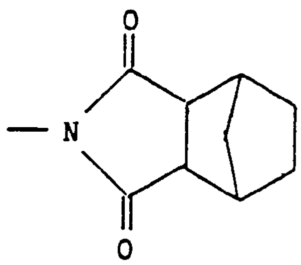

,

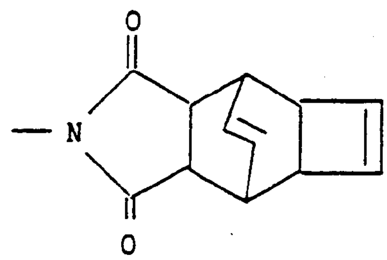

in der:

. Y, Y', die gleichartig oder verschieden sein können, jeweils ein Wasserstoffatom, ein Halogenatom, eine Alkylgruppe, eine Alkoxygruppe oder eine Hydroxylgruppe,

. m eine ganze Zahl mit einem Wert von 1 oder 2 darstellen und

. das Stickstoffatom des Pyridinringes an der α-, β-, γ- oder δ-Stellung der Bindung des Ringes angeordnet ist,

und von deren Enantiomeren, Diastereoisomeren und Epimeren sowie deren Additionssalzen mit einer pharmazeutisch annehmbaren Säure

**dadurch gekennzeichnet,** daß man als Ausgangsmaterial

entweder

a eine Verbindung der Formel (II):

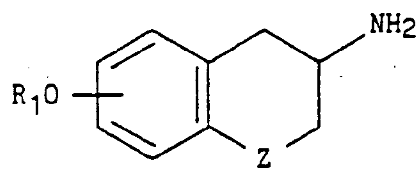

(II)

in der $R_1$ und Z die bezüglich der Formel (I) angegebenen Bedeutungen besitzen, verwendet und mit einem Derivat der Formel (III):

$$R_3\text{-}(CH_2)_n\text{-}X \quad (III)$$

in der $R_3$ und n die bezüglich der Formel (I) angegebenen Bedeutungen besitzen und X ein Halogenatom darstellt, umsetzt zur Bildung eines Derivats der Formel (I/a), einem Sonderfall der Derivate der Formel (I):

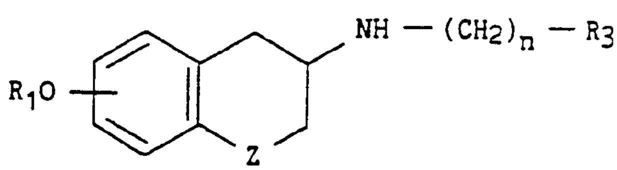

(I/a)

in der $R_1$, $R_3$, Z und n die bezüglich der Formel (I) angegebenen Bedeutungen besitzen, welches man gegebenenfalls der Einwirkung eines Derivats der Formel (IV):

$$R'_2\text{-}X \quad (IV)$$

in der $R'_2$ eine geradkettige oder verzweigte ($C_1$-$C_6$)-Alkylgruppe und X ein Halogenatom darstellen, unterwirft, zur Bildung eines Derivats der Formel (I/b), einem Sonderfall der Derivate der Formel (I):

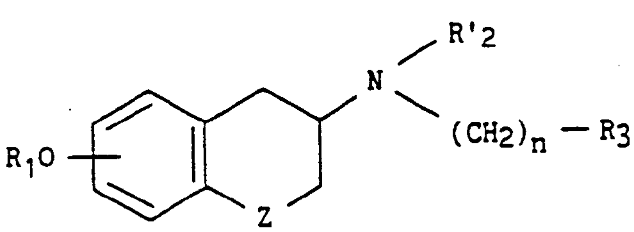

(I/b)

in der $R_1$, Z, $R'_2$, $R_3$ und n die oben angegebenen Bedeutungen besitzen,
oder
b eine Verbindung der Formel (V):

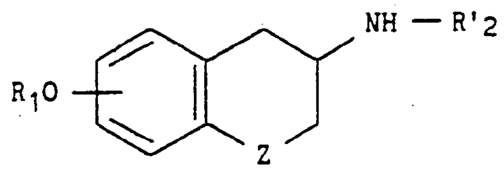

(V)

in der $R_1$, Z und $R'_2$ die oben angegebenen Bedeutungen besitzen, verwendet, welche man

- entweder mit einer Verbindung der Formel (III):

$R_3$-$(CH_2)_n$-X (III)

in der $R_3$, n und X die oben angegebenen Bedeutungen besitzen, umsetzt, so daß man ein Derivat der Formel (I/b) erhält, einem Sonderfall der Derivate der Formel (I):

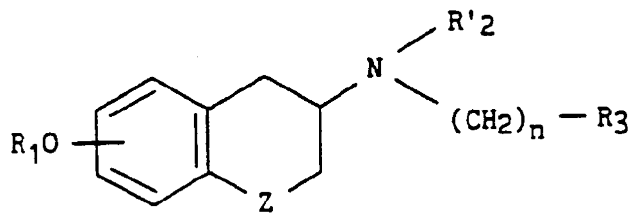

(I/b)

in der $R_1$, Z, $R'_2$, $R_3$ und n die oben angegebenen Bedeutungen besitzen,

- oder mit einer Verbindung der Formel (III/a):

NC-$(CH_2)_m$-X (III/a)

in der X die oben angegebenen Bedeutungen besitzt und m eine ganze Zahl mit einem Wert zwischen 1 und 3 darstellt, umsetzt, so daß man ein Derivat der Formel (I/c) erhält, einem Sonderfall der Derivate der Formel (I):

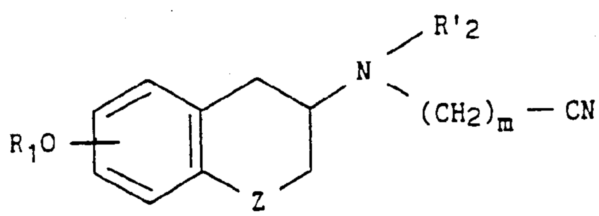

(I/c)

in der $R_1$, Z, $R'_2$ und n die oben angegebenen Bedeutungen besitzen, welche man katalytisch in Gegenwart von Raney-Nickel und Ammoniak oder chemisch durch Lithiumaluminiumhydrid oder Natrium in Alkohol reduziert,
so daß man ein Derivat der Formel (I/d) erhält, einem Sonderfall der Derivate der Formel (I):

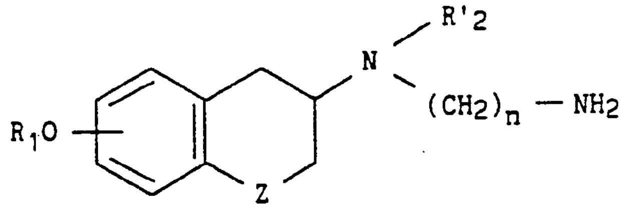

(I/d)

in der $R_1$, Z, $R'_2$ und n die oben angegebenen Bedeutungen besitzen, welches man

. entweder mit einer Verbindung der Formel (VI/A):

$R_{4A}$-$SO_2$-X (VI/A)

in der $R_{4A}$ eine Alkylgruppe oder eine gegebenenfalls durch eine Alkylgruppe substituierte Arylgruppe und X ein Halogenatom darstellen, umsetzt,
zur Bildung eines Derivats der Formel (I/e), einem Sonderfall der Derivate der Formel (I):

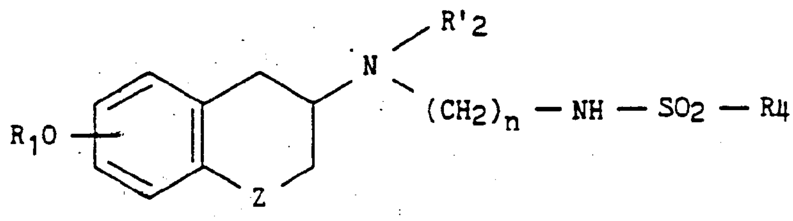

(I/e)

in der $R_1$, Z, $R'_2$, $R_4$ und n die oben angegebenen Bedeutungen besitzen,
. oder mit einer Verbindung der Formel (VI/B):

$$R_{4B}\text{-CO-T} \qquad \text{(VI/B)}$$

in der $R_{4B}$ eine geradkettige oder verzweigte Alkylgruppe mit 1 bis 4 Kohlenstoffatomen und T eine austretende Gruppe, ausgewählt aus Halogenen oder Niedrigalkoxygruppen bedeuten, oder mit einer Verbindung der Formel (VI/C):

$$R_{4B}\text{-CO-O-CO-}R_{4B} \qquad \text{(VI/C)}$$

in der R4B die oben angegebenen Bedeutungen besitzen, umsetzt, so daß man eine Verbindung der Formel (I/F):

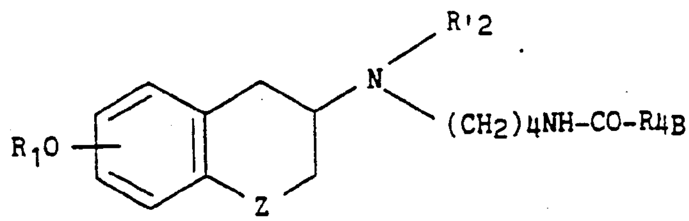

(I/F)

in der $R_1$, Z, $R'_2$, $R_{4B}$ und n die oben angegebenen Bedeutungen besitzen, erhält, einem Sonderfall der Derivate der Formel (I), worin $R_3$ eine durch eine geradkettige oder verzweigte Acylgruppe mit 2 bis 7 Kohlenstoffatomen substituierte Aminogruppe darstellt,
oder
c eine Verbindung der Formel (VII):

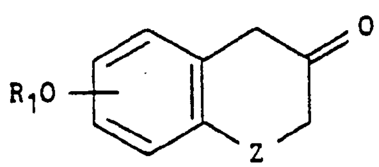

(VII)

in der $R_1$ und Z die bezüglich der Formel (I) angegebenen Bedeutungen besitzen, verwendet, welche man einer reduzierenden Aminierung in Gegenwart eines Derivats der Formel (VIII):

$$R_2\text{-NH-}(CH_2)_n\text{-}R_3 \qquad \text{(VIII)}$$

in der $R_2$, $R_3$ und n die bezüglich der Formel (I) angegebenen Bedeutungen besitzen, unterwirft zur Bildung einer Verbindung der Formel (I), die, wenn $R_2$ ein Wasserstoffatom darstellt, der Einwirkung eines Derivats der Formel (IV):

$$R'_2\text{-X} \qquad \text{(IV)}$$

in der $R'_2$ und X die oben angegebenen Bedeutungen besitzen, unterworfen werden kann,
zur Bildung eines Derivats der Formel (I/b), einem Sonderfall der Derivate der Formel (I):

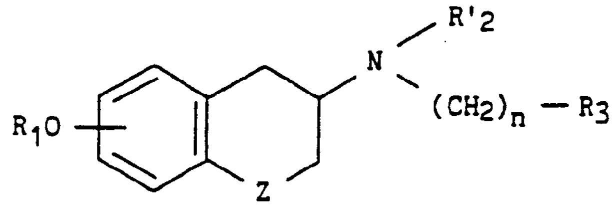

(I/b)

in der $R_1$, Z, $R'_2$, $R_3$ und n die oben angegebenen Bedeutungen besitzen,
oder schließlich
d eine Verbindung der Formel (IX):

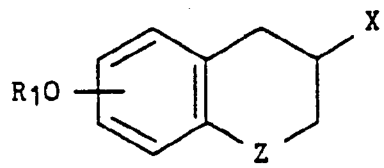

(IX)

in der $R_1$ und Z die bezüglich der Formel (I) angegebenen Bedeutungen besitzen und X ein Halogenatom darstellt, verwendet, welche man der Einwirkung eines Derivats der Formel (X):

$H_2N-(CH_2)_n-R_3$ (X)

in der n und $R_3$ die oben angegebenen Bedeutungen besitzen, unterwirft zur Bildung eines Derivats der Formel (I/a), einem Sonderfall der Derivate der Formel (I):

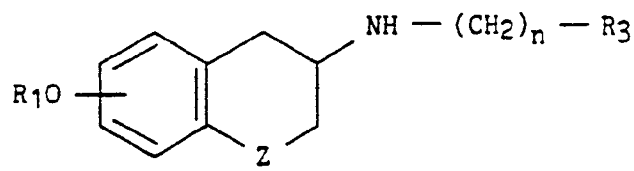

(I/a)

in der $R_1$, Z, n und $R_3$ die oben angegebenen Bedeutungen besitzen, welches man gegebenenfalls der Einwirkung eines Derivates der Formel (IV):

$R'_2$-X (IV)

in der $R'_2$ und X die oben angegebenen Bedeutungen besitzen, unterwirft, zur Bildung eines Derivats der Formel (I/b), einem Sonderfall der Derivate der Formel (I):

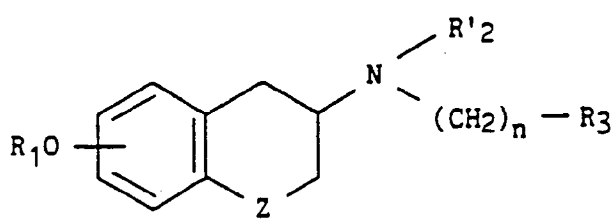

(I/b)

in der $R_1$, Z, $R'_2$, $R_3$ und n die oben angegebenen Bedeutungen besitzen, welche Derivate der Formeln (I/a), (I/b), (I/c), (I/d) und (I/e) man gegebenenfalls mit Hilfe eines klassischen Trennverfahrens in die Isomeren auftrennt, die man mit Hilfe einer klassischen Reinigungsmethode reinigt und gewünschtenfalls mit einer pharmazeutisch annehmbaren Säure in ihre Additionssalze überführt.

2. Verfahren nach Anspruch 1 zur Herstellung von Verbindungen der Formel (I), worin $R_1$ eine Methylgruppe bedeutet.

3. Verfahren nach Anspruch 1 zur Herstellung von Verbindungen, worin $R_2$ eine n-Propylgruppe bedeutet.

4. Verfahren nach Anspruch 1 zur Herstellung von Verbindungen der Formel (I), worin die Gruppe $OR_1$ in

der 5-Stellung steht.

5. Verfahren nach Anspruch 1 zur Herstellung von Verbindungen der Formel (I), worin $R_2$ ein Wasserstoffatom bedeutet.

6. Verfahren nach Anspruch 1 zur Herstellung der Verbindung der Formel (I), nämlich 3-[4-[N-Propyl-N-(5-methoxy-chroman-3-yl)-amino]-butyl]-2,4-dioxo-3-azaspiro[4,5]decan, von dessen Isomeren sowie von dessen Additionssalzen mit einer pharmazeutisch annehmbaren Säure.

7. Verfahren nach Anspruch 1 zur Herstellung der Verbindung der Formel (I), nämlich 3⊖-[4-[Nn-Propyl-N-(5-methoxy-chroman-3-yl)-amino]- butyl]-2,4-dioxo-3-azaspiro[4,5]decan sowie von dessen Additionssalzen mit einer pharmazeutisch annehmbaren Säure.

8. Verfahren nach Anspruch 1 zur Herstellung der Verbindung der Formel (I), namlich 3⊕-[4-[Nn-Propyl-N-(5-methoxy-chroman-3-yl)-amino]-butyl]-2,4-dioxo-3-azaspiro[4,5]decan sowie von dessen Additionssalzen mit einer pharmazeutisch annehmbaren Säure.

9. Verfahren nach Anspruch 1 zur Herstellung der Verbindung der Formel (I), nämlich 5-Methoxy-3-[N-propyl-N-[2-(4-toluol-sulfonylamino)ethyl]-amino]-chroman, von dessen Isomeren sowie von dessen Additionssalzen mit einer pharmazeutisch annehmbaren Säure.

10. Verfahren nach Anspruch 1 zur Herstellung der Verbindung der Formel (I), nämlich 5-Methoxy-3⊕-[N-propyl-N-[2-(4-toluol-sulfonylamino)ethyl]-amino]-chroman und von dessen Additionssalzen mit einer pharmazeutisch annehmbaren Säure.

11. Verfahren nach Anspruch 1 zur Herstellung der Verbindung der Formel (I), nämlich 5-Methoxy-3⊖-[N-propyl-N-[2-(4-toluol-sulfonylamino)-ethyl]-amino]-chroman und von dessen Additionssalzen mit einer pharmazeutisch annehmbaren Säure.

12. Verfahren nach Anspruch 1 zur Herstellung der Verbindung der Formel (I), nämlich 5-Methoxy-3[Nn-propyl-N-[4-[4-toluol-sulfonylamino)butyl]-amino]-chroman und von dessen Isomeren sowie von dessen Additionssalzen mit einer pharmazeutisch annehmbaren Säure.

13. Verfahren nach Anspruch 1 zur Herstellung der Verbindung der Formel (I), nämlich 5-Methoxy-3⊕[Nn-propyl-N-[4-(4-toluol-sulfonylamino)butyl]-amino]-chroman und von dessen Additionssalzen mit einer pharmazeutisch annehmbaren Säure.

14. Verfahren nach Anspruch 1 zur Herstellung der Verbindung der Formel (I), nämlich 5-Methoxy-3⊖-[Nn-propyl-N-[4-(4-toluol-sulfonylamino)-butyl]-amino]-chroman und von dessen Additionssalzen mit einer pharmazeutisch annehmbaren Säure.

15. Verfahren nach Anspruch 1 zur Herstellung der Verbindung der Formel (I), nämlich 3-[4-[N-Propyl-N-(5-methoxy-chroman-3-yl)-amino]-butyl]-2,4-dioxo-3-azabicyclo[3.3.0]octan und von dessen Isomeren und von dessen Additionssalzen mit einer pharmazeutisch annehmbaren Säure.

16. Verfahren nach Anspruch 1 zur Herstellung der Verbindung der Formel (I), nämlich 3⊕-[4-[N-Propyl-N-(5-methoxy-chroman-3-yl)-amino]butyl]-2,4-dioxo-3-azabicyclo[3.3.0]octan und von dessen Additionssalzen mit einer pharmazeutisch annehmbaren Säure.

17. Verfahren nach Anspruch 1 zur Herstellung der Verbindung der Formel (I), nämlich 3⊖-[4-[N-Propyl-N-(5-methoxy-chroman-3-yl)-amino]butyl]-2,4-dioxo-3-azabicyclo[3.3.0]octan und von dessen Additionssalzen mit einer pharmazeutisch annehmbaren Säure.

18. Verfahren zur Herstellung von pharmazeutischen Zubereitungen enthaltend als Wirkstoffmindestens eine Verbindung erhalten nach einem der Ansprüche 1 bis 17 allein oder in Kombination mit einem oder mehreren inerten, nicht toxischen, pharmazeutisch annehmbaren Bindemitteln oder Trägermaterialien.

19. Verfahren zur Herstellung von pharmazeutischen Zubereitungen nach Anspruch 18, enthaltend mindestens einen Wirkstoff erhalten nach einem der Ansprüche 1 bis 17 zur Behandlung der Depression, von Streß, von Psychosen, der Angst, von Schmerzen, der Schizophrenie, der Hypertension, zur Vorbeugung

von Atheromen und als Modifizierungsmittel des Ernährungs- und Sexualverhaltens.

**Patentansprüche für folgenden Vertragsstaat : GR**

1. Verfahren zur Herstellung von Verbindungen der allgemeinen Formel (I):

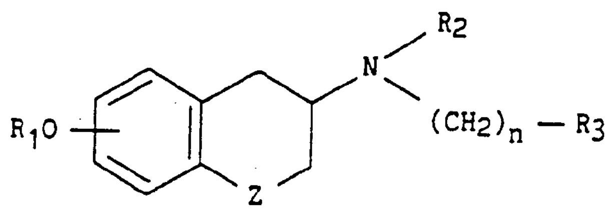

(I)

in der

- Z ein Sauerstoffatom oder ein Schwefelatom,
- $R_1$ ein Wasserstoffatom oder eine geradkettige oder verzweigte ($C_1$-$C_6$)-Alkylgruppe,
- $R_2$ ein Wasserstoffatom oder eine geradkettige oder verzweigte ($C_1$-$C_6$)-Alkylgruppe,
- n eine ganze Zahl zwischen 1 und 6,
- $R_3$ eine Nitrilgruppe, eine Aminogruppe, die gegebenenfalls durch:
  . eine geradkettige oder verzweigte Acylgruppe mit zwei bis sieben Kohlenstoffatomen,
  . eine Alkylsulfonylgruppe,
  . eine Arylsulfonylgruppe, die gegebenenfalls durch eine Alkylgruppe, Alkoxygruppe, Hydroxylgruppe oder ein Halogenatom substituiert ist, oder
  . eine geradkettige oder verzweigte ($C_1$-$C_6$)-Alkylgruppe substituiert ist,
- oder $R_3$ eine der folgenden Gruppen bedeuten:

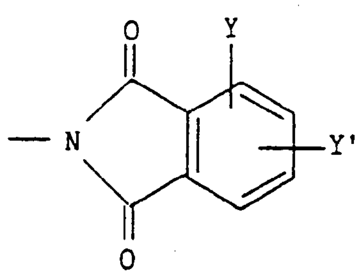

,

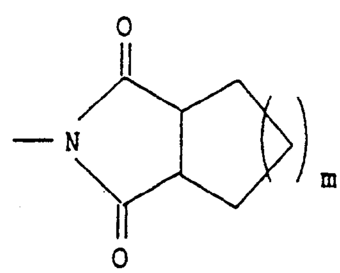

,

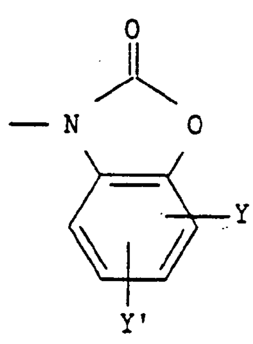

,

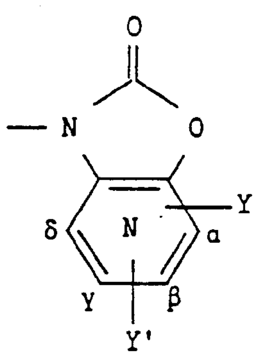

,

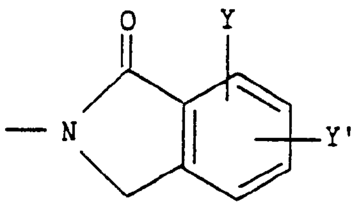 , 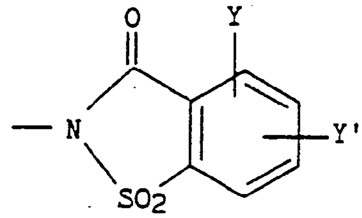 ,

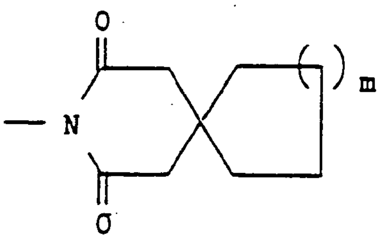 , 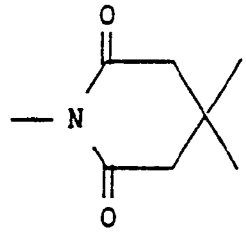 ,

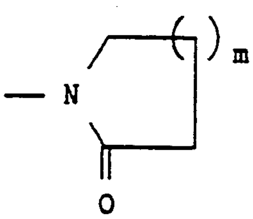

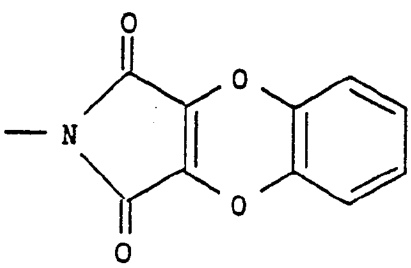 , 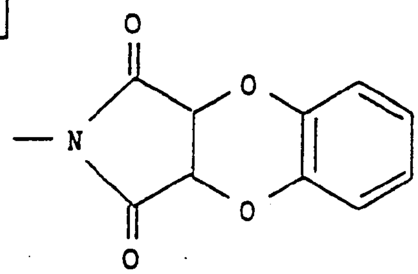 ,

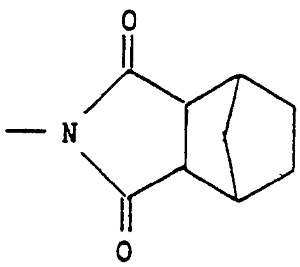 , 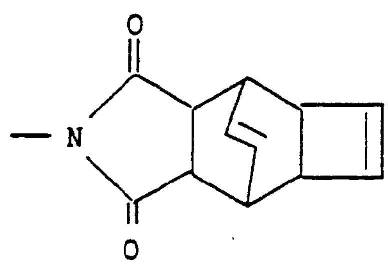

in der:

- . Y, Y′, die gleichartig oder verschieden sein können, jeweils ein Wasserstoffatom, ein Halogenatom, eine Alkylgruppe, eine Alkoxygruppe oder eine Hydroxylgruppe,
- . m eine ganze Zahl mit einem Wert von 1 oder 2 darstellen und
- . das Stickstoffatom des Pyridinringes an der α-, β-, γ- oder δ-Stellung der Bindung des Ringes angeordnet ist,

und von deren Enantiomeren, Diastereoisomeren und Epimeren sowie deren Additionssalzen mit einer pharmazeutisch annehmbaren Säure

**dadurch gekennzeichnet,** daß man als Ausgangsmaterial

entweder

a eine Verbindung der Formel (II):

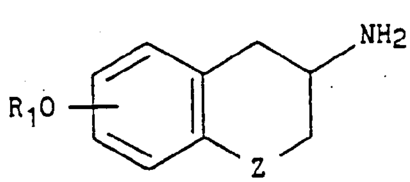

(II)

in der $R_1$ und Z die bezüglich der Formel (I) angegebenen Bedeutungen besitzen, verwendet und mit

einem Derivat der Formel (III):

$R_3$-$(CH_2)_n$-X (III)

in der $R_3$ und n die bezüglich der Formel (I) angegebenen Bedeutungen besitzen und X ein Halogenatom darstellt, umsetzt zur Bildung eines Derivats der Formel (I/a), einem Sonderfall der Derivate der Formel (I):

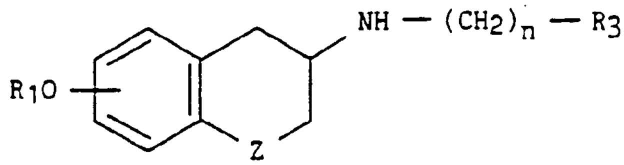

(I/a)

in der $R_1$, $R_3$, Z und n die bezüglich der Formel (I) angegebenen Bedeutungen besitzen, welches man gegebenenfalls der Einwirkung eines Derivats der Formel (IV):

$R'_2$-X (IV)

in der $R'_2$ eine geradkettige oder verzweigte ($C_1$-$C_6$)-Alkylgruppe und X ein Halogenatom darstellen, unterwirft, zur Bildung eines Derivats der Formel (I/b), einem Sonderfall der Derivate der Formel (I):

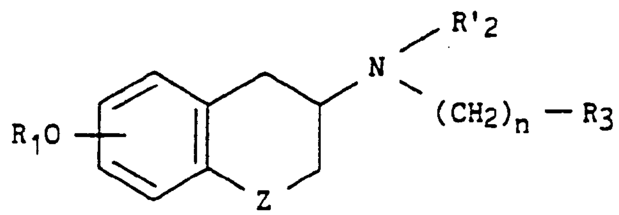

(I/b)

in der $R_1$, Z, $R'_2$, $R_3$ und n die oben angegebenen Bedeutungen besitzen,
oder
b eine Verbindung der Formel (V):

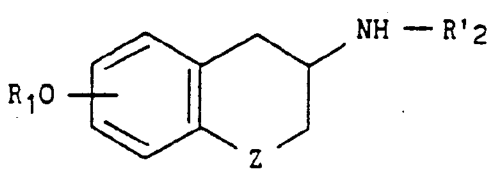

(V)

in der $R_1$, Z und $R'_2$ die oben angegebenen Bedeutungen besitzen, verwendet, welche man

- entweder mit einer Verbindung der Formel (III):

  $R_3$-$(CH_2)_n$-X (III)

  in der $R_3$, n und X die oben angegebenen Bedeutungen besitzen, umsetzt, so daß man ein Derivat der Formel (I/b) erhält, einem Sonderfall der Derivate der Formel (I):

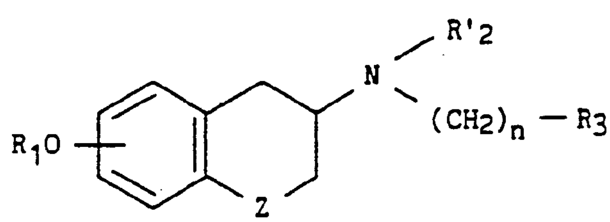

(I/b)

  in der $R_1$, Z, $R'_2$, $R_3$ und n die oben angegebenen Bedeutungen besitzen,
- oder mit einer Verbindung der Formel (III/a):

  NC-$(CH_2)_m$-X (III/a)

in der X die oben angegebenen Bedeutungen besitzt und m eine ganze Zahl mit einem Wert zwischen 1 und 3 darstellt, umsetzt,

so daß man ein Derivat der Formel (I/c) erhält, einem Sonderfall der Derivate der Formel (I):

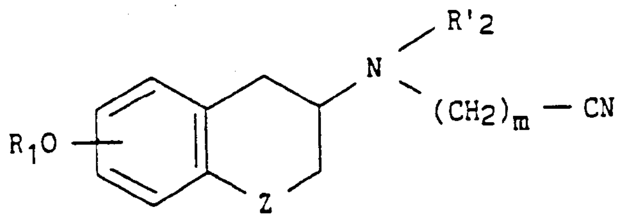

(I/c)

in der $R_1$, Z, $R'_2$ und n die oben angegebenen Bedeutungen besitzen, welche man katalytisch in Gegenwart von Raney-Nickel und Ammoniak oder chemisch durch Lithiumaluminiumhydrid oder Natrium in Alkohol reduziert,
so daß man ein Derivat der Formel (I/d) erhält, einem Sonderfall der Derivate der Formel (I):

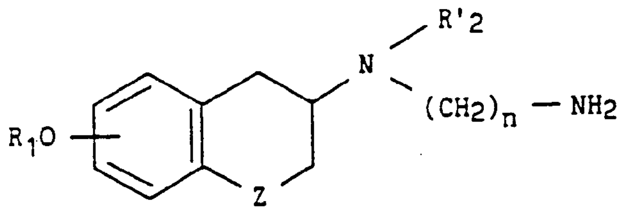

(I/d)

in der $R_1$, Z, $R'_2$ und n die oben angegebenen Bedeutungen besitzen, welches man

. entweder mit einer Verbindung der Formel (VI/A):

$R_{4A}$-$SO_2$-X (VI/A)

in der $R_{4A}$ eine Alkylgruppe oder eine gegebenenfalls durch eine Alkylgruppe substituierte Arylgruppe und X ein Halogenatom darstellen, umsetzt,
zur Bildung eines Derivats der Formel (I/e), einem Sonderfall der Derivate der Formel (I):

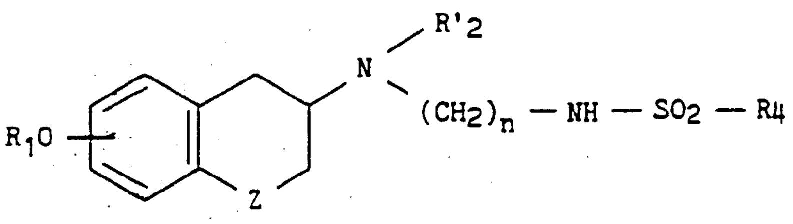

(I/e)

in der $R_1$, Z, $R'_2$, $R_4$ und n die oben angegebenen Bedeutungen besitzen,

. oder mit einer Verbindung der Formel (VI/B):

$R_{4B}$-CO-T (VI/B)

in der $R_{4B}$ eine geradkettige oder verzweigte Alkylgruppe mit 1 bis 4 Kohlenstoffatomen und T eine austretende Gruppe, ausgewählt aus Halogenen oder Niedrigalkoxygruppen bedeuten, oder mit einer Verbindung der Formel (VI/C):

$R_{4B}$-CO-O-CO-$R_{4B}$ (VI/C)

in der R4B die oben angegebenen Bedeutungen besitzen, umsetzt, so daß man eine Verbindung der Formel (I/F):

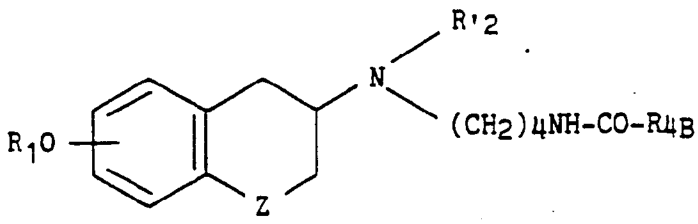

(I/F)

in der $R_1$, Z, $R'_2$, $R_{4B}$ und n die oben angegebenen Bedeutungen besitzen, erhält, einem Sonderfall der Derivate der Formel (I), worin $R_3$ eine durch eine geradkettige oder verzweigte Acylgruppe mit 2 bis 7 Kohlenstoffatomen substituierte Aminogruppe darstellt,

oder

c eine Verbindung der Formel (VII):

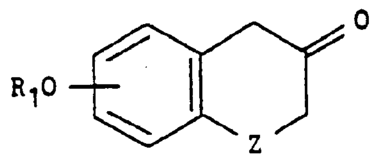

(VII)

in der $R_1$ und Z die bezüglich der Formel (I) angegebenen Bedeutungen besitzen, verwendet, welche man einer reduzierenden Aminierung in Gegenwart eines Derivats der Formel (VIII):

$$R_2\text{-NH-}(CH_2)_n\text{-}R_3 \qquad \text{(VIII)}$$

in der $R_2$, $R_3$ und n die bezüglich der Formel (I) angegebenen Bedeutungen besitzen, unterwirft zur Bildung einer Verbindung der Formel (I), die, wenn $R_2$ ein Wasserstoffatom darstellt, der Einwirkung eines Derivats der Formel (IV):

$$R'_2\text{-X} \qquad \text{(IV)}$$

in der $R'_2$ und X die oben angegebenen Bedeutungen besitzen, unterworfen werden kann,
zur Bildung eines Derivats der Formel (I/b), einem Sonderfall der Derivate der Formel (I):

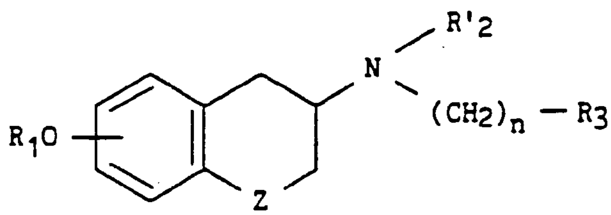

(I/b)

in der $R_1$, Z, $R'_2$, $R_3$ und n die oben angegebenen Bedeutungen besitzen,

oder schließlich

d eine Verbindung der Formel (IX):

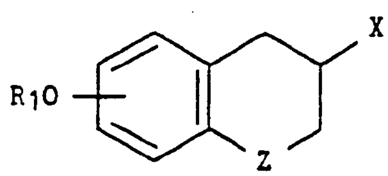

(IX)

in der $R_1$ und Z die bezüglich der Formel (I) angegebenen Bedeutungen besitzen und X ein Halogenatom darstellt, verwendet, welche man der Einwirkung eines Derivats der Formel (X):

$$H_2N\text{-}(CH_2)_n\text{-}R_3 \qquad \text{(X)}$$

in der n und $R_3$ die oben angegebenen Bedeutungen besitzen, unterwirft zur Bildung eines Derivats der Formel (I/a), einem Sonderfall der Derivate der Formel (I):

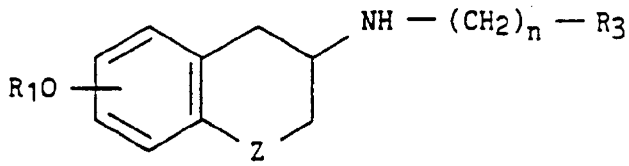

(I/a)

in der $R_1$, Z, n und $R_3$ die oben angegebenen Bedeutungen besitzen, welches man gegebenenfalls der Einwirkung eines Derivates der Formel (IV):

$R'_2$-X (IV)

in der $R'_2$ und X die oben angegebenen Bedeutungen besitzen, unterwirft, zur Bildung eines Derivats der Formel (I/b), einem Sonderfall der Derivate der Formel (I):

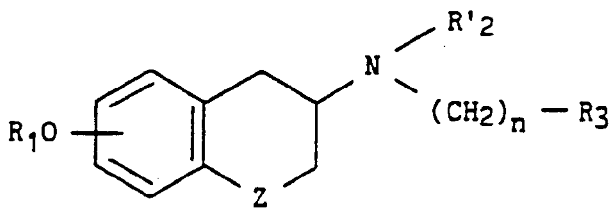

(I/b)

in der $R_1$, Z, $R'_2$, $R_3$ und n die oben angegebenen Bedeutungen besitzen, welche Derivate der Formeln (I/a), (I/b), (I/c), (I/d) und (I/e) man gegebenenfalls mit Hilfe eines klassischen Trennverfahrens in die Isomeren auftrennt, die man mit Hilfe einer klassischen Reinigungsmethode reinigt und gewünschtenfalls mit einer pharmazeutisch annehmbaren Säure in ihre Additionssalze überführt.

2. Verfahren nach Anspruch 1 zur Herstellung von Verbindungen der Formel (I), worin $R_1$ eine Methylgruppe bedeutet.

3. Verfahren nach Anspruch 1 zur Herstellung von Verbindungen, worin $R_2$ eine n-Propylgruppe bedeutet.

4. Verfahren nach Anspruch 1 zur Herstellung von Verbindungen der Formel (I), worin die Gruppe $OR_1$ in der 5-Stellung steht.

5. Verfahren nach Anspruch 1 zur Herstellung von Verbindungen der Formel (I), worin $R_2$ ein Wasserstoffatom bedeutet.

6. Verfahren nach Anspruch 1 zur Herstellung der Verbindung der Formel (I), nämlich 3-[4-[N-Propyl-N-(5-methoxy-chroman-3-yl)-amino]-butyl]-2,4-dioxo-3-azaspiro[4,5]decan, von dessen Isomeren sowie von dessen Additionssalzen mit einer pharmazeutisch annehmbaren Säure.

7. Verfahren nach Anspruch 1 zur Herstellung der Verbindung der Formel (I), nämlich 3⊖-[4-[Nn-Propyl-N-(5-methoxy-chroman-3-yl)-amino]butyl]-2,4-dioxo-3-azaspiro[4,5]decan sowie von dessen Additionssalzen mit einer pharmazeutisch annehmbaren Säure.

8. Verfahren nach Anspruch 1 zur Herstellung der Verbindung der Formel (I), nämlich 3⊕-[4-[Nn-Propyl-N-(5-methoxy-chroman-3-yl)-amino]butyl]-2,4-dioxo-3-azaspiro[4,5]decan sowie von dessen Additionssalzen mit einer pharmazeutisch annehmbaren Säure.

9. Verfahren nach Anspruch 1 zur Herstellung der Verbindung der Formel (I), nämlich 5-Methoxy-3-[N-propyl-N-[2-(4-toluol-sulfonylamino)ethyl]-amino]-chroman, von dessen Isomeren sowie von dessen Additionssalzen mit einer pharmazeutisch annehmbaren Säure.

10. Verfahren nach Anspruch 1 zur Herstellung der Verbindung der Formel (I), nämlich 5-Methoxy-3⊕-[N-propyl-N-[2-(4-toluol-sulfonylamino)ethyl]-amino]-chroman und von dessen Additionssalzen mit einer pharmazeutisch annehmbaren Säure.

11. Verfahren nach Anspruch 1 zur Herstellung der Verbindung der Formel (I), nämlich 5-Methoxy-3⊖-[N-propyl-N-[2-(4-toluol-sulfonylamino)ethyl]-amino]-chroman und von dessen Additionssalzen mit einer pharmazeutisch annehmbaren Säure.

12. Verfahren nach Anspruch 1 zur Herstellung der Verbindung der Formel (I), nämlich 5-Methoxy-3[Nn-propyl-N-[4-(4-toluol-sulfonylamino)butyl]-amino]-chroman und von dessen Isomeren sowie von dessen Additionssalzen mit einer pharmazeutisch annehmbaren Säure.

13. Verfahren nach Anspruch 1 zur Herstellung der Verbindung der Formel (I), nämlich 5-Methoxy-3⊕[Nn-propyl-N-[4-(4-toluol-sulfonylamino)butyl]-amino]-chroman und von dessen Additionssalzen mit einer pharmazeutisch annehmbaren Säure.

14. Verfahren nach Anspruch 1 zur Herstellung der Verbindung der Formel (I), nämlich 5-Methoxy-3⊖-[Nn-propyl-N-[4-(4-toluol-sulfonylamino)-butyl]-amino]-chroman und von dessen Additionssalzen mit einer pharmazeutisch annehmbaren Säure.

15. Verfahren nach Anspruch 1 zur Herstellung der Verbindung der Formel (I), nämlich 3-[4-[N-Propyl-N-(5-methoxy-chroman-3-yl)-amino]-butyl]-2,4-dioxo-3-azabicyclo[3.3.0]octan und von dessen Isomeren und von dessen Additionssalzen mit einer pharmazeutisch annehmbaren Säure.

16. Verfahren nach Anspruch 1 zur Herstellung der Verbindung der Formel (I), nämlich 3⊕-[4-[N-Propyl-N-(5-methoxy-chroman-3-yl)-amino]butyl]-2,4-dioxo-3-azabicyclo[3.3.0]octan und von dessen Additionssalzen mit einer pharmazeutisch annehmbaren Säure.

17. Verfahren nach Anspruch 1 zur Herstellung der Verbindung der Formel (I), nämlich 3⊖-[4-[N-Propyl-N-(5-methoxy-chroman-3-yl]-amino]butyl]-2,4-dioxo-3-azabicyclo[3.3.0]octan und von dessen Additionssalzen mit einer pharmazeutisch annehmbaren Säure.

18. Verfahren zur Herstellung von pharmazeutischen Zubereitungen enthaltend als Wirkstoffmindestens eine Verbindung erhalten nach einem der Ansprüche 1 bis 17 allein oder in Kombination mit einem oder mehreren inerten, nicht toxischen, pharmazeutisch annehmbaren Bindemitteln oder Trägermaterialien.

19. Verfahren zur Herstellung von pharmazeutischen Zubereitungen nach Anspruch 18, enthaltend mindestens einen Wirkstoff erhalten nach einem der Ansprüche 1 bis 17 zur Behandlung der Depression, von Streß, von Psychosen, der Angst, von Schmerzen, der Schizophrenie, der Hypertension, zur Vorbeugung von Atheromen und als Modifizierungsmittel des Ernährungs- und Sexualverhaltens.

## Claims

## Claims for the following Contracting States : AT, LI, NL, BE, DE, FR, IT, SE, CH, DK, GB, LU

1. Compounds of the general formula (I):

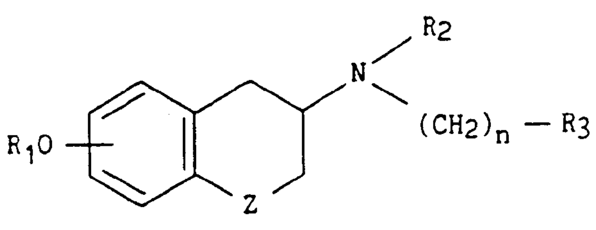

(I)

wherein:

- Z represents an oxygen atom or a sulphur atom,
- $R_1$ represents a hydrogen atom, or a linear or branched ($C_1$-$C_6$)-alkyl grouping,
- $R_2$ represents a hydrogen atom or a linear or branched ($C_1$-$C_6$)-alkyl grouping,
- n is an integer from 1 to 6, and
- $R_3$ represents a nitrile grouping, or an amino grouping optionally substituted by:
  - . a linear or branched acyl grouping containing from 2 to 7 carbon atoms,
  - . an alkylsulphonyl grouping,

. an arylsulphonyl grouping optionally substituted by an alkyl, alkoxy or hydroxy grouping or a halogen atom, or

. a linear or branched ($C_1$-$C_6$)-alkyl grouping,

- or $R_3$ represents any one of the following groupings:

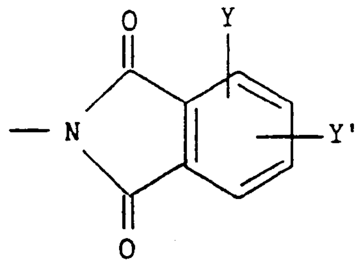 , 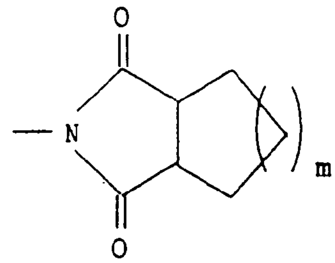 ,

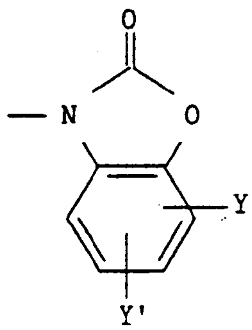 , 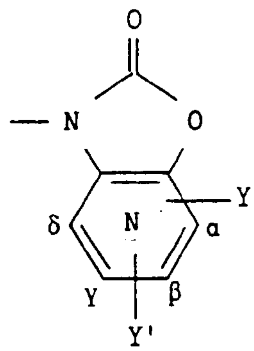 ,

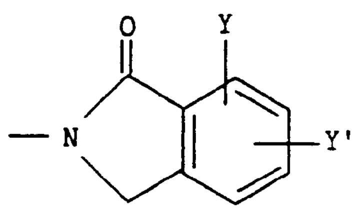 , 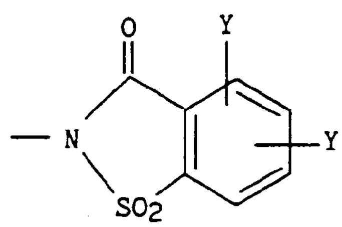 .

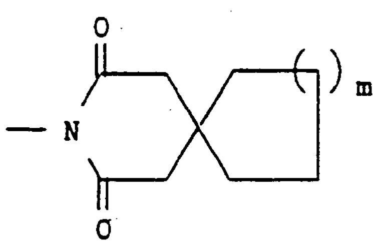 , 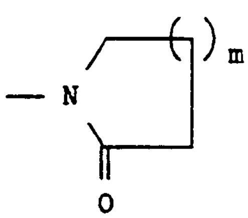 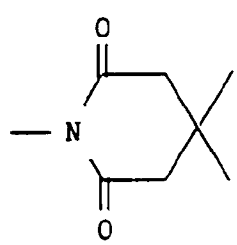 ,

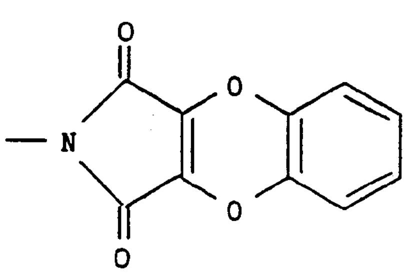 , 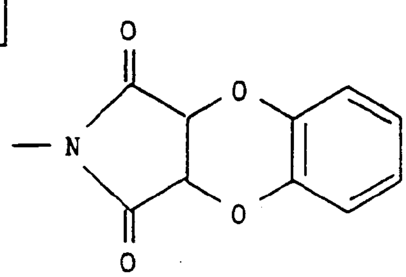 ,

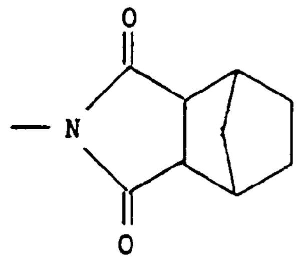 , 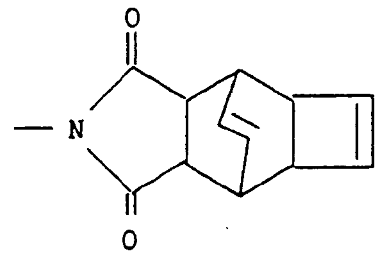

wherein:

- . Y and Y′, which may be identical or different, represent a hydrogen atom, a halogen atom, or an alkyl, alkoxy or hydroxy grouping,
- . m is an integer equal to 1 or 2,
- . and the nitrogen of the pyridine ring is in the α, β, γ or δ position of ring junctions,

their enantiomers, diastereoisomers and epimers and also their addition salts with a pharmaceutically acceptable acid.

2. Compounds according to claim 1 such that $R_1$ is a methyl grouping.

3. Compounds according to claim 1 such that $R_2$ is an n-propyl grouping.

4. Compounds according to claim 1 such that $OR_1$ is in the 5-position.

5. Compounds according to claim 1 such that $R_2$ is a hydrogen atom.

6. Compound according to claim 1 which is 3-[4-[N-propyl- N-(5-methoxychroman-3-yl)-amino]-butyl]-2,4-dioxo-3-azaspiro[4.5]decane, its isomers and also its addition salts with a pharmaceutically acceptable acid.

7. Compound according to claim 1 which is ⊖-3-[4-[N-n-propyl-N-(5-methoxychroman-3-yl)-amino]-butyl]-2,4-dioxo-3-azaspiro[4.5]decane, and also its addition salts with a pharmaceutically acceptable acid.

8. Compound according to claim 1 which is ⊕-3-[4-[N-n-propyl-N-(5-methoxychroman-3-yl)-amino]-butyl]-2,4-dioxo-3-azaspiro[4.5]decane, and also its addition salts with a pharmaceutically acceptable acid.

9. Compound according to claim 1 which is 5-methoxy-3-[N-propyl-N-[2-(4-toluenesulphonylamino)-ethyl]-amino]chroman, its isomers and also its addition salts with a pharmaceutically acceptable acid.

10. Compound according to claim 1 which is ⊕-5-methoxy-3-[N-propyl-N-[2-(4-toluenesulphonylamino)-ethyl]amino]-chroman, and also its addition salts with a pharmaceutically acceptable acid.

11. Compound according to claim 1 which is ⊖-5-methoxy-3-[N-propyl-N-[2-(4-toluenesulphonylamino)-ethyl]-amino]chroman, and also its addition salts with a pharmaceutically acceptable acid.

12. Compound according to claim 1 which is 5-methoxy-3-[N-n-propyl-N-[4-(4-toluenesulphonylamino)-butyl]-amino]chroman, its isomers and also its addition salts with a pharmaceutically acceptable acid.

13. Compound according to claim 1 which is ⊕-5-methoxy-3-[N-n-propyl-N-[4-(4-toluenesulphonylamino)-butyl]amino]-chroman, and also its addition salts with a pharmaceutically acceptable acid.

14. Compound according to claim 1 which is ⊖-5-methoxy-3-[N-n-propyl-N-[4-(4-toluenesulphonylamino)-butyl]amino]-chroman, and also its addition salts with a pharmaceutically acceptable acid.

15. Compound according to claim 1 which is 3-[4-[N-propyl-N-(5-methoxychroman-3-yl)-amino]-butyl]-2,4-dioxo-3-azabicyclo[3.3.0]octane, its isomers and also its addition salts with a pharmaceutically acceptable acid.

16. Compound according to claim 1 which is ⊕-3-[4-[N-propyl-N-(5-methoxychroman-3-yl)-amino]-butyl]-2,4-dioxo-3-azabicyclo[3.3.0]octane, and also its addition salts with a pharmaceutically acceptable acid.

**17.** Compound according to claim 1 which is ⊖-3-[4-[N-propyl-N-(5-methoxychroman-3-yl)-amino]-butyl]-2,4-dioxo-3-azabicyclo[3.3.0]octane, and also its addition salts with a pharmaceutically acceptable acid.

**18.** Process for the preparation of the compounds of formula (I), characterised in that there is used as starting material:

a a compound of formula (II):

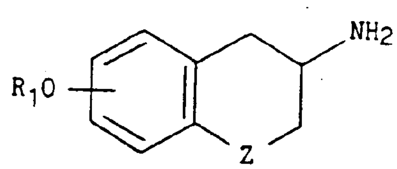

(II),

wherein $R_1$ and Z are as defined in formula (I), which is reacted with a compound of formula (III):

$R_3$-$(CH_2)_n$-X (III),

wherein $R_3$ and n are as defined in formula (I) and X is a halogen atom,
to yield a compound of formula (I/a), a particular case of the compounds of formula (I):

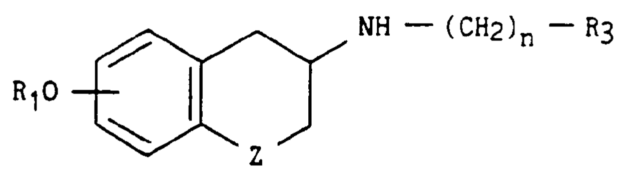

(I/a),

wherein $R_1$, $R_3$, Z and n are as defined in formula (I), which is optionally subjected to the action of a compound of formula (IV):

$R'_2$-X (IV),

wherein $R'_2$ represents a linear or branched ($C_1$-$C_6$)-alkyl grouping and X represents a halogen atom, to yield a compound of formula (I/b), a particular case of the compounds of formula (I):

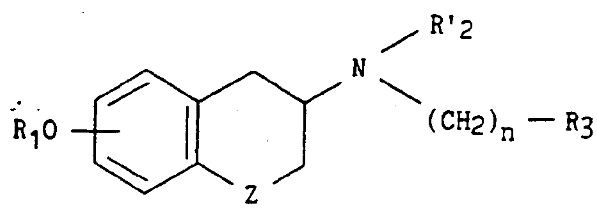

(I/b)

wherein $R_1$, Z, $R'_2$, $R_3$ and n are as defined above,
or
b a compound of formula (V):

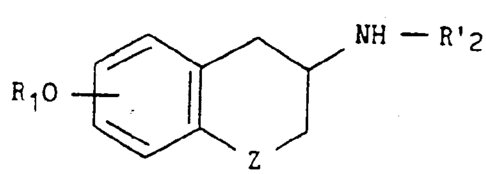

(V),

wherein $R_1$, Z and $R'_2$ are as defined above, which is reacted:

- either with a compound of formula (III):

  $R_3$-$(CH_2)_n$-X (III),

  wherein $R_3$, n and X are as defined above,
  to yield a compound of formula (I/b), a particular case of the compounds of formula (I):

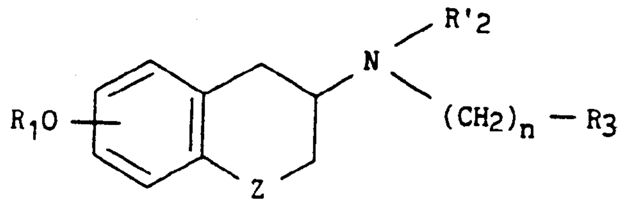

(I/b),

wherein $R_1$, Z, $R'_2$, $R_3$ and n are as defined above,

- or with a compound of formula (III/a):

$NC-(CH_2)_m-X$ (III/a),

wherein X is as defined above and m is an integer from 1 to 3,

to yield a compound of formula (I/c), a particular case of the compounds of formula (I):

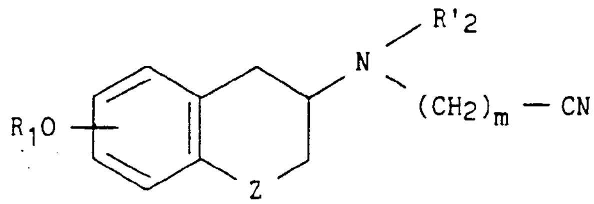

(I/c),

wherein $R_1$, Z, $R'_2$ and m are as defined above, which is reduced catalytically in the presence of Raney nickel and ammonia, or chemically by lithium aluminium hydride or by sodium in alcohol, to yield the compound of formula (I/d), a particular case of the compounds of formula (I):

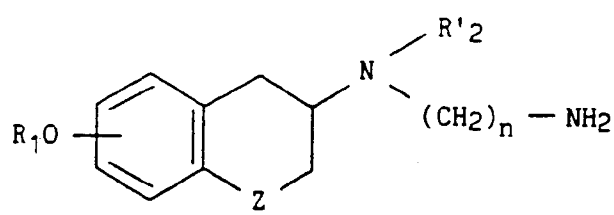

(I/d),

wherein $R_1$, Z, $R'_2$ and n are as defined above, which is reacted:

. either with a compound of formula (VI/A):

$R_{4A}-SO_2-X$ (VI/A),

wherein $R_{4A}$ is an alkyl grouping or an aryl grouping optionally substituted by an alkyl grouping and X is a halogen atom, to yield a compound of formula (I/e), a particular case of the compounds of formula (I):

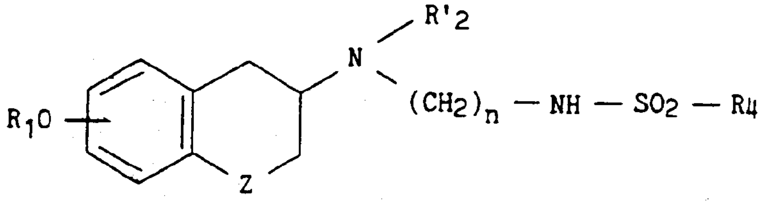

(I/e),

wherein $R_1$, Z, $R'_2$, $R_4$ and n are as defined above,

. or with a compound of formula (VI/B):

$R_{4B}-CO-T$ (VI/B)

wherein $R_{4B}$ represents a linear or branched alkyl grouping having from 1 to 6 carbon atoms and T is a leaving group selected from halogen, or lower alkoxy or with a compound of formula (VI/C):

$R_{4B}$-CO-O-CO-$R_{4B}$ (VI/C),

wherein $R_{4B}$ is as defined above,
to yield a compound of formula (I/F):

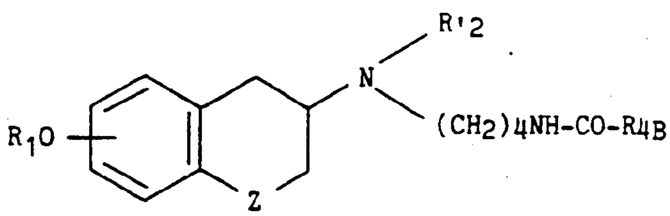

(I/F),

wherein $R_1$, Z, $R'_2$, $R_{4B}$ and n are as defined above, a particular case of the compounds of formula (I) in which $R_3$ represents an amino grouping substituted by a linear or branched acyl grouping having from 2 to 7 carbon atoms,
or
c a compound of formula (VII):

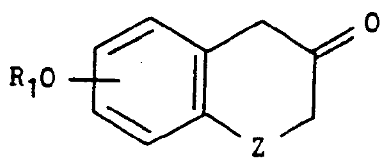

(VII),

wherein $R_1$ and Z are as defined in formula (I), which is subjected to a reductive amination in the presence of a compound of formula (VIII):

$R_2$-NH-$(CH_2)_n$-$R_3$ (VIII),

wherein $R_2$, $R_3$ and n are as defined in formula (I), to yield a compound of formula (I) which, when $R_2$ represents a hydrogen atom, may be subjected to the action of a compound of formula (IV):

$R'_2$-X (IV)

wherein $R'_2$ and X are as defined above, to yield a compound of formula (I/b), a particular case of the compounds of formula (I):

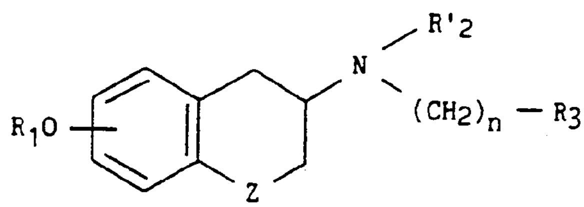

(I/b),

wherein $R_1$, Z, $R'_2$, $R_3$ and n are as defined above,
or
d a compound of formula (IX):

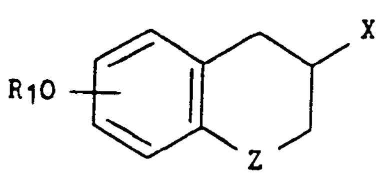

(IX),

wherein $R_1$ and Z are as defined in formula (I) and X is a halogen atom, which is subjected to the action of a compound of formula (X):

$H_2N$-$(CH_2)_n$-$R_3$ (X),

wherein n and $R_3$ are as defined above,
to yield a compound of formula (I/a), a particular case of the compounds of formula (I):

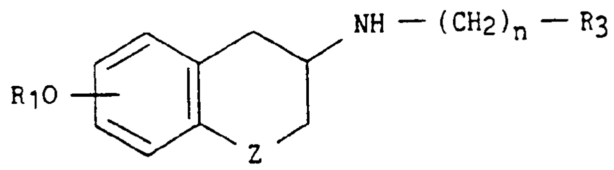

(I/a)

wherein $R_1$, Z, n and $R_3$ are as defined above, which is optionally subjected to the action of a compound of formula (IV)

$R'_2$-X (IV)

wherein $R'_2$ and X are as defined above,
to yield a compound of formula (I/b), a particular case of the compounds of formula (I):

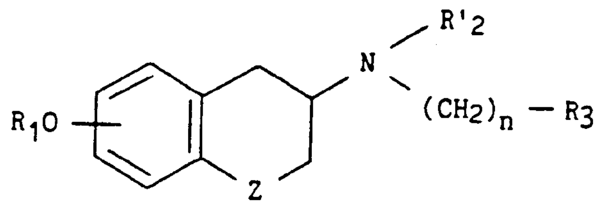

(I/b)

wherein $R_1$, Z, $R'_2$, $R_3$ and n are as defined above,
the isomers of which compounds of formulae (I/a), (I/b), (I/c), (I/d) and (I/e) are optionally separated in accordance with a conventional separation technique, and which compounds are purified by a conventional purification technique and are converted, if desired, into their addition salts with a pharmaceutically acceptable acid.

**19.** Pharmaceutical compositions containing as active ingredient at least one compound according to any one of claims 1 to 17, alone or in combination with one or more pharmaceutically acceptable inert, non-toxic excipients or carriers.

**20.** Pharmaceutical compositions according to claim 19 containing at least one active ingredient according to any one of claims 1 to 17, for use in the treatment of depression, stress, psychoses, anxiety, pain, schizophrenia, hypertension, prevention of atheromas and as modifiers of eating and sexual behaviour.

**Claims for the following Contracting State : ES**

**1.** Process for the preparation of compounds of the general formula (I):

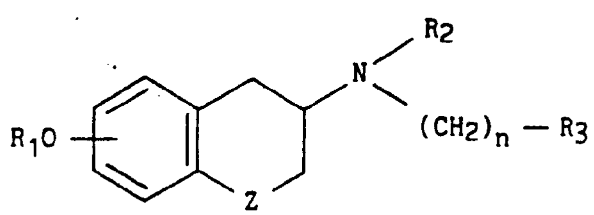

(I)

wherein:
- Z represents an oxygen atom or a sulphur atom,
- $R_1$ represents a hydrogen atom, or a linear or branched ($C_1$-$C_6$)-alkyl grouping,
- $R_2$ represents a hydrogen atom or a linear or branched ($C_1$-$C_6$)-alkyl grouping,
- n is an integer from 1 to 6, and
- $R_3$ represents a nitrile grouping, or an amino grouping optionally substituted by:
  - a linear or branched acyl grouping containing from 2 to 7 carbon atoms,
  - an alkylsulphonyl grouping,
  - an arylsulphonyl grouping optionally substituted by an alkyl, alkoxy or hydroxy grouping or a halogen atom, or

- a linear or branched ($C_1$-$C_6$)-alkyl grouping,
- or $R_3$ represents any one of the following groupings:

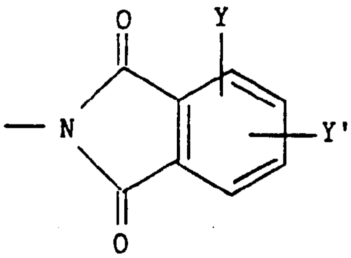 , 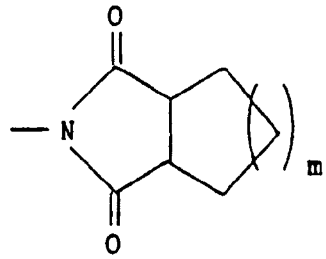 ,

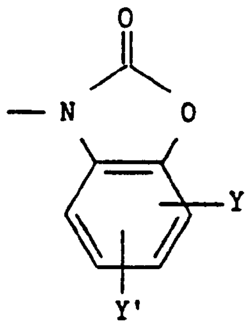 , 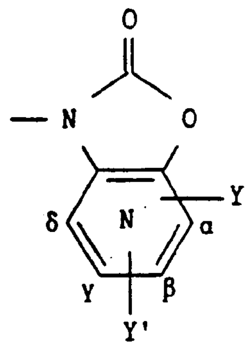 ,

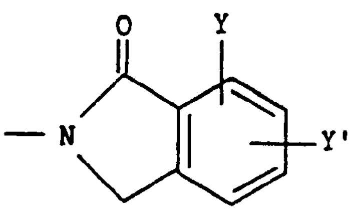 , 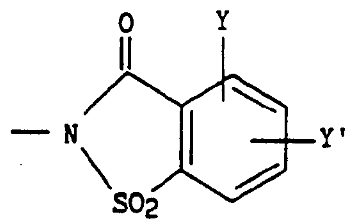 ,

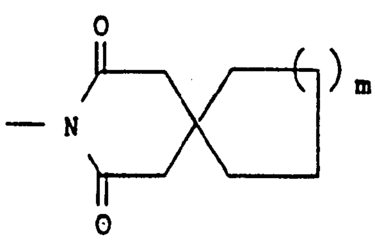 , 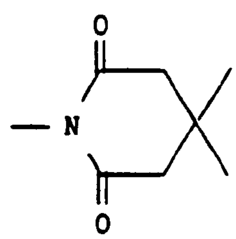 ,

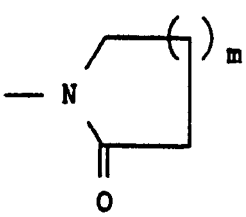

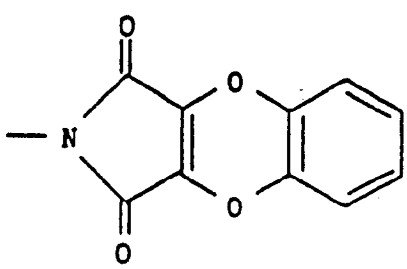 , 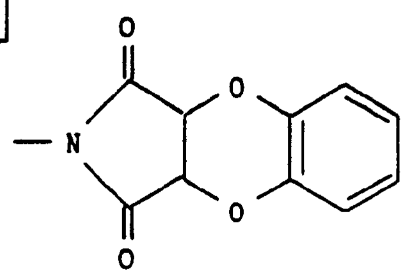 ,

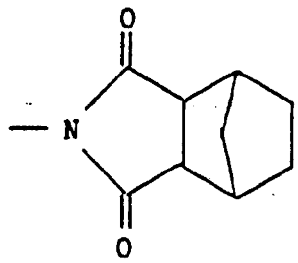 , 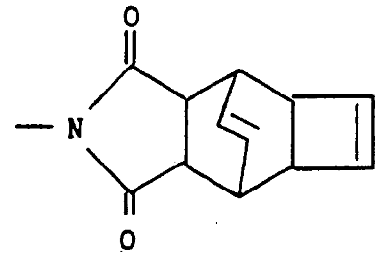

wherein:

. Y and Y′, which may be identical or different, represent a hydrogen atom, a halogen atom, or an alkyl, alkoxy or hydroxy grouping,

. m is an integer equal to 1 or 2,

. and the nitrogen of the pyridine ring is in the α, β, γ or δ position of ring junctions,

their enantiomers, diastereoisomers and epimers and also their addition salts with a pharmaceutically acceptable acid,

characterised in that there is used as starting material:

a a compound of formula (II):

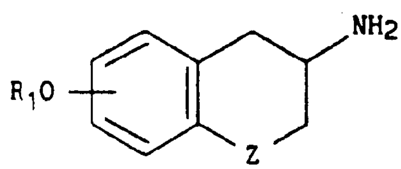

(II),

wherein $R_1$ and Z are as defined in formula (I), which is reacted with a compound of formula (III):

$R_3$-$(CH_2)_n$-X (III)

, wherein $R_3$ and n are as defined in formula (I) and X is a halogen atom,

to yield a compound of formula (I/a), a particular case of the compounds of formula (I):

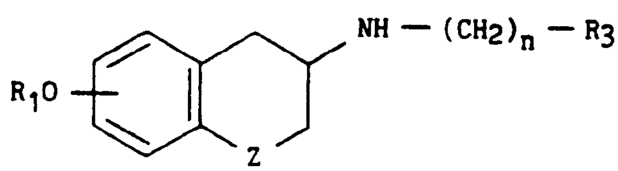

(I/a),

wherein $R_1$, $R_3$, Z and n are as defined in formula (I), which is optionally subjected to the action of a compound of formula (IV):

$R'_2$-X (IV),

wherein $R'_2$ represents a linear or branched ($C_1$-$C_6$)-alkyl grouping and X represents a halogen atom, to yield a compound of formula (I/b), a particular case of the compounds of formula (I):

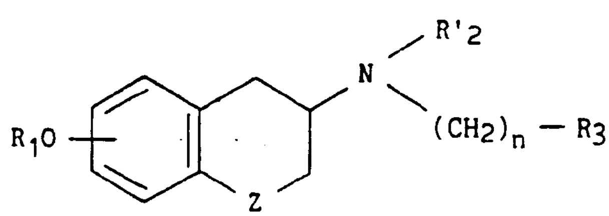

(I/b)

wherein R, Z, $R'_2$, $R_3$ and n are as defined above,

or

b a compound of formula (V):

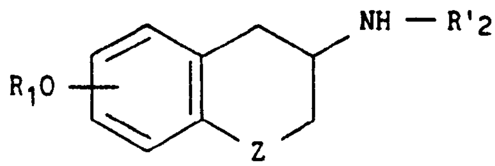 (V),

wherein $R_1$, Z and $R'_2$ are as defined above, which is reacted:

- either with a compound of formula (III):

$R_3$-$(CH_2)_n$-X (III),

wherein $R_3$, n and X are as defined above, to yield a compound of formula (I/b), a particular case of the compounds of formula (I):

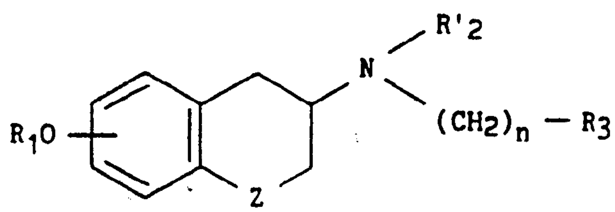 (I/b),

wherein $R_1$, Z, $R'_2$, $R_3$ and n are as defined above,

- or with a compound of formula (III/a):

NC-$(CH_2)_m$-X (III/a),

wherein X is as defined above and m is an integer from 1 to 3,
to yield a compound of formula (I/c), a particular case of the compounds of formula (I):

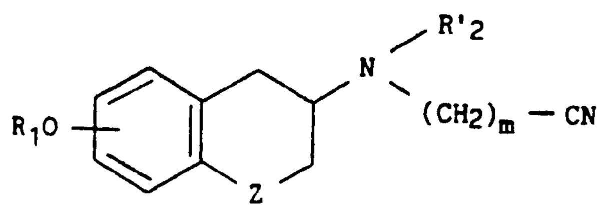 (I/c),

wherein $R_1$, Z, $R'_2$ and m are as defined above,
which is reduced catalytically in the presence of Raney nickel and ammonia, or chemically by lithium aluminium hydride or by sodium in alcohol,
to yield the compound of formula (I/d), a particular case of the compounds of formula (I):

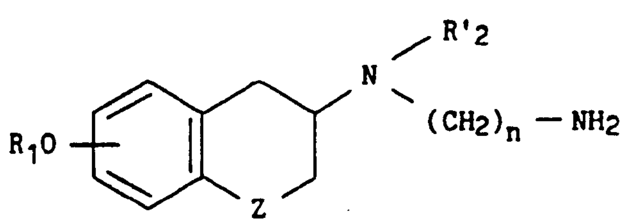 (I/d),

wherein $R_1$, Z, $R'_2$ and n are as defined above, which is reacted:

. either with a compound of formula (VI/A):

$R_{4A}$-$SO_2$-X (VI/A),

wherein $R_{4A}$ is an alkyl grouping or an aryl grouping optionally substituted by an alkyl grouping and X is a halogen atom,
to yield a compound of formula (I/e), a particular case of the compounds of formula (I):

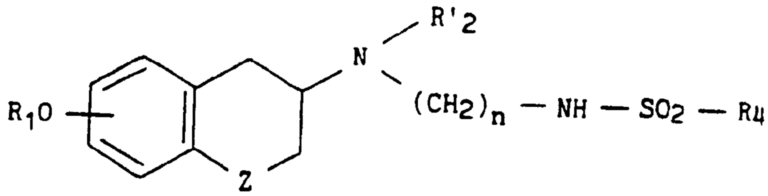 (I/e),

wherein $R_1$, Z, $R'_2$, $R_4$ and n are as defined above,
. or with a compound of formula (VI/B):

$R_{4B}$-CO-T (VI/B)

wherein $R_{4B}$ represents a linear or branched alkyl grouping having from 1 to 6 carbon atoms and T is a leaving group selected from halogen, or lower alkoxy or with a compound of formula (VI/C):

$R_{4B}$-CO-O-CO-$R_{4B}$ (VI/C),

wherein $R_{4B}$ is as defined above,
to yield a compound of formula (I/F):

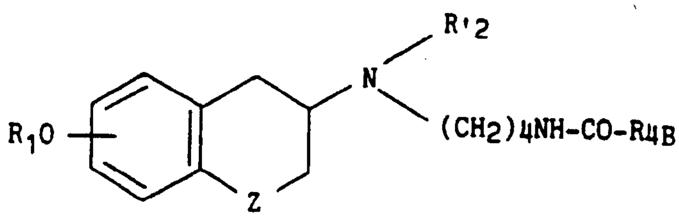 (I/F),

wherein $R_1$, Z, $R'_2$, $R_{4B}$ and n are as defined above, a particular case of the compounds of formula (I) in which $R_3$ represents an amino grouping substituted by a linear or branched acyl grouping having from 2 to 7 carbon atoms,
or
c a compound of formula (VII):

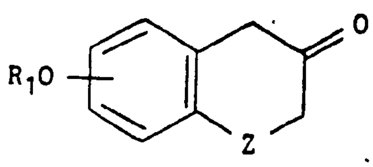 (VII),

wherein $R_1$ and Z are as defined in formula (I), which is subjected to a reductive amination in the presence of a compound of formula (VIII):

$R_2$-NH-$(CH_2)_n$-$R_3$ (VIII),

wherein $R_2$, $R_3$ and n are as defined in formula (I), to yield a compound of formula (I) which, when $R_2$ represents a hydrogen atom, may be subjected to the action of a compound of formula (IV):

$R'_2$-X (IV)

wherein $R'_2$ and X are as defined above,
to yield a compound of formula (I/b), a particular case of the compounds of formula (I):

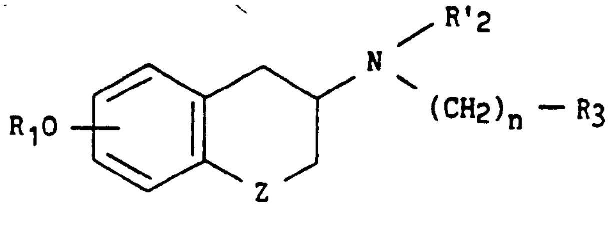 (I/b),

wherein $R_1$, Z, $R'_2$, $R_3$ and n are as defined above,
or

d a compound of formula (IX):

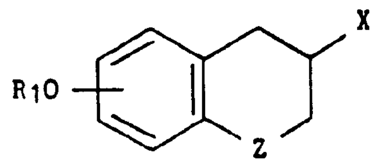

(IX),

wherein $R_1$ and Z are as defined in formula (I) and X is a halogen atom, which is subjected to the action of a compound of formula (X):

$$H_2N\text{-}(CH_2)_n\text{-}R_3 \qquad (X),$$

wherein n and $R_3$ are as defined above,
to yield a compound of formula (I/a), a particular case of the compounds of formula (I):

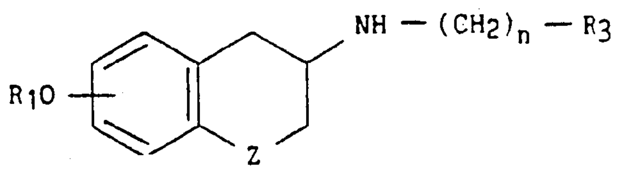

(I/a)

wherein $R_1$, Z, n and $R_3$ are as defined above, which is optionally subjected to the action of a compound of formula (IV)

$$R'_2\text{-}X \qquad (IV)$$

wherein $R'_2$ and X are as defined above,
to yield a compound of formula (I/b), a particular case of the compounds of formula (I):

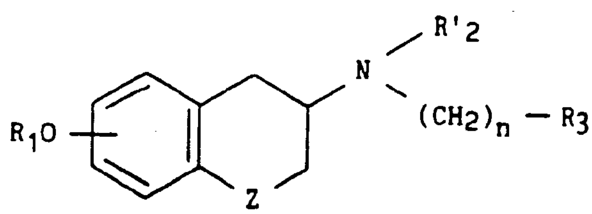

(I/b)

wherein $R_1$, Z, $R'_2$, $R_3$ and n are as defined above,
the isomers of which compounds of formulae (I/a), (I/b), (I/c), (I/d) and (I/e) are optionally separated in accordance with a conventional separation technique, and which compounds are purified by a conventional purification technique and are converted, if desired, into their addition salts with a pharmaceutically acceptable acid.

2. Process for the preparation according to claim 1 of compounds of formula (I) such that $R_1$ is a methyl grouping.

3. Process for the preparation according to claim 1 such that $R_2$ is an n-propyl grouping.

4. Process for the preparation according to claim 1 of compounds of formula (I) such that the grouping $OR_1$ is in the 5-position.

5. Process for the preparation according to claim 1 of compounds of formula (I) such that $R_2$ is a hydrogen atom.

6. Process for the preparation according to claim 1 of the compound of formula (I) which is 3-[4-[N-propyl-N-(5-methoxychroman-3-yl)-amino]-butyl]-2,4-dioxo-3-azaspiro[4.5]decane, its isomers and also its addition salts with a pharmaceutically acceptable acid.

7. Process for the preparation according to claim 1 of the compound of formula (I) which is ⊖-3-[4-[N-n-propyl-N-(5-methoxychroman-3-yl)-amino]-butyl]-2,4-dioxo-3-azaspiro[4.5]decane, and also its addition salts with a pharmaceutically acceptable acid.

8. Process for the preparation according to claim 1 of the compound of formula (I) which is ⊕-3-[4-[N-n-propyl-N-(5-methoxychroman-3-yl)-amino]-butyl]-2,4-dioxo-3-azaspiro[4.5]decane, and also its addition salts with a pharmaceutically acceptable acid.

9. Process for the preparation according to claim 1 of the compound of formula (I) which is 5-methoxy-3-[N-propyl-N-[2-(4-toluenesulphonylamino)-ethyl]-amino]chroman, its isomers and also its addition salts with a pharmaceutically acceptable acid.

10. Process for the preparation according to claim 1 of the compound of formula (I) which is ⊕-5-methoxy-3-[N-propyl-N-[2-(4-toluenesulphonylamino)-ethyl]-amino]chroman, and also its addition salts with a pharmaceutically acceptable acid.

11. Process for the preparation according to claim 1 of the compound of formula (I) which is ⊖-5-methoxy-3-[N-propyl-N-[2-(4-toluenesulphonylamino)-ethyl]-amino]chroman, and also its addition salts with a pharmaceutically acceptable acid.

12. Process for the preparation according to claim 1 of the compound of formula (I) which is 5-methoxy-3-[N-n-propyl-N-[4-(4-toluenesulphonylamino)-butyl]-amino]chroman, its isomers and also its addition salts with a pharmaceutically acceptable acid.

13. Process for the preparation according to claim 1 of the compound of formula (I) which is ⊕-5-methoxy-3-[N-n-propyl-N-[4-(4-toluenesulphonylamino)-butyl]-amino]chroman, and also its addition salts with a pharmaceutically acceptable acid.

14. Process for the preparation according to claim 1 of the compound of formula (I) which is ⊖-5-methoxy-3-[N-n-propyl-N-[4-(4-toluenesulphonylamino)-butyl]-amino]chroman, and also its addition salts with a pharmaceutically acceptable acid.

15. Process for the preparation according to claim 1 of the compound of formula (I) which is 3-[4-[N-propyl-N-(5-methoxychroman-3-yl)-amino]-butyl]-2,4-dioxo-3-azabicyclo[3.3.0]octane, its isomers and also its addition salts with a pharmaceutically acceptable acid.

16. Process for the preparation according to claim 1 of the compound of formula (I) which is ⊕-3-[4-[N-propyl-N-(5-methoxychroman-3-yl)-amino]-butyl]-2,4-dioxo-3-azabicyclo[3.3.0]octane, and also its addition salts with a pharmaceutically acceptable acid.

17. Process for the preparation according to claim 1 of the compound of formula (I) which is ⊖-3-[4-[N-propyl-N-(5-methoxychroman-3-yl)-amino]-butyl]-2,4-dioxo-3-azabicyclo[3.3.0]octane, and also its addition salts with a pharmaceutically acceptable acid.

18. Process for the preparation of pharmaceutical compositions containing as active ingredient at least one compound obtained according to any one of claims 1 to 17, alone or in combination with one or more pharmaceutically acceptable inert, non-toxic excipients or carriers.

19. Process for the preparation of pharmaceutical compositions obtained according to claim 18 containing at least one active ingredient obtained according to any one of claims 1 to 17, for use in the treatment of depression, stress, psychoses, anxiety, pain, schizophrenia, hypertension, prevention of atheromas and as modifiers of eating and sexual behaviour.

**Claims for the following Contracting State : GR**

1. Process for the preparation of compounds of the general formula (I):

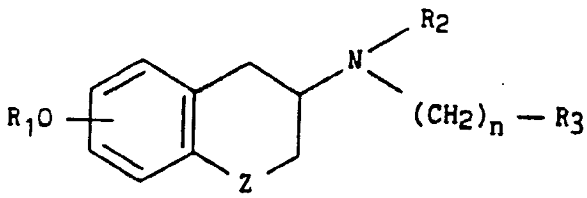 (I)

wherein:

- Z represents an oxygen atom or a sulphur atom,
- $R_1$ represents a hydrogen atom, or a linear or branched ($C_1$-$C_6$)-alkyl grouping,
- $R_2$ represents a hydrogen atom or a linear or branched ($C_1$-$C_6$)-alkyl grouping,
- n is an integer from 1 to 6, and
- $R_3$ represents a nitrile grouping, or an amino grouping optionally substituted by:
  . a linear or branched acyl grouping containing from 2 to 7 carbon atoms,
  . an alkylsulphonyl grouping,
  . an arylsulphonyl grouping optionally substituted by an alkyl, alkoxy or hydroxy grouping or a halogen atom, or
  . a linear or branched ($C_1$-$C_6$)-alkyl grouping,
- or $R_3$ represents any one of the following groupings:

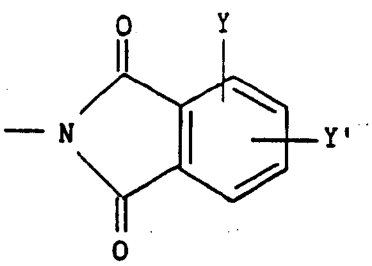 ,

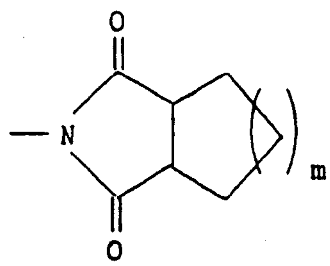 ,

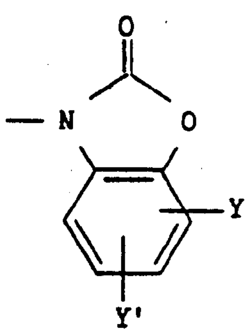 ,

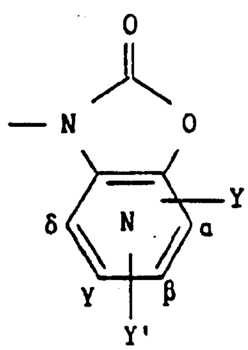 ,

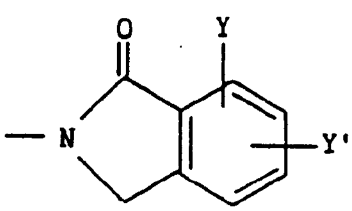 ,

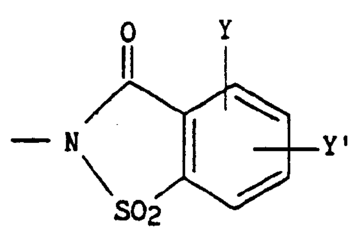 ,

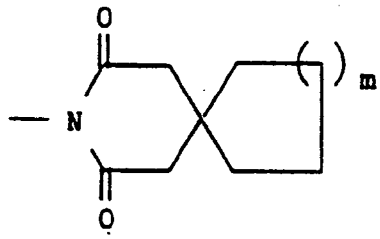

,

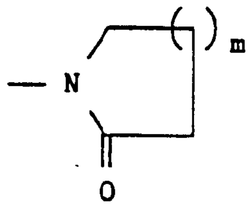

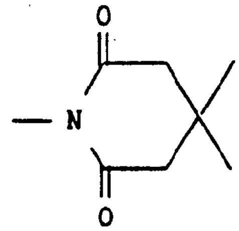

,

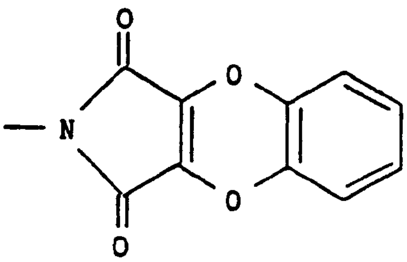

,

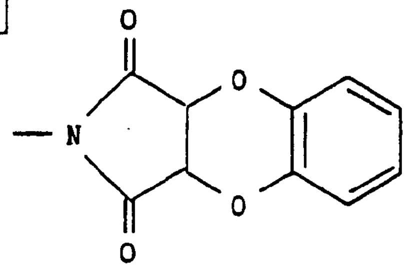

,

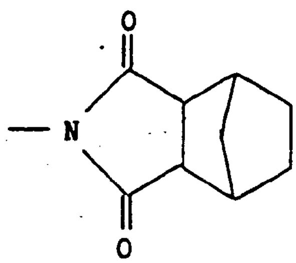

,

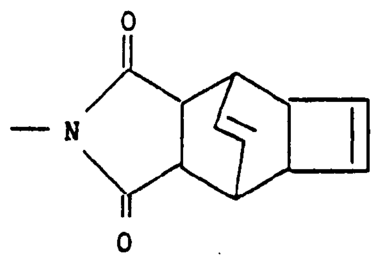

wherein:

. Y and Y′, which may be identical or different, represent a hydrogen atom, a halogen atom, or an alkyl, alkoxy or hydroxy grouping,

. m is an integer equal to 1 or 2,

. and the nitrogen of the pyridine ring is in the α, β, γ or δ position of ring junctions,

their enantiomers, diastereoisomers and epimers and also their addition salts with a pharmaceutically acceptable acid,

characterised in that there is used as starting material:

a a compound of formula (II):

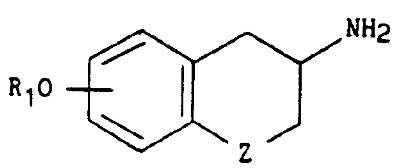

(II),

wherein $R_1$ and Z are as defined in formula (I), which is reacted with a compound of formula (III):

$$R_3\text{-}(CH_2)_n\text{-}X \qquad (III),$$

wherein $R_3$ and n are as defined in formula (I) and X is a halogen atom,

to yield a compound of formula (I/a), a particular case of the compounds of formula (I):

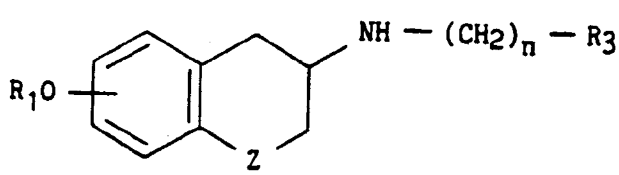

(I/a),

wherein $R_1$, $R_3$, Z and n are as defined in formula (I), which is optionally subjected to the action of a compound of formula (IV):

$$R'_2\text{-}X \qquad (IV),$$

wherein $R'_2$ represents a linear or branched ($C_1$-$C_6$)-alkyl grouping and X represents a halogen atom, to yield a compound of formula (I/b), a particular case of the compounds of formula (I):

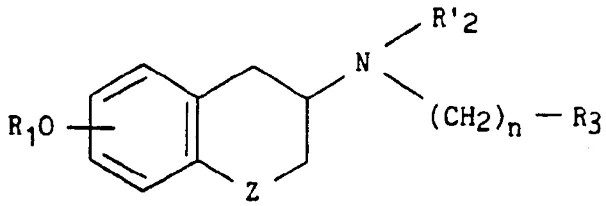

(I/b)

wherein R, Z, $R'_2$, $R_3$ and n are as defined above,

or

b a compound of formula (V):

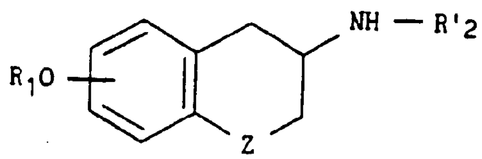

(V),

wherein $R_1$, Z and $R'_2$ are as defined above, which is reacted:

- either with a compound of formula (III):

$$R_3\text{-}(CH_2)_n\text{-}X \qquad (III),$$

wherein $R_3$, n and X are as defined above,
to yield a compound of formula (I/b), a particular case of the compounds of formula (I):

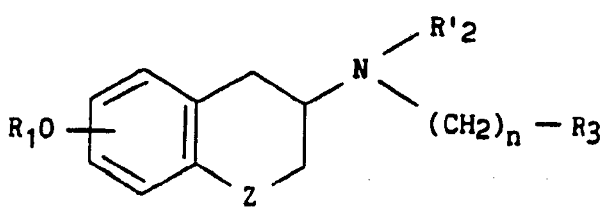

(I/b),

wherein $R_1$, Z, $R'_2$, $R_3$ and n are as defined above,

- or with a compound of formula (III/a):

$$NC\text{-}(CH_2)_m\text{-}X \qquad (III/a),$$

wherein X is as defined above and m is an integer from 1 to 3,
to yield a compound of formula (I/c), a particular case of the compounds of formula (I):

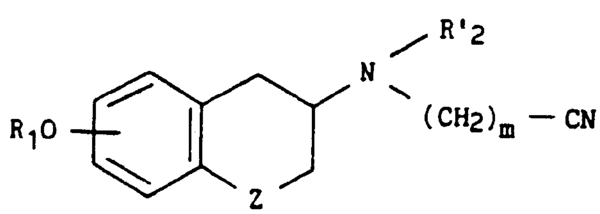

(I/c),

wherein $R_1$, Z, $R'_2$ and m are as defined above,
which is reduced catalytically in the presence of Raney nickel and ammonia, or chemically by lithium aluminium hydride or by sodium in alcohol,
to yield the compound of formula (I/d), a particular case of the compounds of formula (I):

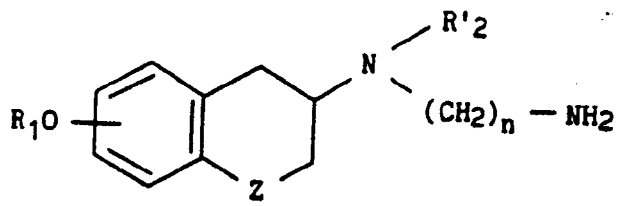

(I/d),

wherein $R_1$, Z, $R'_2$ and n are as defined above, which is reacted:

. either with a compound of formula (VI/A):

$R_{4A}$-$SO_2$-X (VI/A),

wherein $R_{4A}$ is an alkyl grouping or an aryl grouping optionally substituted by an alkyl grouping and X is a halogen atom,

to yield a compound of formula (I/e), a particular case of the compounds of formula (I):

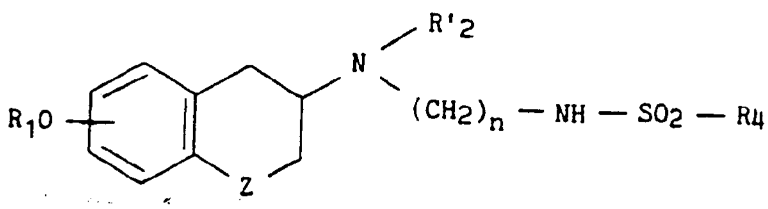

(I/e),

wherein $R_1$, Z, $R'_2$, $R_4$ and n are as defined above,

. or with a compound of formula (VI/B):

$R_{4B}$-CO-T (VI/B)

wherein $R_{4B}$ represents a linear or branched alkyl grouping having from 1 to 6 carbon atoms and T is a leaving group selected from halogen, or lower alkoxy or with a compound of formula (VI/C):

$R_{4B}$-CO-O-CO-$R_{4B}$ (VI/C),

wherein $R_{4B}$ is as defined above,

to yield a compound of formula (I/F):

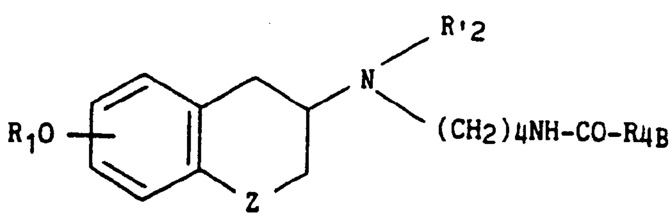

(I/F),

wherein $R_1$, Z, $R'_2$, $R_{4B}$ and n are as defined above, a particular case of the compounds of formula (I) in which $R_3$ represents an amino grouping substituted by a linear or branched acyl grouping having from 2 to 7 carbon atoms,

or

c a compound of formula (VII):

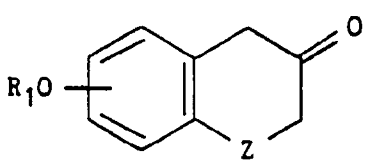

(VII),

wherein $R_1$ and Z are as defined in formula (I), which is subjected to a reductive amination in the presence of a compound of formula (VIII):

$R_2$-NH-$(CH_2)_n$-$R_3$ (VIII),

wherein $R_2$, $R_3$ and n are as defined in formula (I), to yield a compound of formula (I) which, when $R_2$ represents a hydrogen atom, may be subjected to the action of a compound of formula (IV):

$R'_2$-X (IV)

wherein $R'_2$ and X are as defined above,
to yield a compound of formula (I/b), a particular case of the compounds of formula (I):

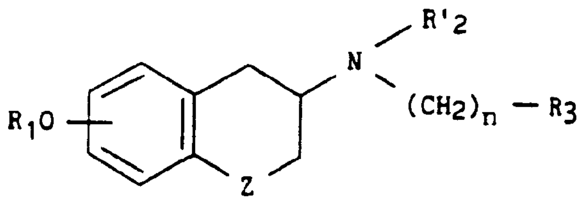

(I/b),

wherein $R_1$, Z, $R'_2$, $R_3$ and n are as defined above,
or
d a compound of formula (IX):

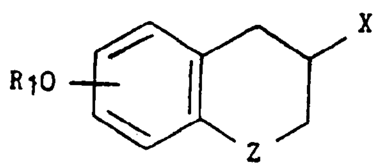

(IX),

wherein $R_1$ and Z are as defined in formula (I) and X is a halogen atom, which is subjected to the action of a compound of formula (X):

$H_2N-(CH_2)_n-R_3$ (X),

wherein n and $R_3$ are as defined above,
to yield a compound of formula (I/a), a particular case of the compounds of formula (I):

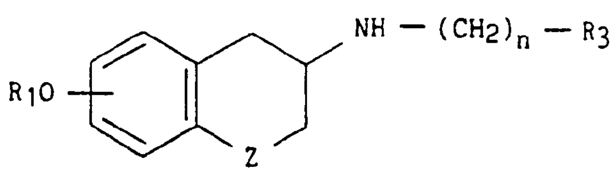

(I/a)

wherein $R_1$, Z, n and $R_3$ are as defined above, which is optionally subjected to the action of a compound of formula (IV)

$R'_2$-X (IV)

wherein $R'_2$ and X are as defined above,
to yield a compound of formula (I/b), a particular case of the compounds of formula (I):

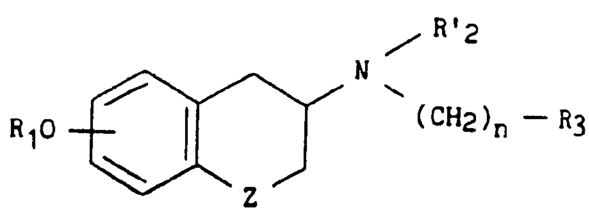

(I/b)

wherein $R_1$, Z, $R'_2$, $R_3$ and n are as defined above,
the isomers of which compounds of formulae (I/a), (I/b), (I/c), (I/d) and (I/e) are optionally separated in accordance with a conventional separation technique, and which compounds are purified by a conventional purification technique and are converted, if desired, into their addition salts with a pharmaceutically acceptable acid.

2. Process for the preparation according to claim 1 of compounds of formula (I) such that $R_1$ is a methyl grouping.

3. Process for the preparation according to claim 1 such that $R_2$ is an n-propyl grouping.

4. Process for the preparation according to claim 1 of compounds of formula (I) such that the grouping $OR_1$ is in the 5-position.

5. Process for the preparation according to claim 1 of compounds of formula (I) such that $R_2$ is a hydrogen atom.

6. Process for the preparation according to claim 1 of the compound of formula (I) which is 3-[4-[N-propyl-N-(5-methoxychroman-3-yl)-amino]-butyl]-2,4-dioxo-3-azaspiro[4.5]decane, its isomers and also its addition salts with a pharmaceutically acceptable acid.

7. Process for the preparation according to claim 1 of the compound of formula (I) which is ⊖-3-[4-[N-n-propyl-N-(5-methoxychroman-3-yl)-amino]-butyl]-2,4-dioxo-3-azaspiro[4.5]decane, and also its addition salts with a pharmaceutically acceptable acid.

8. Process for the preparation according to claim 1 of the compound of formula (I) which is ⊕-3-[4-[N-n-propyl-N-(5-methoxychroman-3-yl)-amino]-butyl]-2,4-dioxo-3-azaspiro[4.5]decane, and also its addition salts with a pharmaceutically acceptable acid.

9. Process for the preparation according to claim 1 of the compound of formula (I) which is 5-methoxy-3-[N-propyl-N-[2-(4-toluenesulphonylamino)-ethyl]-amino]chroman, its isomers and also its addition salts with a pharmaceutically acceptable acid.

10. Process for the preparation according to claim 1 of the compound of formula (I) which is ⊕-5-methoxy-3-[N-propyl-N-[2-(4-toluenesulphonylamino)-ethyl]-amino]chroman, and also its addition salts with a pharmaceutically acceptable acid.

11. Process for the preparation according to claim 1 of the compound of formula (I) which is ⊖-5-methoxy-3-[N-propyl-N-[2-(4-toluenesulphonylamino)-ethyl]-amino]chroman, and also its addition salts with a pharmaceutically acceptable acid.

12. Process for the preparation according to claim 1 of the compound of formula (I) which is 5-methoxy-3-[N-n-propyl-N-[4-(4-toluenesulphonylamino)-butyl]-amino]chroman, its isomers and also its addition salts with a pharmaceutically acceptable acid.

13. Process for the preparation according to claim 1 of the compound of formula (I) which is ⊕-5-methoxy-3-[N-n-propyl-N-[4-(4-toluenesulphonylamino)-butyl]-amino]chroman, and also its addition salts with a pharmaceutically acceptable acid.

14. Process for the preparation according to claim 1 of the compound of formula (I) which is ⊖-5-methoxy-3-[N-n-propyl-N-[4-(4-toluenesulphonylamino)-butyl]-amino]-chroman, and also its addition salts with a pharmaceutically acceptable acid.

15. Process for the preparation according to claim 1 of the compound of formula (I) which is 3-[4-[N-propyl-N-(5-methoxychroman-3-yl)-amino]-butyl]-2,4-dioxo-3-azabicyclo[3.3.0]octane, its isomers and also its addition salts with a pharmaceutically acceptable acid.

16. Process for the preparation according to claim 1 of the compound of formula (I) which is ⊕-3-[4-[N-propyl-N-(5-methoxychroman-3-yl)-amino]-butyl]-2,4-dioxo-3-azabicyclo[3.3.0]octane, and also its addition salts with a pharmaceutically acceptable acid.

17. Process for the preparation according to claim 1 of the compound of formula (I) which is ⊖-3-[4-[N-propyl-N-(5-methoxychroman-3-yl)-amino]-butyl]-2,4-dioxo-3-azabicyclo[3.3.0]octane, and also its addition salts with a pharmaceutically acceptable acid.

18. Process for the preparation of pharmaceutical compositions containing as active ingredient at least one compound obtained according to any one of claims 1 to 17, alone or in combination with one or more pharmaceutically acceptable inert, non-toxic excipients or carriers.

19. Process for the preparation of pharmaceutical compositions obtained according to claim 18 containing at least one active ingredient obtained according to any one of claims 1 to 17, for use in the treatment of depression, stress, psychoses, anxiety, pain, schizophrenia, hypertension, prevention of atheromas and as modifiers of eating and sexual behaviour.